# EUROPEAN PATENT SPECIFICATION

(11) **EP 4 303 213 B1**
(45) Date of publication and mention of the grant of the patent: **02.09.2026**
(21) Application number: 22763364.1
(22) Date of filing: 02.03.2022
(51) Int. Cl.: C07D 257/02, C07D 213/60, C07D 487/04, A61K 51/02, A61K 51/04

(54) **COMPOUND AND RADIOACTIVE LABELING COMPOUND**
VERBINDUNG UND RADIOAKTIVE MARKIERUNGSVERBINDUNG
COMPOSÉ ET COMPOSÉ DE MARQUAGE RADIOACTIF

(30) Priority: 04.03.2021 JP 2021034026; 09.09.2021 JP 2021146833
(43) Date of publication of application: 10.01.2024
(73) Proprietor: Nihon Medi-Physics Co., Ltd., Tokyo 136-0075 (JP); Kyoto University, Kyoto-shi, Kyoto 606-8501 (JP)
(72) Inventor: MAYA, Yoshifumi, Tokyo 136-0075 (JP); ICHIKAWA, Hiroaki, Tokyo 136-0075 (JP); HIGAKI, Yusuke, Tokyo 136-0075 (JP); ONO, Masahiro, Kyoto-shi, Kyoto 606-8501 (JP); IIKUNI, Shimpei, Kyoto-shi, Kyoto 606-8501 (JP)
(74) Representative: TBK
(86) International application number: PCT/JP2022/009003
(87) International publication number: WO 2022/186311

(56) References cited:
- EP-A1- 4 116 306
- WO-A1-2018/098390
- WO-A1-2018/187631
- WO-A1-2018/215627
- WO-A1-2019/075583
- WO-A1-2020/106886
- WO-A1-2020/124237
- WO-A1-2020/220023
- WO-A1-2021/177390
- WO-A1-2021/177390
- JP-A- 2019 535 754
- JP-A- 2020 516 611
- JP-A- 2020 520 902
- JP-A- 2021 503 490
- SHIMPEI IIKUNI: "Radiotheranostics Using a Novel 225 Ac-Labeled Radioligand with Improved Pharmacokinetics Targeting Prostate-Specific Membrane Antigen", JOURNAL OF MEDICINAL CHEMISTRY, vol. 64, no. 18, 3 September 2021 (2021-09-03), US, pages 13429 - 13438, XP093154406, ISSN: 0022-2623, DOI: 10.1021/acs.jmedchem.1c00772

## Description

### Technical Field

The present invention relates to a These are defined in the claims.

### Background Art

Radiolabeled compounds, having a structure containing a radioactive nuclide, have been used as a reagent for detecting a target molecule, a diagnostic agent, or medicine to treat a disease. For the purpose of further improving performance in detecting a lesion and therapeutic effect, studies are in progress on: improving specific accumulation at a target tissue and site; and reducing accumulation at a non-target tissue and site.

Patent Literature 1 discloses a derivative (HTK01169) that has, as an albumin-binding site, an added iodophenylbutyryl group that branches from a PSMA-617 structure. It is also disclosed that this derivative targets and binds to prostate-specific membrane antigen (PSMA) and can be used in order to detect and treat prostate cancer.

Patent Literature 2 discloses an albumin-binding PSMA inhibitor. It is also disclosed that this inhibitor also targets and binds to PSMA as disclosed in Patent Literature 1 and can be used in order to detect and treat prostate cancer.

WO 2020/220023 A1 relates to a compound according to a specific Formula I displaying very good binding affinities to the PSMA binding sites and comprising a radioactive isotope or a chelating moiety that can be labeled with a radioactive metal such as ⁶⁸Ga or ¹⁷⁷Lu, as well as to pharmaceutical compositions comprising a pharmaceutical acceptable carrier and the compound of Formula I or a complex thereof, or a pharmaceutically acceptable salt thereof.

WO 2018/215627 A1 relates to compounds that are useful as radiopharmaceuticals, imaging agents and for treatment of cancer.

WO 2020/106886 A1 relates to compounds as well as compositions including such compounds useful in targeted radiotherapy of cancer and/or mammalian tissue overexpressing PSMA where the compounds are represented by a specific Formulas (I), or a pharmaceutically acceptable salt thereof (IA), or a pharmaceutically acceptable salt thereof (II), or a pharmaceutically acceptable salt thereof, containing an alpha-emitting radionuclide.

WO 2018/187631 A1 relates to compounds, as well as compositions including such compounds, useful for imaging and/or treatment of a glioma, a breast cancer, an adrenal cortical cancer, a cervical carcinoma, a vulvar carcinoma, an endometrial carcinoma, a primary ovarian carcinoma, a metastatic ovarian carcinoma, a non-small cell lung cancer, a small cell lung cancer, a bladder cancer, a colon cancer, a primary, gastric adenocarcinoma, a primary colorectal adenocarcinoma, a renal cell carcinoma, and/or a prostate cancer.

WO 2020/124237 A1 relates to a compound or molecular complex, comprising a metal chelator configured for chelation with a radioactive isotope or a non-radioactive isotope; and a trifluoroborate (BF3)-containing moiety configured for ¹⁹F/¹⁸F exchange or a precursor thereof; and optionally a cell-targeting domain.

WO 2021/177390 A1 relates to a compound having a chemical structure which, when viewed macroscopically, is arranged such that a chelating portion is positioned in the center of the structure, an atomic group containing an albumin binding site is bonded to one side of the chelating portion, and an atomic group containing a target molecule binding site is bonded to the other side of the chelating portion, the chelating portion is preferably DOTA or a derivative thereof, the target molecule binding site preferably has a structure which binds to a target molecule expressed in cancer tissue, and to a radioactive labeling compound in which the compound is coordinated with a radioactive metal ion.

Shimpei Iikuni et al (J. Med. Chem. 2021, 64, 18, 13429-13438) relates to radiotheranostics using a ²²⁵Ac-labeled radioligand with improved pharmacokinetics targeting PSMA.

### Citation List

### Patent Literature

Patent Literature 1: WO 2019/075583 A1
Patent Literature 2: WO 2018/098390 A1

### Summary of Invention

In achieving: improvement in specific accumulation at a target tissue and site; and reduction in accumulation at a non-target tissue and site, it is important to control pharmacokinetics, such as retention in blood. As a method to effectively control pharmacokinetics, chemical structures are desired to be further optimized. In general, a compound with a small molecular weight has poor retention in blood, which may result in: insufficient accumulation at a target tissue; and unintended accumulation at a normal tissue. In addition, the compounds disclosed in Patent Literature 1 and 2 undesirably show much unspecific accumulation at the kidney, still having had room for improvement in this respect.

Therefore, the present invention relates to a compound and a radiolabeled compound capable of achieving both of: improvement in accumulation at a target tissue; and reduction in accumulation at a non-target tissue, particularly reduction in accumulation at the kidney.

The present invention provides a compound including a structure having: a chelating part capable of coordinating with a radioactive metal ion; a first atomic group containing an albumin-binding part; and a second atomic group containing a PSMA molecule-binding part,
wherein the first atomic group and the second atomic group are bonded with each other through the chelating part, the compound being represented by the general formula (1) as defined by claim 1.

The present invention provides a radiolabeled compound comprising a radioactive metal ion and the compound which is coordinated with a radioactive metal ion.

The present invention provides a method for manufacturing a radiolabeled compound, the method including: coordinating the compound to a radioactive metal ion to obtain a radiolabeled compound.

### Brief Description of Drawings

[Fig.1] Fig. 1 is graphs showing the results of the cell-binding assay in Example 1-1 ([¹¹¹In]In-PSMA-DA1) and Comparative Example 1-1 ([¹¹¹In]In-PSMA-DB).
[Fig. 2] Fig. 2 is a graph showing the results of the albumin-binding experiments in [¹¹¹In]In-PSMA-DA1 and [¹¹¹In]In-PSMA-DB.
[Fig. 3] Fig. 3 is graphs showing the results of the internal radioactivity distribution experiments in [¹¹¹In]In-PSMA-DA1 and [¹¹¹In]In-PSMA-DB.
[Fig. 4] Fig. 4 is SPECT/CT images in [¹¹¹In]In-PSMA-DA1 and [¹¹¹In]In-PSMA-DB.
[Fig. 5] Fig. 5 is graphs showing changes in tumor volume and mouse body weight in Example 1-2 ([⁹⁰Y]Y-PSMA-DA1) and Comparative Example 1-2 ([⁹⁰Y]Y-PSMA-DB).
[Fig. 6] Fig. 6 is graphs showing changes in tumor volume and mouse body weight in Example 1-3 ([²²⁵Ac]Ac-PSMA-DA1) and Comparative Example 1-3 ([²²⁵Ac]Ac-PSMA-DB).
[Fig. 7] Fig. 7 is an HPLC chart showing the results of stability in plasma in Reference Example 2 ([¹¹¹In]In-PtDA).
[Fig. 8] Fig. 8 is a graph showing the results of the cell-binding assay in [¹¹¹In]In-PtDA.
[Fig. 9] Fig. 9 is a graph showing the results of the albumin-binding experiments in [¹¹¹In]In-PtDA.
[Fig. 10]Fig. 10 is SPECT/CT images in [¹¹¹In]In-PtDA.
[Fig. 11] Fig. 11 is graphs showing the results of the cell-binding experiments in Reference Example 3-1 ([¹¹¹In]In-Octapa-2), Reference Example 3-2 ([¹¹¹In]In-Octapa-3), Reference Example 3-3 ([¹¹¹In]In-Neunpa-2), and Comparative Example 2-1 ([¹¹¹In]In-Octapa-1) and Comparative Example 2-2 ([¹¹¹In]In-Neunpa-1).
[Fig. 12]Fig. 12 is a graph showing the results of the albumin-binding experiments in Examples 3-1 to 3-3 and Comparative Examples 2-1 to 2-2.
[Fig. 13]Fig. 13 is graphs showing the results of the internal radioactivity distribution experiments in Examples 3-1 to 3-3 and Comparative Examples 2-1 to 2-2.
[Fig. 14]Fig. 14 is a SPECT/CT image in Reference Example 3-1 ([¹¹¹In]In-Octapa-2).
[Fig. 15]Fig. 15 is a graph showing the results of the cell-binding assay in Example 4-1 ([¹¹¹In]In-PSMA-NAT-DA1).
[Fig. 16]Fig. 16 is a graph showing the results of the albumin-binding experiments in Example 4-1 ([¹¹¹In]In-PSMA-NAT-DA1).
[Fig. 17]Fig. 17 is SPECT/CT images in Example 4-1 ([¹¹¹In]In-PSMA-NAT-DA1).

### Description of Embodiments

Hereinafter, the compound of the present invention and the radiolabeled compound using the same will be described based on preferred embodiments thereof. In the following description, "T to U [V]" (T and U are arbitrary numbers, and [V] is a unit.) means "T [V] or more and U [V] or less" unless otherwise specified. In addition, when an asymmetric carbon atom is present in the structure, unless otherwise specified, each independently may be the S configuration or the R configuration.

The compound of the present invention, roughly classified based on its chemical structure, includes a structure having three atomic groups: a chelating part capable of coordinating with a radioactive metal ion; a first atomic group containing an albumin-binding part; and a second atomic group containing a part binding to a prostate-specific membrane antigen (Hereinafter, it is also referred to as PSMA.) molecule.

Having such a chemical structure, the radiolabeled compound in which the compound of the present invention is coordinated with a radioactive metal ion is capable of achieving both of: improvement in accumulation at a target tissue expressing a PSMA molecule; and reduction in accumulation at a non-target tissue, particularly at the kidney. The compound of the present invention is a precursor compound to be used for labeling with a radioisotope such as a radioactive metal, that is, preferably a compound to be used as a labeling precursor. The radioactive metal will be described later.

**In** the embodiment, the compound of the present invention is a compound in which a first atomic group containing an albumin-binding part is bonded with a second atomic group containing a PSMA molecule-binding part through a chelating part.

Such a compound is represented by the following general formula (1).

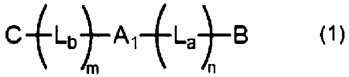

In the formula (1), A₁ represents a chelating part capable of coordinating with a radioactive metal ion; B represents an atomic group containing an albumin-binding part; and C represents an atomic group containing a PSMA molecule-binding part.

Lₐ represents a linker structure comprising a structure derived from a compound capable of forming an amide bond.

L_{b} represents a linker structure that is identical to or different from Lₐ, comprising a structure derived from an amino acid having a structure containing an alicyclic of 5 to 10 carbon atoms.

"m" and "n" each independently represent 0 (zero) or 1.

B or Lₐ is bonded to an arbitrary position of A₁.

C or L_{b} is bonded to A₁ at a position different from the position at which B or Lₐ is bonded to A₁,
wherein in the formula (1), the chelating part represented by A₁ has a structure derived from a compound represented by any one of the following formulas (A1) to (A9),
wherein, in the formula (1), the albumin-binding part has a structure represented by the following formula (B 1) or (B2), and
wherein, in the formula (1), C represents the following formula (C1).

As described above, the compound in the embodiment, seen as its macroscopic chemical structure, is a compound in which the chelating part (in the general formula (1), represented by symbol A₁) is positioned at the center of the structure, and the first atomic group containing an albumin-binding part (in the general formula (1), represented by symbol B) and the second atomic group containing a PSMA molecule-binding part (in the general formula (1), represented by symbol C) are linearly arranged through the chelating part.

In the general formula (1), each of "between A₁ and B" and "between A₁ and C", independently, may be indirectly bonded via the linker structure, or may be directly bonded without the linker structure. In the formula (1), when each of "between A₁ and B" and "between A₁ and C" has a linker structure, and the linker structures may be the same as or different from each other.

Details of the linker structure will be described later.

From the viewpoint that the radiolabeled compound made from the compound of the present invention achieves, in higher level, both of: improvement in accumulation at a target tissue expressing a PSMA; and reduction in accumulation at a non-target tissue, particularly at the kidney, it is preferable in the formula (1) that: A₁ has a cyclic structure, the cyclic structure has two or more nitrogen atoms, and each of the nitrogen atoms is connected with one another through two or more adjacent carbon atoms; or A₁ has an open chain structure, the open chain structure has two or more nitrogen atoms, and each of the nitrogen atoms is connected with one another through two or more adjacent carbon atoms.

In the formula (1), when A₁ has a cyclic structure, the skeleton of the cyclic structure may be composed only of nitrogen atoms and carbon atoms, or may be composed of oxygen atoms in addition to nitrogen atoms and carbon atoms. The bonds between carbon atoms in the cyclic structure may be a chain or may form a cyclic structure.

In the formula (1), when A₁ has an open chain structure, the bonds between carbon atoms in the open chain structure may be divided by a nitrogen atom. The bonds between carbon atoms in the open chain structure may be a chain or may form a cyclic structure.

In the formula (1), when A₁ has a cyclic structure or an open chain structure, A₁ preferably has a nitrogen-bonding atomic group directly bonded with a nitrogen atom constituting the cyclic structure or the open chain structure. As a specific example of the nitrogen-bonding atomic group, an atomic group containing one or more groups selected from the group consisting of a carboxy group, a phosphate group, an amide group, a benzene ring, and a pyridine ring is preferable, and the atomic group is more preferably a chain.

Furthermore, in the formula (1), when A₁ has a cyclic structure or an open chain structure, under the condition that: B is bonded to an arbitrary position of A₁; and C is bonded to an arbitrary position of A₁ different from the bonding position of B, it is preferable that, when B is bonded to the nitrogen-bonding atomic group through Lₐ or without Lₐ, C is bonded to a position other than a nitrogen-bonding atomic group to which B is bonded through Lₐ or without Lₐ.

Specifically, in the formula (1), the chelating part capable of coordinating with a radioactive metal represented by symbol A₁ has a structure derived from a compound represented by any one of the following formulas (A1) to (A9), and preferably has a structure derived from a compound represented by the following formula (A1). That is, the compound of the present invention is a derivative of a compound represented by any one of the following formulas (A1) to (A9), and preferably a derivative of a compound represented by the following formula (A1). These structures can be appropriately selected according to the type of radioactive metal described later. The chelating part having any of the structures achieves both of: improvement in accumulation at a target tissue expressing PSMA; and reduction in accumulation at a non-target tissue, particularly at the kidney.

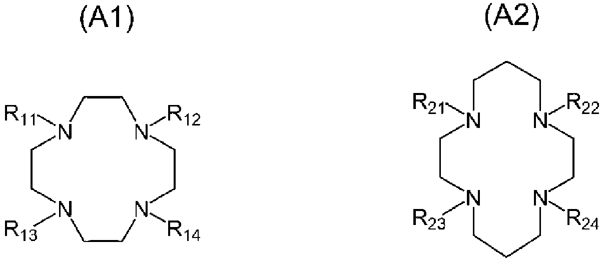

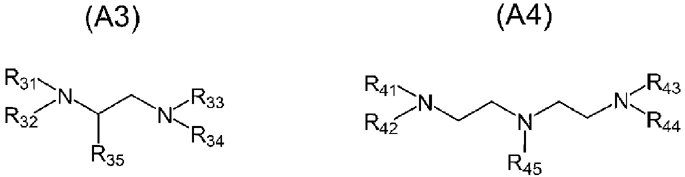

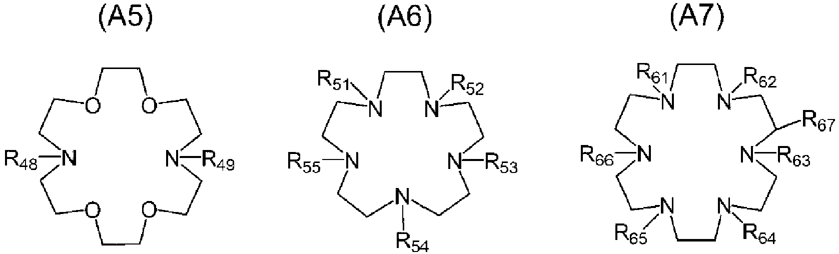

In the formula (A1), R₁₁, R₁₂, R₁₃, and R₁₄ are each independently any of groups consisting of -(CH₂)ₚCOOH, -(CH₂)ₚC₅H₅N, -(CH₂)ₚPO₃H₂, -(CH₂)ₚCONH₂, and - (CHCOOH)(CH₂)ₚCOOH; and "p" is an integer of 0 or more and 3 or less.

In the formula (A2), R₂₁, R₂₂, R₂₃, and R₂₄ each independently represent a carboxy group or a carboxyalkyl group having 2 or 3 carbon atoms.

In the formula (A3), R₃₁, R₃₂, R₃₃, and R₃₄ each independently represent an atomic group having a hydrogen atom and 2 or more and 10 or less carbon atoms and optionally containing a nitrogen atom or an oxygen atom; and R₃₅ represents a hydrogen atom, a carboxy group, or a carboxyalkyl group having 2 or 3 carbon atoms.

In the formula (A4), R₄₁, R₄₂, R₄₃, and R₄₄ each independently represent an atomic group having a hydrogen atom and 2 or more and 10 or less carbon atoms and optionally containing a nitrogen atom or an oxygen atom; and R₄₅ represents a hydrogen atom, a carboxy group, or a carboxyalkyl group having 2 or 3 carbon atoms.

In the formula (A5), R₄₈ and R₄₉ each independently represent an atomic group having a hydrogen atom and 2 or more and 10 or less carbon atoms and optionally containing a nitrogen atom or an oxygen atom.

In the formula (A6), R₅₁, R₅₂, R₅₃, R₅₄, and R₅₅ each independently represent an atomic group having a hydrogen atom and 2 or more and 10 or less carbon atoms and optionally containing a nitrogen atom or an oxygen atom.

In the formula (A7), R₆₁, R₆₂, R₆₃, R₆₄, R₆₅, and R₆₆ each independently represent an atomic group having a hydrogen atom and 2 or more and 10 or less carbon atoms and optionally containing a nitrogen atom or an oxygen atom; and R₆₇ represents a hydrogen atom, a carboxy group, or a carboxyalkyl group having 2 or 3 carbon atoms.

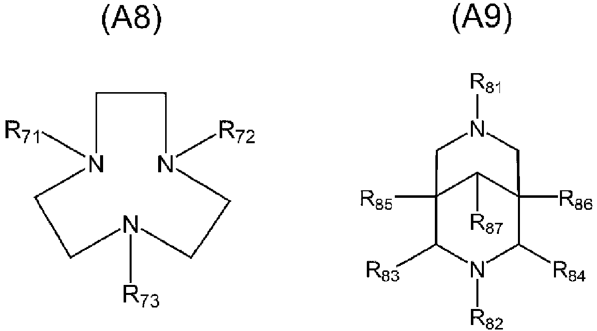

**In** the formula (A8), R₇₁, R₇₂ and R₇₃ each independently represent an atomic group having a hydrogen atom and 2 or more and 10 or less carbon atoms and optionally containing a nitrogen atom or an oxygen atom.

**In** the formula (A9), R₈₁ and R₈₂ are each independently an alkyl group having 1 or more and 5 or less carbon atoms, and the terminal of the alkyl group may be substituted with a pyridyl group substituted with 1 or more carboxy groups; R₈₇ is a hydroxyl group or the oxygen atom (=O) of a carbonyl group; R₈₃ and R₈₄ are a substituted or unsubstituted pyridinyl group; R₈₅ and R₈₆ are each independently -COO-Rₐ; and Rₐ is an alkyl group having 1 or more and 5 or less carbon atoms.

Examples of the specific structure represented by the formula (A1) include structures represented by the following formulas (A1-1) to (A1-7).

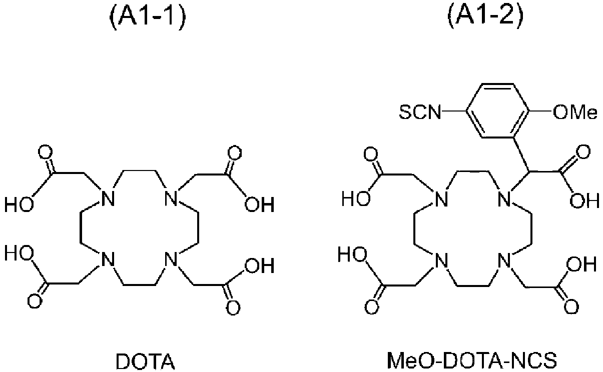

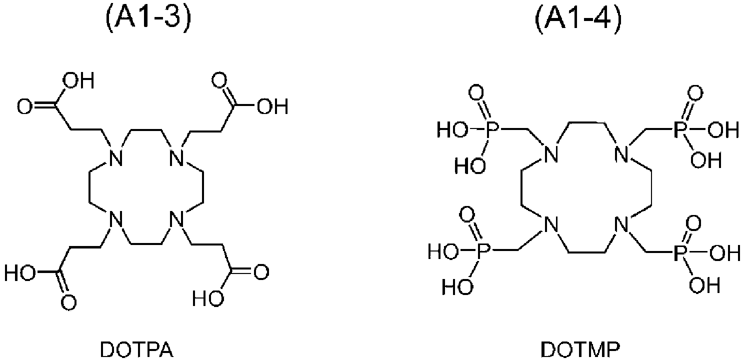

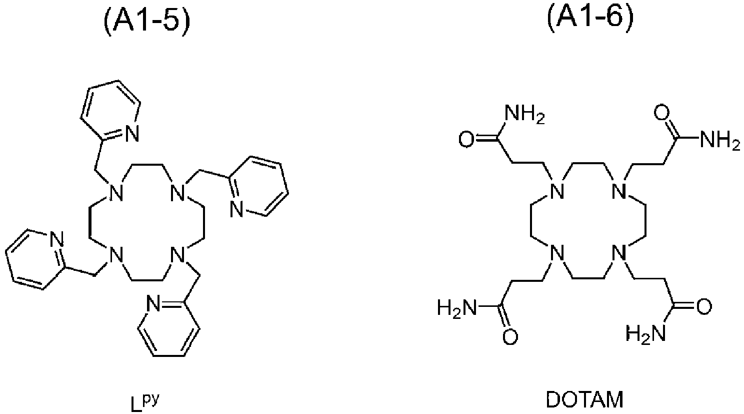

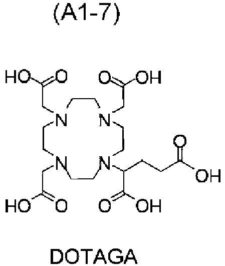

Examples of the specific structure represented by the formula (A2) include structures represented by the following formulas (A2-1) to (A2-2).

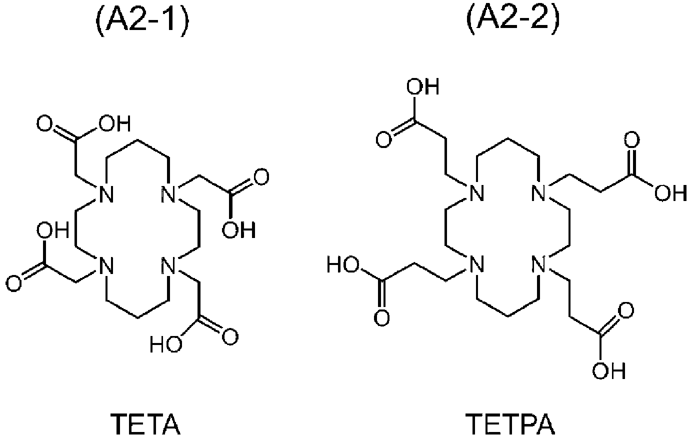

Examples of the specific structure represented by the formula (A3) include structures represented by the following formulas (A3-1) to (A3-7).

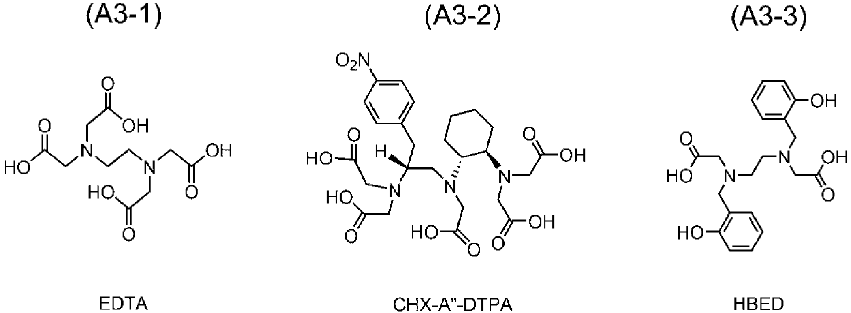

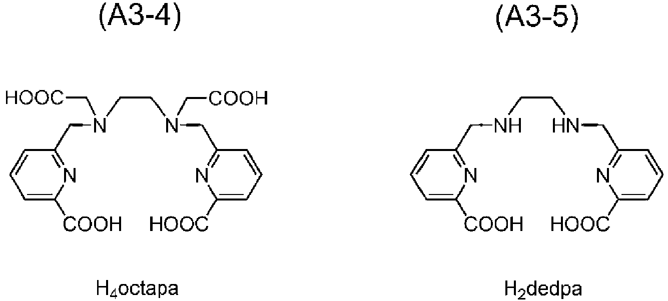

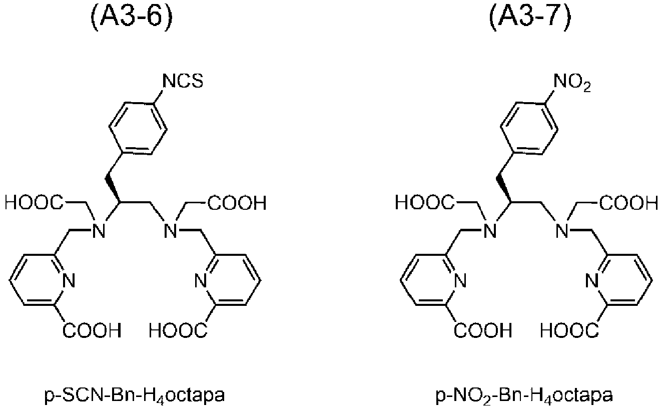

Examples of the specific structure represented by the formula (A4) include structures represented by the following formulas (A4-1) to (A4-2).

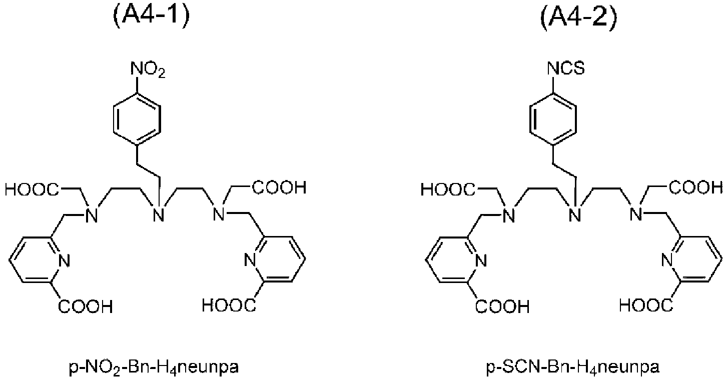

Examples of the specific structure represented by the formula (A5) include structures represented by the following formulas (A5-1) to (A5-3).

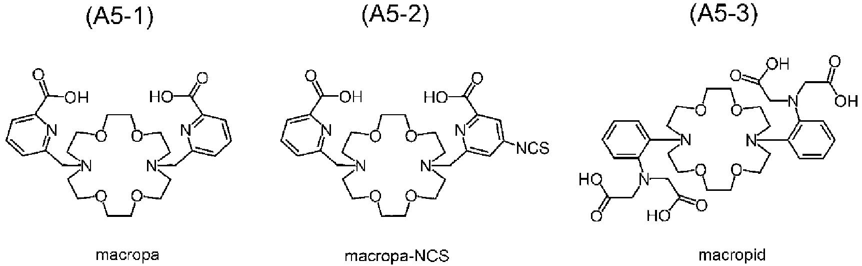

Examples of the specific structure represented by the formula (A6) include a structure represented by the following formula (A6-1).

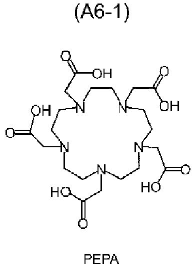

Examples of the specific structure represented by the formula (A7) include structures represented by the following formulas (A7-1) to (A7-2).

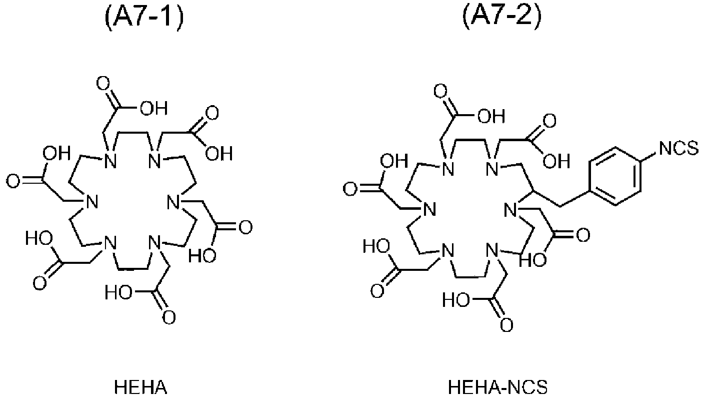

Examples of the specific structure represented by the formula (A8) include structures represented by the following formulas (A8-1) to (A8-3).

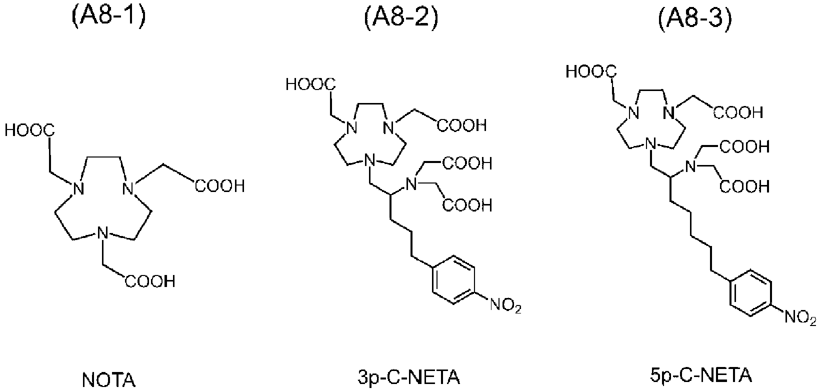

Examples of the specific structure represented by the formula (A9) include structures represented by the following formulas (A9-1) to (A9-4).

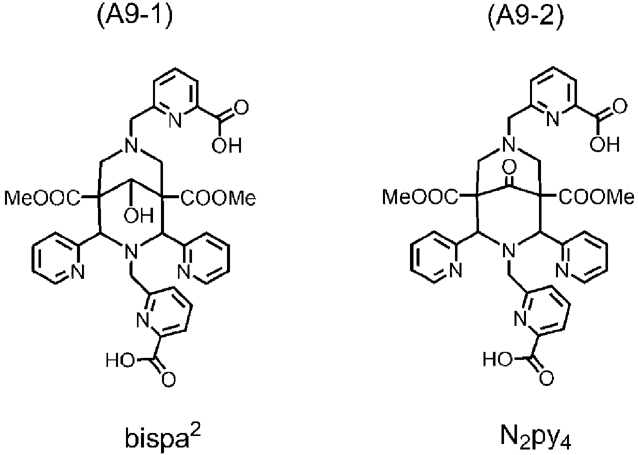

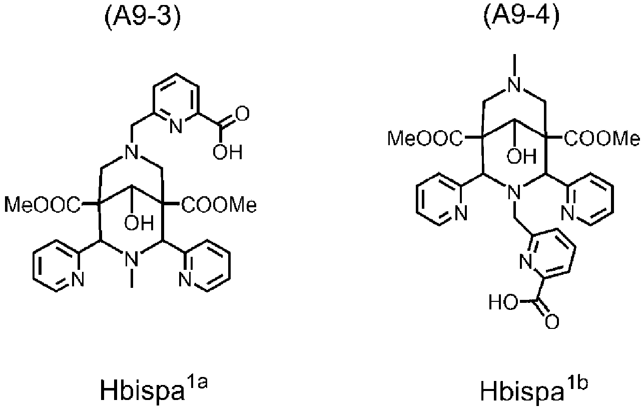

Another embodiment of the compound of the present invention is described below. In this embodiment, differences from the embodiment described above will be mainly described. Unless specifically described, the description of the embodiment described above is appropriately applied thereto.

Hereinafter, what is applicable to the above-described embodiment will be described.

In the formula (1), the position represented by symbol B is an atomic group containing an albumin-binding part, which has an affinity to albumin, preferably serum albumin, more preferably human serum albumin, and has a chemical structure capable of reversibly binding to albumin. The compound of the present invention, containing such a structure, can enhance retention in blood and reduce accumulation at the kidney when the compound is labeled with a radioactive metal and administered to a living body.

Specifically, the compound having an albumin-binding part in the molecule is easily bonded to albumin in blood, and the compound bonded to albumin no longer undergoes glomerular filtration in the kidney, thereby reducing migration into the kidney or urine; and excretion to improve retention in blood. As a result, accumulation at the kidney, a normal tissue, is reduced and the compound can be further improved in transferability into a target tissue, such as a tumor tissue expressing PSMA.

When the compound represented by the formula (1) is used, the distance between the albumin-binding part and the PSMA molecule-binding part can be appropriately secured, so that both affinity to albumin and affinity to a PSMA molecule can be provided. In particular, the albumin-binding part is disposed at one structural terminal of the compound of the present invention and the PSMA molecule-binding part is disposed at the other structural terminal of the compound of the present invention. Thus it is possible to sufficiently secure the distance between the albumin-binding part and PSMA molecule-binding part, which is further advantageous in that both affinity to albumin and affinity to a PSMA molecule can be achieved in higher level.

In the formula (1), from the viewpoint of obtaining a compound applicable to a living body and reducing accumulation at an unintended normal organ such as the kidney, as the structure of the albumin-binding part, the present invention uses one or more of a substituted or unsubstituted phenylbutyric acid and Evans blue, and derivatives thereof.

The substituted or unsubstituted phenylbutyric acid applicable as the albumin-binding part is a structure represented by the following formula (B1): 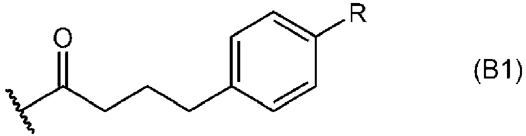 wherein, in the formula (B1), R represents a hydrogen atom, a halogen atom, or an alkyl group having 1 or more and 5 or less carbon atoms; and the wavy line part represents a part bonding to another structure.

In the formula (B1), R is preferably a hydrogen atom, an iodine atom, a bromine atom, or a methyl group.

Evans blue and a derivative thereof applicable as the albumin-binding part is a structure represented by the following formula (B2): 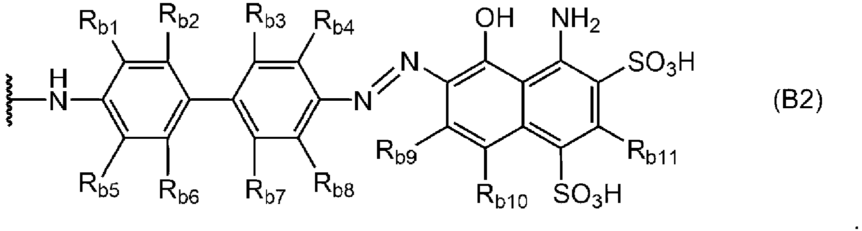 wherein, in the formula (B2), R_{b1} to R_{b11} each independently represent a hydrogen atom, a halogen atom, a hydroxyl group, a cyano group, a substituted or unsubstituted alkyl group having 1 or more and 6 or less carbon atoms, or a substituted or unsubstituted alkoxy group having 1 or more and 6 or less carbon atoms; and the wavy line part represents a part bonding to another structure.

In the formula (B2), it is preferable that both R_{b1} and R_{b4} are a methyl group, and all of R_{b2}, R_{b3}, and R_{b5} to R_{b11} are a hydrogen atom.

In the formula (1), the PSMA molecule-binding part represented by symbol C is an atomic group containing a PSMA molecule-binding part that has affinity to a PSMA molecule expressed in a tissue that causes a disease such as cancer and has a chemical structure capable of reversibly binding to the PSMA molecule. The compound of the present invention, containing such a structure, can efficiently accumulate at a tissue to be treated or diagnosed to enhance the efficiency of treatment or diagnosis when the compound is labeled with a radioactive metal and administered to a living body.

PSMA is a membrane-bound protein that is accelerated to express particularly in prostate cancer. Although PSMA expresses in a smaller amount in a normal tissue including the prostate, PSMA is accelerated to express in a higher amount as the grade of prostate cancer increases. Therefore, PSMA is one of the useful target molecules in the present invention, and is particularly useful as a target for diagnosis and treatment of prostate cancer.

Examples of cancer expressing a PSMA molecule include prostate cancer. The prostate cancer may be primary or metastatic.

In the above formula (1), the PSMA molecule-binding part has a structure represented by the following formula (C1). The compound of the present invention, containing such a structure, can efficiently accumulate at a tissue to be treated or diagnosed to enhance the efficiency of treatment or diagnosis when the compound is labeled with a radioactive metal and administered.

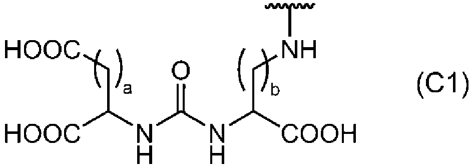

In the formula (C1), "a" and "b" each independently represent an integer of 1 or more and 7 or less;
and in the formula (C1), the wavy line part represents a part bonding to A₁ or L_{b} in the formula (1).

The compound of the present invention having a structure of the formula (1) is preferable to have a structure represented by the following general formula (1S), and is also more preferable to have a structure represented by the following general formula (1T).

Such a structure makes it possible to have sufficient hetero atoms capable of coordinating with a radioactive metal in the structure. Therefore, complex formation efficiency can be enhanced when a radioactive metal is coordinated with the compound of the present invention. In addition, since degree of freedom in molecular mobility is increased within the albumin-binding part and the PSMA molecule-binding part, both affinity to albumin and affinity to a PSMA molecule can be achieved in higher level. Furthermore, unintended accumulation at a normal tissue, such as the liver and kidney, is reduced.

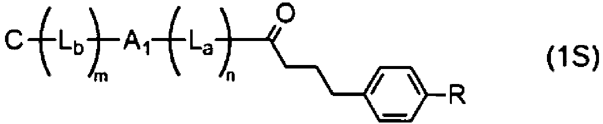

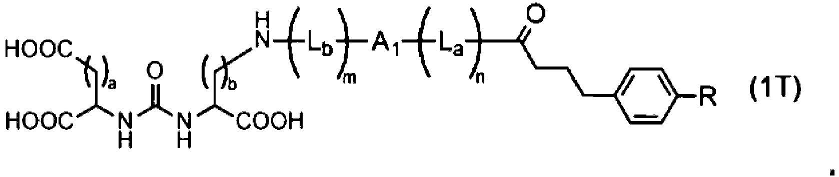

In the formula (1S) and the formula (1T), A₁ each independently represents a chelating part capable of coordinating with a radioactive metal ion as defined above.

In the formula (1S), C represents an atomic group containing a PSMA molecule-binding part as defined above.

In the formula (1S) and the formula (1T), Lₐ each independently represents a linker structure as defined above.

In the formula (1S) and the formula (1T), L_{b} each independently represents a linker structure that is identical to or different from Lₐ as defined above.

In the formula (1S) and the formula (1T), "m" and "n" each independently represent 0 (zero) or 1.

In the formula (1S) and the formula (1T), the same description as in the above formula (B1) is appropriately applied.

In the formula (1S) and the formula (1T), when "n" is 1, Lₐ is bonded to an arbitrary position of A₁.

In the formula (1S) and the formula (1T), when "n" is 0, the carbon atom of the carbonyl group bonded to the phenylalkyl group is bonded to an arbitrary position of A₁.

In the formula (1S) and the formula (1T), when "m" is 1, L_{b} bonds to A₁ at a position different from the position at which Lₐ is bonded to A₁.

In the formula (1S) and the formula (1T), when "m" is 0, C or the nitrogen atom is bonded to A₁ at a position different from the position at which La is bonded to A₁.

The compound of the present invention having the structure of the formula (1) is more preferable to have a structure represented by the following general formula (3).

Such a structure makes it possible to have sufficient hetero atoms capable of coordinating with a radioactive metal in the structure. Therefore, complex formation efficiency can be enhanced when a radioactive metal is coordinated with the compound of the present invention. In addition, since degree of freedom in molecular mobility is increased within the albumin-binding part and the PSMA molecule-binding part, both affinity to albumin and affinity to a PSMA molecule can be achieved in higher level. Furthermore, unintended accumulation at a normal tissue, such as the liver and kidney, is reduced.

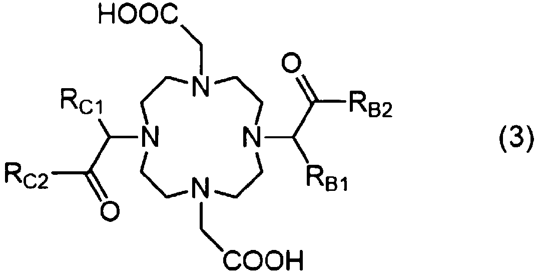

In the formula (3), one of R_{B1} and R_{B2} is an atomic group containing an albumin-binding part, and the other is a hydrogen atom, a hydroxyl group, or a carboxy group; and one of R_{C1} and R_{C2} is an atomic group containing a PSMA molecule-binding part, and the other is a hydrogen atom, a hydroxyl group, or a carboxy group.

Among them, in the formula (3), it is preferable that R_{B1} is an atomic group containing an albumin-binding part, R_{C1} is an atomic group containing a PSMA molecule-binding part, and both R_{B2} and R_{C2} are a hydroxyl group.

Alternatively, in the formula (3), it is also preferable that R_{B2} is an atomic group containing an albumin-binding part, R_{C2} is an atomic group containing a PSMA molecule-binding part, and R_{B1} and R_{C1} are both a hydrogen atom or each independently a carboxyalkyl group having 1 to 5 carbon atoms.

In any of the embodiments described above, it is preferable that the albumin-binding part is disposed at one structural terminal of the compound and the PSMA molecule-binding part is disposed at the other structural terminal of the compound, and therefore, seen as the macroscopic chemical structure of the compound of the present invention, the chelating part, the albumin-binding part, and the PSMA molecule-binding part are almost linearly disposed.

In particular, in the formula (3), the atomic group containing an albumin-binding part is an atomic group containing the structure represented by the formula (B1) or the formula (B2) described above as the albumin-binding part. Specific examples of such a chemical structure are shown in the following formulas (3-1) to (3-4).

In the following formulas (3-1) and (3-2), L₁ each independently represents an alkyl group having 1 or more and 8 or less carbon atoms and containing a carboxy group.

In the following formulas (3-3) and (3-4), each independently, "g" represents an integer of 1 or more and 5 or less; and "h" represents 0 or 1.

Furthermore, in the following formulas (3-1) to (3-4), regarding the descriptions of R, R_{b1} to R_{b11}, and R_{C1} and R_{C2}, the descriptions regarding the above-described formulas (B1) and (B2), formula (C1), and formula (3) are appropriately applied independently.

That is, R is a hydrogen atom, a halogen atom, or an alkyl group having 1 or more and 5 or less carbon atoms, and preferably R is a hydrogen atom, an iodine atom, a bromine atom, or a methyl group.

R_{b1} to R_{b11} each independently represent a hydrogen atom, a halogen atom, a hydroxyl group, a cyano group, a substituted or unsubstituted alkyl group having 1 or more and 6 or less carbon atoms, or a substituted or unsubstituted alkoxy group having 1 or more and 6 or less carbon atoms. Preferably, both R_{b1} and R_{b4} represent a methyl group, and all of R_{b2}, R_{b3}, and R_{b5} to R_{b11} represent a hydrogen atom.

One of R_{C1} and R_{C2} is an atomic group containing a PSMA molecule-binding part represented by the formula (C1), and the other is a hydrogen atom, a hydroxyl group, or a carboxyalkyl group having 1 to 5 carbon atoms.

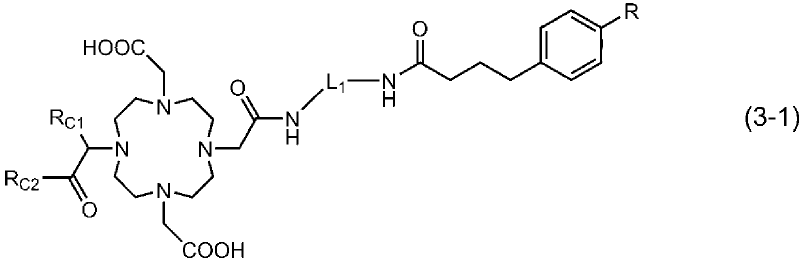

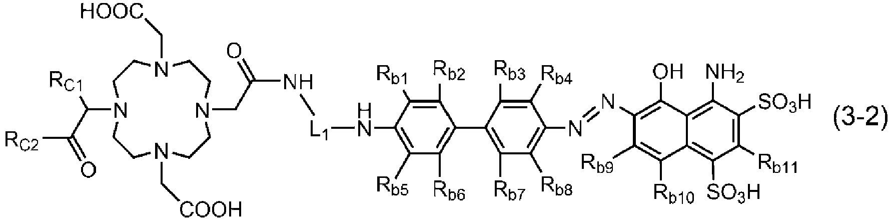

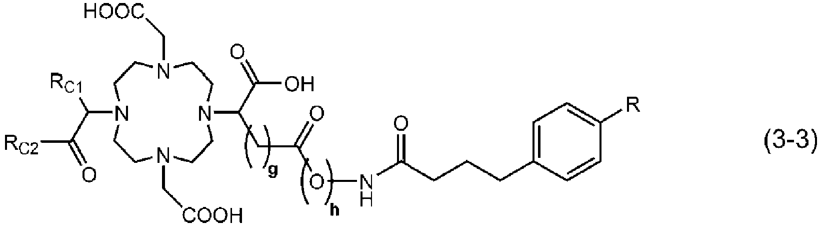

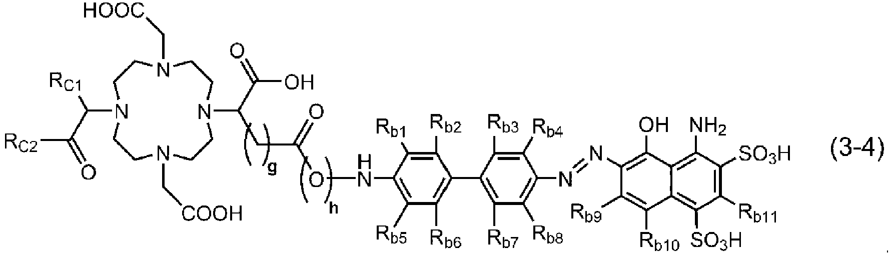

The compound of the present invention having each of the structures related to the formulas (1) and (3) can be manufactured, for example, by the method and synthetic route described in Examples described later.

The compound of the present invention, preferably in a condition that it has been dissolved in an aqueous solution such as a solvent and a buffer solution, is reacted with a radioactive metal ion to coordinate the compound in each of the embodiments to a radioactive metal ion, thereby obtaining the radiolabeled compound. This radiolabeled compound is a radioactive metal complex in which the chelating part of the compound is coordinated to a radioactive metal ion.

When the compound represented by the formula (1) is used in obtaining a radiolabeled compound, it is preferable that B in the formula (1) has a structure represented by the formula (B1). Specifically, in obtaining a radiolabeled compound, it is more preferable to use a compound represented by the formula (1S). Thereby, complex formation efficiency can be enhanced.

From the viewpoint of enhancing complex formation efficiency, the radioactive metal to be reacted with the compound is preferably used in the form of an ionizable radioactive metal compound, and more preferably used in the form of a radioactive metal ion (Hereinafter, these forms are also collectively referred to as "radioactive metal source".). As the radioactive metal source, for example, a radioactive metal ion-containing liquid in which a radioactive metal ion is dissolved or dispersed in a solvent mainly composed of water can be used.

From the viewpoint of enhancing the efficiency of complex formation with a radioactive metal regardless of the combination of the compound's chelating part and the radioactive metal, the complex is formed preferably by heating and reacting the compound and the radioactive metal. Under such reaction conditions, even when a radioactive metal nuclide emitting a low-energy radiation difficult to detect or α-rays is used, complex formation can proceed well. Therefore, a target radiolabeled compound can be obtained in high yield.

In obtaining the radiolabeled compound, the order of adding the compound and the radioactive metal source is not limited as long as the compound and the radioactive metal ion can form a complex. For example, it is possible that one of the compound and the radioactive metal source is added to a reaction vessel containing a solvent, and then the other is added to cause a reaction. It is also possible that a solution in which one of the compound and the radioactive metal source is dissolved in a solvent is added with the other to cause a reaction. Alternatively, they may be simultaneously added to a reaction vessel containing a solvent to cause a reaction.

The reaction conditions for obtaining the radiolabeled compound can be, for example, the following conditions. As the solvent used in this step, for example, water, saline, or a buffer solution, such as a sodium acetate buffer solution, an ammonium acetate buffer solution, a phosphate buffer solution, a phosphate buffer saline solution, a Tris buffer solution, a HEPES buffer solution, and a tetramethylammonium acetate buffer solution can be used. The reaction temperature may be, for example, room temperature (25°C) or under heating conditions.

As the radioactive metal source, for example, a solution in which a radioactive metal ion is dispersed in a solvent mainly composed of water can be used.

The amount of the reaction liquid in this step is not particularly limited, but from the viewpoint of practicality in the production step, 0.01 mL to 100 mL is practical at the start of this step. In addition, it is preferable that the concentrations of the compound and the radioactive metal ion in the reaction liquid are each independently 1 µM to 100 µM at the start of this step from the viewpoint of the yield of an intended radiolabeled compound.

The obtained radiolabeled compound may be used as it is, or may be purified using a filtration filter, a membrane filter, a column packed with various fillers, chromatography, or the like.

If necessary, in the subsequent steps, a solvent mainly containing water and other pharmaceutically acceptable components may be added to the radiolabeled compound to form a radioactive pharmaceutical composition containing the radiolabeled compound as an active ingredient. The radioactive pharmaceutical composition can be produced, for example, by dissolving the radiolabeled compound produced by the above-described method in a solvent that is mainly composed of water and is substantially isotonic with a living body. The radioactive pharmaceutical composition is administered to a living body orally, or parenterally, for example, intravenously, subcutaneously, intraperitoneally, and intramuscularly, and is used for treatment and diagnosis of a disease, detection of a lesion, or the like.

As the radioactive metal coordinated in the radiolabeled compound in an ionic state, a metal nuclide that emits radiation of α-rays, β-rays, γ-rays, or a combination thereof can be used. Examples of the nuclide of such a radioactive metal include a radioisotope of an alkali metal, an alkaline earth metal, a lanthanoid, an actinoid, a transition metal, or a metal other than these metals.

Among them, it is preferable to use ⁴⁴Sc, ⁵¹Cr, ⁵⁷Co, ⁵⁸Co, ⁶⁰Co, ⁵⁹Fe, ⁶⁴Cu, ⁶⁷Cu, ⁶⁷Ga, ⁶⁸Ga, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁷Hg, ¹⁹⁸Au, ²⁰¹Tl, ²⁰³Hg, ²¹²Pb, ²¹²Bi, ²¹³Bi, ²²⁵Ac, or ²²⁷Th as a radioactive metal nuclide from the viewpoint of being commercially available and improving complex formation. These radioactive metals can be produced by a conventional method. These radionuclides are preferably obtained as a solution containing a radioactive metal in an ionized state.

When the radiolabeled compound is used for the purpose of treating a disease, it is preferable to use an α-ray-emitting nuclide or a β⁻-ray-emitting nuclide as the radioactive metal from the viewpoint of enhancing therapeutic effect. The α-ray-emitting nuclide may be any nuclide that emits α-rays in the disintegration process of the radioactive metal. Specifically, ²¹²Bi, ²¹³Bi, ²²⁵Ac, ²²⁷Th or the like is preferably used. The nuclide is more preferably ²²⁷Th or ²²⁵Ac, and still more preferably ²²⁵Ac.

The β⁻-ray-emitting nuclide may be any nuclide that emits β⁻-rays in the disintegration process of the radioactive metal. Specifically, ⁵⁹Fe, ⁶⁰Co, ⁶⁴Cu, ⁶⁷Cu, ⁸⁹Sr, ⁹⁰Y, ^{99m}Tc, ¹⁰³Ru, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰³Hg, ²¹²Pb, ²¹²Bi, ²¹³Bi, or the like is preferably used, more preferably ⁶⁴Cu, ⁶⁷Cu, ⁸⁹Sr, ⁹⁰Y, ¹⁷⁷Lu, ¹⁸⁶Re, or ¹⁸⁸Re is used, and still more preferably ⁹⁰Y, is used.

In addition, when the radiolabeled compound is used for the purpose of diagnosis of a disease or detection of a lesion, it is preferable to use a β⁺-ray-emitting nuclide, an electron-capturing disintegration nuclide, or a γ-ray-emitting nuclide as the radioactive metal from the viewpoint of improving diagnostic performance. The β⁺-ray-emitting nuclide may be a nuclide that emits positrons in the disintegration process of the radioactive metal, and ⁴⁴Sc, ⁵⁸Co, ⁶⁸Ga, ⁶⁴Cu, ⁸⁹Zr, or the like is preferably used, and ⁶⁴Cu or ⁸⁹Zr is more preferably used.

The electron-capturing disintegration nuclide may be any nuclide that emits Auger electrons or characteristic X-rays in the disintegration process of the radioactive metal, and ⁵¹Cr, ⁵⁷Co, ⁵⁸Co, ⁶⁴Cu, ⁶⁷Ga, ⁶⁸Ga, ⁸⁹Zr, ¹¹¹In, ¹⁸⁶Re, ¹⁹⁷Hg, ²⁰¹Tl, or the like is preferably used.

The γ-ray-emitting nuclide may be a nuclide that emits γ-rays through γ-decay, and ⁶⁸Ga, ^{99m}Tc, or ²⁰¹Tl is preferably used as a nuclide that emits γ-rays through γ-decay.

When the radioactive metal to be coordinated in the radioactive metal complex in an ionic state is selected on the basis of ionic radius, examples of the radioactive metal having an ionic radius of about 70 to 130 pm include ⁶⁴Cu, ⁶⁷Cu, ⁶⁷Ga, ⁶⁸Ga, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Pb, ²¹²Bi, ²¹³Bi, and ²²⁵Ac, which can form a complex of the radioactive metal ion with the compound of the present invention having the chelating part preferably having a structure represented by the formula (A1) to (A9).

For example, when the radiolabeled compound is used for the purpose of treating a disease and ²²⁵Ac is used as the radioactive metal, as the compound of the present invention, a compound having a chelating part having a structure represented by any of the formulas (A1), (A3) to (A5), or (A7) is preferably used, and a compound having a chelating part having a structure represented by the formulas (A1), (A3), or (A4) is more preferably used. When ⁹⁰Y is used as the radioactive metal, as the compound of the present invention, a compound having a chelating part having a structure represented by any of the formulas (A1) to (A3) or (A8) is preferably used, and a compound having a chelating part having a structure represented by the formula (A1) is more preferably used.

When the radiolabeled compound is used for the purpose of diagnosing a disease or detecting a lesion and ⁸⁹Zr is used as the radioactive metal, as the compound of the present invention, a compound having a chelating part having a structure represented by any of the formulas (A1), (A3), or (A4) is preferably used, and a compound having a chelating part having a structure represented by the formula (A1) is more preferably used. When ⁶⁸Ga or ¹¹¹In is used as the radioactive metal, as the compound of the present invention, a compound having a chelating part having a structure represented by any of the formulas (A1) to (A4) or (A9) is preferably used, and a compound having a chelating part having a structure represented by the formula (A1) is more preferably used.

Regarding the bond between the chelating part and the atomic group containing the albumin-binding part, the chelating part and the albumin-binding part may be directly bonded without the later-described linker structure, or the chelating part and the atomic group containing the albumin-binding part may be indirectly bonded through the later-described linker structure.

Similarly, regarding the bond between the chelating part and the atomic group containing the PSMA molecule-binding part, the chelating part and the PSMA molecule-binding part may be directly bonded without the later-described linker structure, or the chelating part and the atomic group containing the albumin-binding part may be indirectly bonded through the later-described linker structure.

Whether the above "directly" form or "indirectly" form is employed, it is preferable to bond with each other through an amide bond from the viewpoint of achieving both easiness of synthesis and stability of chemical structure.

The linker structure of Lₐ in the formula (1) is a structure derived from a compound capable of forming an amide bond and L_{b} represents a linker structure, which includes a structure derived from an amino acid having a structure containing an alicyclic of 5 to 10 carbon atoms. Specific examples of a structure derived from a compound capable of forming an amide bond include a structure derived from: an L-form or D-form amino acid, such as an acidic amino acid, including glutamic acid and aspartic acid, and a basic amino acid, including lysine; a dicarboxylic acid, such as oxalic acid and malonic acid; a diamine, such as ethylenediamine; an amino acid having a structure containing an alicyclic of 5 to 10 carbon atoms or an aromatic of 6 to 14 carbon atoms; and the like. These can be employed alone or by connecting more than one through an amide bond. The above-described structures may be each independently unsubstituted or substituted with various substituents.

When the structure derived from an amino acid or the like is included as the above-described linker structure, for example, for the purpose of controlling kinetics in a living body, peptide linkers described in WO 2017/150549 A1, WO 2019/065774 A1, WO 2019/221269 A1, WO 2020/075746 A1, WO 2020/145227 A1, WO 2020/145228 A1, and the like can be used.

In the above-described embodiment, examples of the substituent that can be substituted in each atomic group, each structure, and each chemical structure of the compound and the radiolabeled compound include a halogen atom, a saturated or unsaturated alkyl group, a hydroxy group, an aldehyde group, a carboxy group, an acyl group, an amino group, a nitro group, an ester group, an isothiocyanate group, a thioxy group, a cyano group, an amide group, an imide group, a phosphate group, a phenyl group, a benzyl group, a pyridyl group, and a naphthyl group. These substituents may be one alone or a group in which two or more substituents are combined.

Although the present invention has been described above based on the preferred embodiments thereof, the present invention is not limited to the above-described embodiments. For example, in each of the above-described embodiments, a compound having one chelating part, one albumin-binding part, and one PSMA molecule-binding part has been described. However, as long as the present invention is exhibited, at least one of the albumin-binding part and the PSMA molecule-binding part may be contained at a plurality of positions in one chemical structure.

### Examples

Hereinafter, the present invention will be described in more detail with reference to Examples. However, the scope of the present invention is not limited to such examples.

In the following Examples, unless otherwise specified, NMR is always with JNM-AL400 FT-NMR apparatus, which is manufactured by JEOL Ltd., using tetramethylsilane (TMS) as an internal standard substance, and setting TMS resonance to 0.00 ppm. All chemical shifts were in ppm on the delta scale (δ) and signal microfission was indicated using abbreviations (s: singlet, d: doublet, t: triplet, m: multiplet, br: broad).

In mass spectrometry, LCMS 2020 (manufactured by SHIMADZU CORPORATION) was used when MS was performed, and LCMS-IT-TOF (manufactured by SHIMADZU CORPORATION) was used when HRMS was performed.

### [Examples 1-1 to 1-4 and Comparative Examples 1-1 to 1-3]

In the examples, two compounds (PSMA-DA1 and PSMA-DB) whose target molecule is PSMA were synthesized. Next, radiolabeled compounds were obtained, in which each of the compounds was coordinated with a ¹¹¹In ion, a ⁹⁰Y ion, or a ²²⁵Ac ion as a radioactive metal. Details are described below.

PSMA-DA1, used in Examples 1-1 to 1-4, has a chemical structure linearly including a PSMA molecule-binding part and an albumin-binding part through a chelating part, as represented by the general formula (1). In PSMA-DA1, the chelating part and the PSMA molecule-binding part are directly bonded to each other by an amide bond, and the chelating part and the atomic group containing the albumin-binding part are indirectly bonded to each other through a lysine-derived linker structure.

PSMA-DB, used in Comparative Examples 1-1 to 1-3, has a structure containing a chelating part and a PSMA molecule-binding part, but does not contain an albumin-binding part.

### <Examples 1-1 to 1-4>

The outlines of synthesis routes in Examples 1-1 to 1-4 are defined as synthesis routes (V-1) and (V-2), and are shown below.

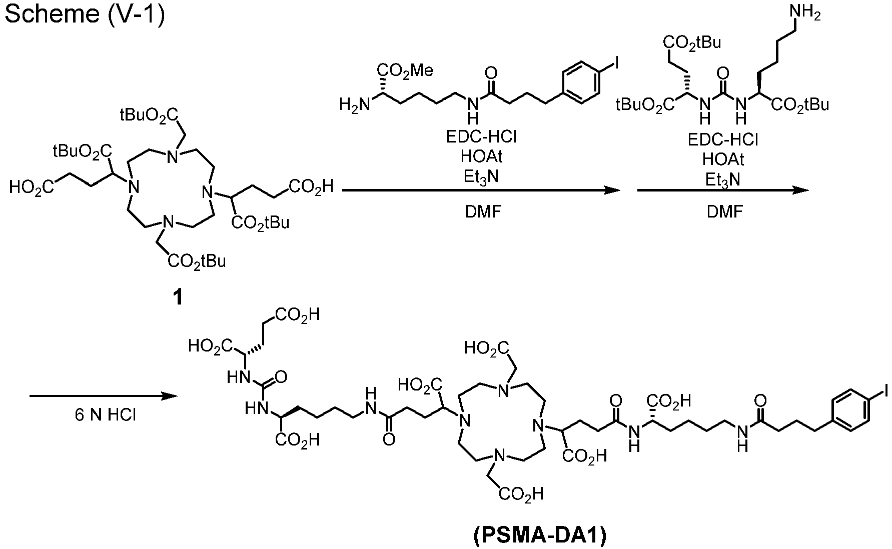

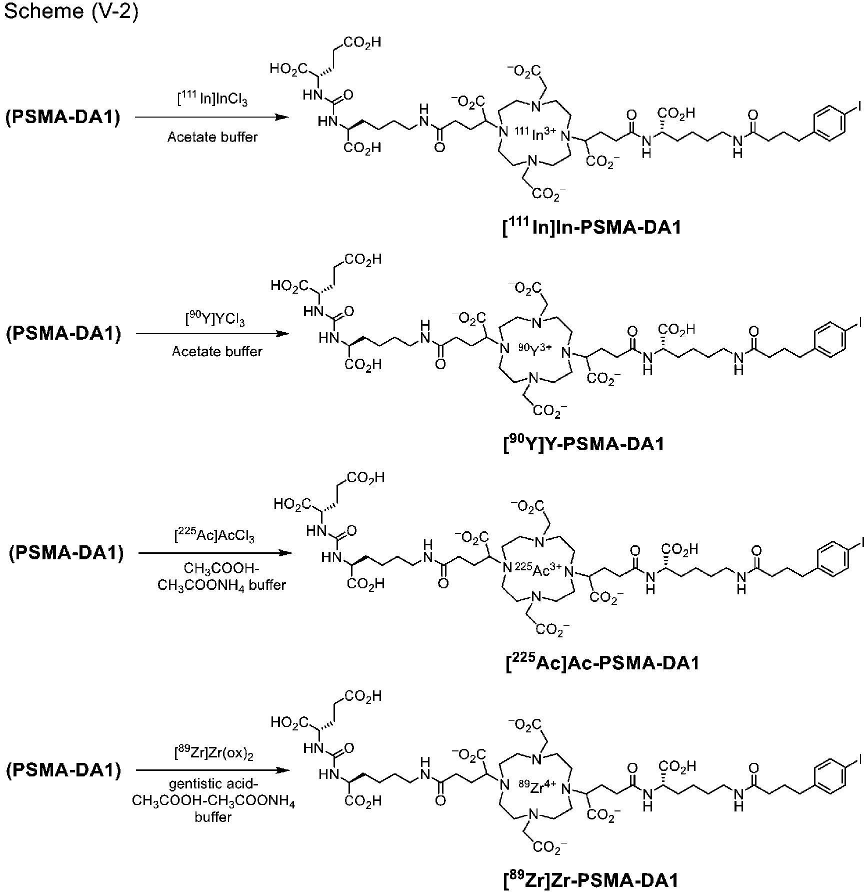

### (1) Synthesis of PSMA-DA1 (Compound 1)

Compound 1 was synthesized from 1,4,7,10-tetraazacyclododecane in 3 steps according to the method of Chem Commun. 2008, 28, 3248-3250. This compound 1 (20 mg, 0.026 mmol) was dissolved in N,N-dimethylformamide (DMF) (2 mL), and methyl-N⁶-(4-(4-iodophenyl)butanoyl)-L-lysinate (11 mg, 0.0254 mmol), 1-ethyl-3-(3-dimethylaminopropyl)-carbodiimide (EDC) hydrochloride (7.0 mg, 0.037 mmol), 1-hydroxy-7-azabenzotriazole (HOAt) (5.0 mg, 0.037 mmol), and triethylamine (5 µL, 0.036 mmol) were added thereto, and then the mixture was stirred at room temperature for 24 hours. Thereafter, (S)-di-tert-butyl 2-(3-((S)-6-amino-1-tert-butoxy-1-oxohexan-2-yl)ureido) pentanedioate (13 mg, 0.0267 mmol), EDC hydrochloride (7.0 mg, 0.037 mmol), HOAt (5.0 mg, 0.037 mmol) and triethylamine (5 µL, 0.036 mmol) were added thereto, and the mixture was stirred at room temperature for 72 hours. After removing the solvent, 6 N hydrochloric acid (3 mL) was added to the residue, the mixture was stirred at 40°C for 24 hours, and then purified by reverse phase HPLC under the following conditions to obtain the desired compound (PSMA-DA1). The yield and MS were as follows.

Purification conditions: Cosmosil 5C₁₈-AR-II column (10 × 250 mm); mobile phase: MeCN/H₂O/trifluoroacetic acid (TFA) [10/90/0.1 (0 min) to 100/0/0.1 (90 min)]; flow rate: 4 mL/min.

Recovery amount: 1.0 mg (Yield: 3%; calculated from the amount of substance of PSMA-DA1 obtained relative to the amount of substance of compound 1).

MS(ESI): m/z1250.5[M+H]⁺.

### (2) ¹¹¹In labeling (Example 1-1)

A solution (3.7 MBq, 100 µL) of [¹¹¹In]InCl₃ and a DMSO solution of PSMA-DA1 (1 mM, 10 µL) were added to an acetate buffer (0.1 M, pH 5.5, 200 µL), and the mixture was allowed to stand at 90°C for 30 minutes. Thereafter, the reaction solution was purified by reverse phase HPLC under the following conditions to obtain the desired radiolabeled compound ([¹¹¹In]In-PSMA-DA1).

The radioactivity of the obtained radiolabeled compound was measured with a Curie meter, and the percentage to the radioactivity of the solution of [¹¹¹In]InCl₃ used in the reaction was defined as radiochemical yield (%).

In addition, a part of the HPLC preparative liquid of the radiolabeled compound was analyzed under the same HPLC conditions as the purification conditions, and the percentage of the area value of the radiolabeled compound to the area value of all detected peaks was defined as radiochemical purity (%).

As a result, the radiochemical yield was 61 to 90%, and the radiochemical purity was 95% or more.

Purification conditions for ¹¹¹In labeling: Cosmosil 5C₁₈-PAQ column (4.6 × 250 mm); mobile phase: MeCN/H₂O/TFA [20/80/0.1 (0 min) to 50/50/0.1 (30 min) or 5/95/0.1 (0 to 10 min), 5/95/0.1 (10 min) to 35/65/0.1 (40 min)]; flow rate: 1 mL/min.

The compound in which PSMA-DA1 is coordinated to non-radioactive In can be produced, for example, by the following method. The obtained In complex was used to identify the HPLC retention time of the radiolabeled compound.

PSMA-DA1 (1 mg) and indium (III) chloride anhydrous (2 mg) were dissolved in dimethyl sulfoxide (DMSO) (100 µL), and 2-(N-morpholino) ethanesulfonic acid buffer (0.1 M, pH 5.6, 900 µL) was added. The reaction solution was stirred at 60°C for 12 hours, and then the solution was purified through reverse phase HPLC according to the following method to obtain a compound having the following MS.

Purification conditions: Cosmosil 5C₁₈-AR-II column (10 × 250 mm); mobile phase: MeCN/H₂O/TFA [10/90/0.1 (0 min) to 100/0/0.1 (90 min)]; flow rate: 4 mL/min.

MS(ESI): m/z1362.4[M+H]⁺.

### (3) ⁹⁰Y labeling (Example 1-2)

A solution (65-118 MBq, 10 µL) of [⁹⁰Y]YCl₃ and a DMSO solution of PSMA-DA1 (1 mM, 10 µL) were added to an acetate buffer (0.1 M, pH 5.5, 200 µL), and the mixture was allowed to stand at 90°C for 30 minutes. Thereafter, the reaction solution was purified by reverse phase HPLC under the following conditions to obtain the target radiolabeled compound ([⁹⁰Y]Y-PSMA-DA1).

The radiochemical yield was measured in the same manner as in Example 1-1. As the HPLC conditions used for the analysis of radiochemical purity, the following purification conditions for ⁹⁰Y, labeling were used.

As a result, the radiochemical yield was 49 to 79%, and the radiochemical purity was 95% or more.

Purification conditions for ⁹⁰Y labeling: Cosmosil 5C₁₈-PAQ column (4.6 × 250 mm); mobile phase: MeCN/H₂O/TFA [20/80/0.1 (0 min) to 50/50/0.1 (30 min) or 5/95/0.1 (0 to 10 min), 5/95/0.1 (10 min) to 35/65/0.1 (40 min)]; flow rate: 1 mL/min.

### (4) ²²⁵Ac labeling (Example 1-3)

To a 0.2 M hydrochloric acid solution (1.5 MBq, 10 µL) of [²²⁵Ac]AcCl₃, a 0.1 M acetic acid-ammonium acetate buffer solution (pH 5.5, 170 µL) and a DMSO solution (2.0 mM, 10 µL) of PSMA-DA1 were added, and the mixture was allowed to stand at 70°C for 1 hour. H₂O (800 µL) was added to the reaction solution, and the mixture was passed through an Oasis HLB Light column. H₂O (10 mL) was passed through the column, and then 70% EtOH (0.5 mL) was passed through the column to obtain a purified solution. The purified solution was heated and distilled off to dryness, and then a 158 mM acetic acid-sodium acetate buffer containing 5% ethanol (pH 6.5) was added to obtain a solution for administration of the target radiolabeled compound ([²²⁵Ac]Ac-PSMA-DA1).

The radiochemical yield was measured by the following method. The radioactivity of the obtained radiolabeled compound was measured with a gamma ray spectrometer, and the percentage to the radioactivity of the solution of [²²⁵Ac]AcCl₃ used in the reaction was defined as radiochemical yield (%).

The radiochemical purity of the obtained radiolabeled compound was measured by the following method. That is, a part of the solution of the radiolabeled compound was analyzed by TLC (iTLC-SG; mobile phase: mixed solution of MeCN/H₂O = 1 : 1), and the percentage of the area value of the radiolabeled compound to the area value of all detected peaks was defined as radiochemical purity (%).

As a result, the radiochemical yield was 49%, and the radiochemical purity was 87%.

### (5) ⁸⁹Zr labeling (Example 1-4)

A 1.0 M hydrochloric acid solution (2.2 MBq, 10 µL) of [⁸⁹Zr]Zr(Ox)₂ was dispensed into a TypeI Plus vial (2R) (manufactured by SCHOTT) and heated to 110°C, and the solvent was distilled off under an Ar gas flow for about 40 minutes. To the vial were added 0.1 mol/L hydrochloric acid (100 µL), 300 mM gentisic acid-0.78 M acetic acid-sodium acetate buffer (pH 5.5, 50 µL), a PSMA-DA1-DMSO solution (2 mM, 75 µL), and DMSO (75 µL), and the vial was allowed to stand at 70°C for 1 hour to obtain the target radiolabeled compound ([⁸⁹Zr]Zr-PSMA-DA1).

The radiochemical purity of the obtained radiolabeled compound was measured in the same manner as in Example 1-3. As a result, the radiochemical purity was 96%.

### <Comparative Examples 1-1 to 2-3>

The outlines of synthesis routes in Comparative Examples 1-1 to 2-3 are defined as synthesis routes (VI-1) and (VI-2), and are shown below.

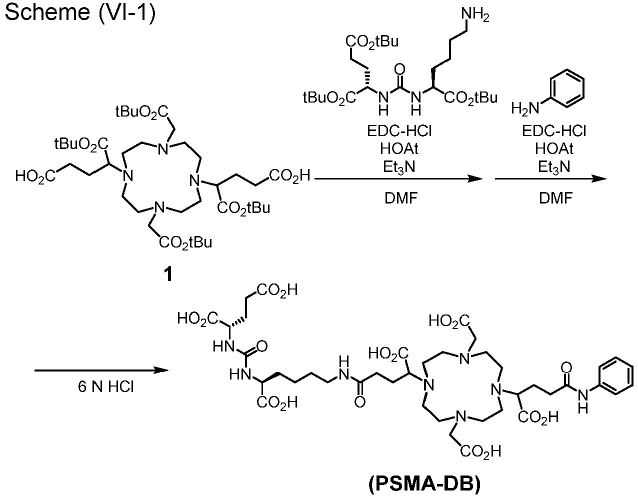

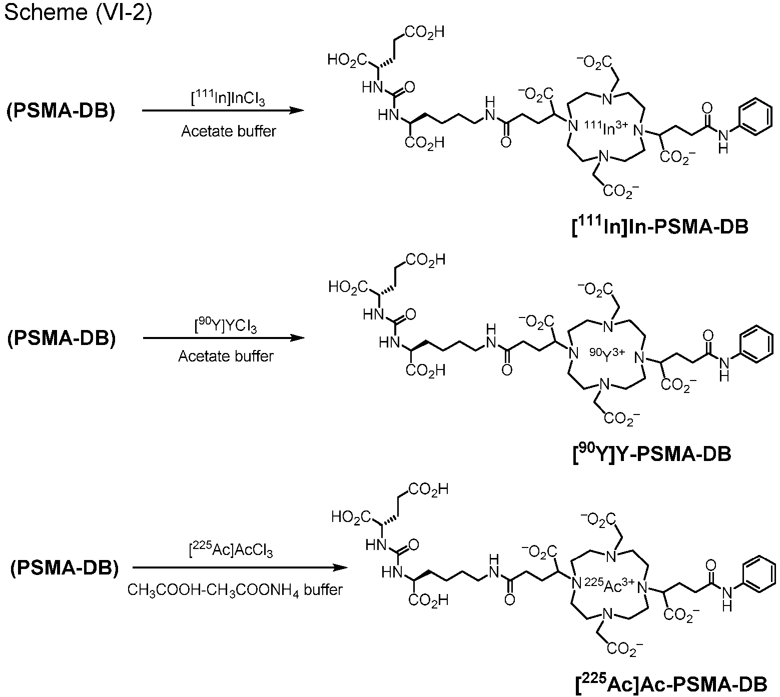

### (1) Synthesis of PSMA-DB

Compound 1 (35 mg, 0.045 mmol) synthesized in the same manner as in Examples 1-1 to 1-4 was dissolved in DMF (2 mL). Then (S)-di-tert-butyl 2-(3-((S)-6-amino-1-tert-butoxy-1-oxohexan-2-yl)ureido) pentanedioate (22 mg, 0.045 mmol), EDC hydrochloride (10 mg, 0.052 mmol), HOAt (7.0 mg, 0.051 mmol), and triethylamine (7 µL, 0.050 mmol) were added thereto, and the mixture was stirred at room temperature for 24 hours. Thereafter, aniline (5 µL, 0.055 mmol), EDC hydrochloride (10 mg, 0.052 mmol), HOAt (7.0 mg, 0.051 mmol), and triethylamine (7 µL, 0.050 mmol) were added thereto, and the mixture was stirred at room temperature for 24 hours. After the solvent was removed, TFA (1.8 mL), triisopropylsilane (100 µL), and H₂O (100 µL) were added to the residue, and the mixture was stirred at room temperature for 24 hours. After removing the solvent, the residue was purified by reverse phase HPLC under the following conditions to obtain the desired compound (PSMA-DB). The yield and MS were as follows.

Purification conditions: Cosmosil 5C₁₈-AR-II column (10 × 250 mm); mobile phase: MeCN/H₂O/TFA [5/95/0.1 (0 to 10 minutes), 5/95/0.1 (10 minutes) to 35/65/0.1 (40 minutes)]; flow rate: 4 mL/min.

Recovery amount: 5.0 mg (Yield: 12%; calculated from the amount of substance of PSMA-DB obtained relative to the amount of substance of compound 1).

MS(ESI)m/z925.3[M+H]⁺.

### (2) ¹¹¹In labeling (Comparative Example 1-1)

The desired radiolabeled compound ([¹¹¹In]In-PSMA-DB) was obtained in the same manner as in Example 1-1 except that PSMA-DB was used instead of PSMA-DA1.

The radiochemical yield and the radiochemical purity were measured in the same manner as described in Example 1-1. As the purification conditions and HPLC conditions used to measure the radiochemical purity, the following conditions were used.

As a result, the radiochemical yield was 61 to 90%, and the radiochemical purity was 95% or more.

The compound in which PSMA-DB is coordinated to non-radioactive In can be produced, for example, by the following method. The obtained In complex was used to identify the HPLC retention time of the radiolabeled compound.

A solution of PSMA-DB (1 eq) in H₂O/MeCN/TFA (49.95/49.95/0.1, 300 µL) was added with indium (III) chloride anhydrous (10 eq). After stirring at room temperature for 18 hours, the solution was purified by reverse phase HPLC.

Purification conditions: Cosmosil 5C₁₈-AR-II column (4.6 × 150 mm); mobile phase: MeCN/H₂O/TFA [5/95/0.1 (0 to 10 minutes), 5/95/0.1 (10 minutes) to 35/65/0.1 (40 minutes)]; flow rate: 1 mL/min.

### (3) ⁹⁰Y, labeling (Comparative Example 1-2)

The desired radiolabeled compound ([⁹⁰Y]Y-PSMA-DB) was obtained in the same manner as in Example 1-2 except that PSMA-DB was used instead of PSMA-DA1.

The radiochemical yield and radiochemical purity were measured in the same manner as described in Example 1-1. The radiochemical purity was measured under the HPLC conditions shown in the purification conditions of the above-described compound in which PSMA-DB was coordinated to non-radioactive In.

As a result, the radiochemical yield was 49 to 79%, and the radiochemical purity was 95% or more.

### (4) ²²⁵Ac labeling (Comparative Example 1-3)

The desired radiolabeled compound ([²²⁵Ac]Ac-PSMA-DB) was obtained in the same manner as in Example 1-3 except that PSMA-DB was used instead of PSMA-DA1.

The radiochemical yield and radiochemical purity were measured in the same manner as described in Example 1-3.

As a result, the radiochemical yield was 48%, and the radiochemical purity was 83%.

### <Evaluation of stability in plasma>

A saline (20 µL) solution of [¹¹¹In]In-PSMA-DA1 (370 kBq) or [⁹⁰Y]Y-PSMA-DA1 (3.7 MBq) was added to mouse plasma (200 µL), and the mixture was allowed to stand at 37°C for 24 hours (n = 3). Then, MeCN (200 µL) was added, and the mixture was centrifuged at 10,000 × g for 5 minutes. The supernatant was filtered, and the filtrate was analyzed by reverse phase HPLC under the following conditions.
(Analytical conditions: Cosmosil 5C₁₈-PAQ column (4.6 × 250 mm); mobile phase: MeCN/H₂O/TFA [20/80/0.1 (0 min) to 50/50/0.1 (30 min)]; flow rate: 1 mL/min.)
As a result, 95% or more of all the labeling compounds were stably present in mouse plasma even after standing at 37°C for 24 hours.

### <Binding assay using cultured cells>

LNCaP cells (PSMA-positive, human prostate cancer) and PC-3 cells (PSMA-negative, human prostate cancer) were used. These cells were purchased from American Type Culture Collection and DS Biomedical, respectively. Each of the cells were cultured in RPMI 1640, which is manufactured by NACALAI TESQUE, INC. and contains antibiotics (penicillin and streptomycin) and 10% inactivated fetal bovine serum, at 37°C under 5% CO₂.

The LNCaP cells and PC-3 cells were each seeded on a 12 well plate at 4.0 × 10⁵ cells/well, and left standing at 37°C under 5% CO₂ for 48 hours.

The culture medium was removed, and an assay medium (0.5% FBS-containing RPMI 1640 medium) solution (1 mL) containing [¹¹¹In]In-PSMA-DA1 or [¹¹¹In]In-PSMA-DB (37 kBq) was added. Thereafter, the plate was allowed to stand at 37°C under 5% CO₂ for 1 hour.

In the inhibition assays, after removing the culture medium, an assay medium (1 mL) solution containing: [¹¹¹In]In-PSMA-DA1 or [¹¹¹In]In-PSMA-DB (37 kBq); and 2-(phosphonomethyl) pentanedioic acid (2-PMPA) (PSMAinhibitor; final concentration: 100 µM) was added. Thereafter, the plate was allowed to stand at 37°C under 5% CO₂ for 1 hour.

After removing the assay medium, each well was washed with an assay medium (1 mL) containing neither the radiolabeled compound nor 2-PMPA, and the cells were lysed with 1 N aqueous sodium hydroxide solution (200 µL × 2).

The radioactivity for each of the assay medium and the cell lysate was measured with a gamma counter. Separately, the total protein concentration in the cell lysate was calculated using BCA Protein Assay Kit, which is manufactured by Thermo Fisher Scientific K.K. The value (% ID/mg protein) obtained by dividing the percentage (% ID) of the sample's radioactivity amount to the added radioactivity amount by the total protein amount was calculated for each sample.

The data were expressed as mean value ± standard deviation. The significant difference test was performed using Student's t-test and one-way analysis of variance (ANOVA) test with Dunnet's post-hoc test, and p < 0.05 was defined as having a significant difference.

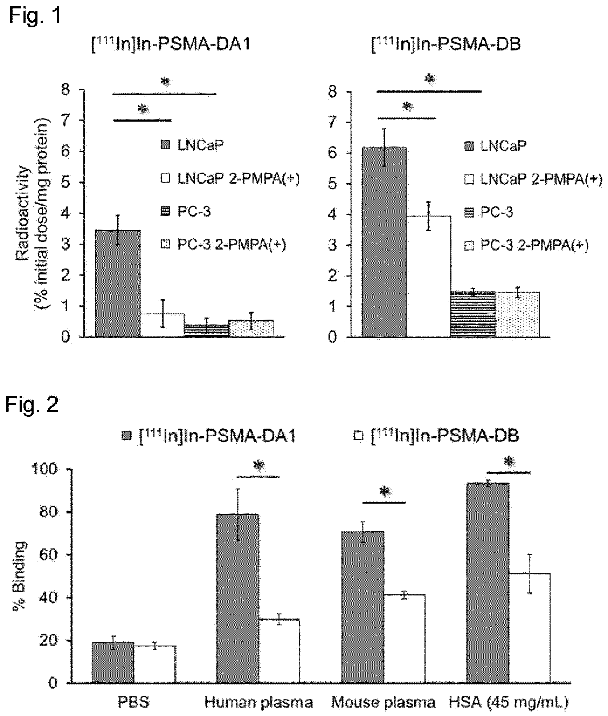

The results of the evaluation of binding to cultured cells are shown in Fig. 1. The higher the value, the higher the abundance of the radiolabeled compound, which indicates that the compound is highly accumulated.

[¹¹¹In]In-PSMA-DA1 and [¹¹¹In]In-PSMA-DB showed high binding ability to LNCaP cells compared to PC-3 cells and the binding was significantly reduced by adding an excess amount of the PSMA inhibitor (2-PMPA). These results showed that [¹¹¹In]In-PSMA-DA1 and [¹¹¹In]In-PSMA-DB specifically bind to highly PSMA-expressing cells.

### <Evaluation of binding to albumin>

A PBS solution (37 kBq, 50 µL) of [¹¹¹In]In-PSMA-DA1 or [¹¹¹In]In-PSMA-DB was added to 200 µL of PBS, mouse plasma, human plasma, or a human serum albumin (HSA) solution (45 mg/mL) in PBS, respectively, and the mixtures were allowed to stand at 37°C for 10 minutes. Thereafter, the reaction liquids were added to a spin column (Sephadex G-100; manufactured by Cytiva), and centrifuged at 1500 × g at 4°C for 2 minutes. After separation, the radioactivity for each of the column and the eluate was measured with a gamma counter.

The data were expressed as mean value ± standard deviation. The significant difference test was performed using Student's t-test and one-way analysis of variance (ANOVA) test with Dunnet's post-hoc test, and p < 0.05 was defined as having a significant difference.

The results of the evaluation of binding to albumin are shown in Fig. 2. The higher the value, the higher the binding ability to albumin.

When a compound to be evaluated binds to albumin to form a composite, the compound passes through the column because of the increased molecular size. However, when it does not bind to albumin, it is retained on the gel in the column.

When [¹¹¹In]In-PSMA-DA1 and [¹¹¹In]In-PSMA-DB were left to stand in PBS and then applied to the column, no significant radioactivity was observed in the eluate. On the other hand, when left to stand in mouse plasma, human plasma, and the HSA solution, the radioactivity in the eluate of [¹¹¹In]In-PSMA-DA1 was significantly higher than that of [¹¹¹In]In-PSMA-DB, indicating that [¹¹¹In]In-PSMA-DA1 binds to plasma albumin.

### <Evaluation of internal radioactivity distribution using LNCaP or PC-3 tumor-transplanted mouse>

The animal test was performed in compliance with the guidelines of Kyoto University Animal Experiment Committee. Male CBI7/IcrJcl-Prkdc^{scid} mice were purchased from CLEAJapan, Inc. The animals were kept under 12 h/12 h, day/night cycle conditions and fed ad libitum with food and water. LNCaP cells (1.0 × 10⁷ cells/mouse) or PC-3 cells (1.0 × 10⁷ cells/mouse) were suspended in a mixture of PBS and Matrigel manufactured by Corning Life Sciences (1 : 1, 150 µL) and the suspension was subcutaneously transplanted to the right shoulder of mice under isoflurane anesthesia. Thereafter, the mice were kept for 40 to 60 days.

LNCaP or PC-3 tumor-implanted mice were administered saline solutions (185 kBq, 100 µL) of [¹¹¹In]In-PSMA-DA1 or [¹¹¹In]In-PSMA-DB from the tail vein (n = 3 each). Mice were euthanized 1, 4, 24, 48, 96, and 192 hours after administration. Thereafter, blood and each organ were recovered, and the mass and radioactivity of the organs were measured.

The percentage (% ID) of the radioactivity amount to the administered radioactivity amount (injected dose) is shown as the value (% ID/g) divided by the blood mass or the organ mass (g). The higher the value of %ID/g, the higher the abundance of the radiolabeled compound, which indicates that the compound is highly accumulated at the target organ.

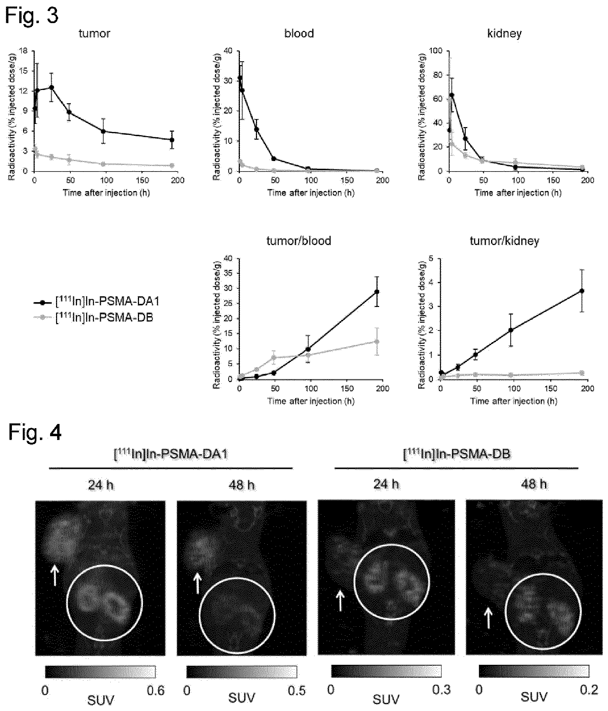

The results (Mean value ± standard deviation, each n = 3) in LNCaP tumor-transplanted mice and [¹¹¹In]In-PSMA-DA1 are shown in Table 1 and Fig. 3 below.

The results (Mean value ± standard deviation, each n = 3) in LNCaP tumor-transplanted mice and [¹¹¹In]In-PSMA-DB are shown in Table 2 and Fig. 3 below.

[¹¹¹In]In-PSMA-DA1 showed high accumulation at the LNCaP tumor (9.41 to 12.6% ID/g; 1 to 24 hours after administration). In addition, retention in blood was shown (14.0% ID/g; 24 hours after administration), and 48 hours after administration, the tumor/kidney ratio exceeded 1. On the other hand, [¹¹¹In]In-PSMA-DB less accumulated at the tumor than [¹¹¹In]In-PSMA-DA1 at any time point, showing lower tumor/kidney ratio.

From these results, it has been clear that [¹¹¹In]In-PSMA-DA1 highly accumulates at a highly PSMA-expressing tumor and exhibits a more excellent internal distribution than that of [¹¹¹In]In-PSMA-DB.

**[Table 1]**

| [¹¹¹In]In-PSMA-DA1 | 1 h | 4 h | 24 h | 48 h | 96 h | 192 h |
|---|---|---|---|---|---|---|
| Blood | 31.1 ± 3.99 | 26.9 ± 9.56 | 14.0 ± 3.27 | 4.18 ± 0.56 | 0.76 ± 0.58 | 0.16 ± 0.03 |
| Spleen | 8.56 ± 2.90 | 9.52 ± 4.28 | 6.01 ± 2.33 | 4.28 ± 0.79 | 2.42 ± 1.54 | 2.69 ± 0.83 |
| Pancreas | 3.34 ± 0.40 | 2.48 ± 1.03 | 2.12 ± 0.49 | 1.06 ± 0.12 | 0.64 ± 0.24 | 0.46 ± 0.08 |
| Stomach (% ID) | 0.52 ± 0.02 | 0.51 ± 0.05 | 0.40 ± 0.09 | 0.25 ± 0.05 | 0.08 ± 0.03 | 0.05 ± 0.01 |
| Intestine | 2.63 ± 0.42 | 3.17 ± 0.73 | 1.53 ± 0.44 | 0.96 ± 0.25 | 0.75 ± 0.44 | 0.12 ± 0.03 |
| Kidney | 34.3 ± 7.79 | 63.5 ± 13.9 | 27.1 ± 9.25 | 9.04 ± 2.70 | 3.51 ± 2.54 | 1.33 ± 0.42 |
| Liver | 4.95 ± 0.27 | 4.20 ± 1.51 | 2.64 ± 0.38 | 1.59 ± 0.14 | 1.10 ± 0.27 | 0.69 ± 0.12 |
| Heart | 7.93 ± 2.10 | 7.10 ± 1.82 | 4.26 ± 0.89 | 1.66 ± 0.23 | 0.75 ± 0.33 | 0.41 ± 0.08 |
| Lung | 16.2 ± 3.86 | 17.9 ± 6.62 | 10.5 ± 2.58 | 4.17 ± 0.26 | 1.55 ± 0.86 | 0.75 ± 0.22 |
| Brain | 0.56 ± 0.06 | 0.52 ± 0.15 | 0.36 ± 0.11 | 0.16 ± 0.01 | 0.07 ± 0.03 | 0.05 ± 0.01 |
| Muscle | 2.02 ± 0.17 | 2.15 ± 1.09 | 1.38 ± 0.39 | 0.54 ± 0.10 | 0.23 ± 0.09 | 0.15 ± 0.05 |
| LNCaP Tumor | 9.41 ± 2.30 | 12.1 ± 404 | 12.6 ± 2.15 | 8.82 ± 1.26 | 5.98 ± 1.87 | 4.69 ± 1.32 |

**[Table 2]**

| [¹¹¹In]In-PSMA-DB | 1 h | 4 h | 24 h | 48 h | 96 h | 192 h |
|---|---|---|---|---|---|---|
| Blood | 3.33 ± 0.21 | 2.00 ± 0.29 | 0.68 ± 0.17 | 0.27 ± 0.16 | 0.14 ± 0.04 | 0.07 ± 0.03 |
| Spleen | 3.05 ± 0.53 | 2.39 ± 0.05 | 3.37 ± 0.75 | 2.63 ± 0.83 | 2.82 ± 0.87 | 3.46 ± 0.32 |
| Pancreas | 0.74 ± 0.25 | 0.54 ± 0.22 | 0.71 ± 0.10 | 0.71 ± 0.17 | 0.85 ± 0.39 | 1.04 ± 0.18 |
| Stomach (% ID) | 0.10 ± 0.04 | 0.13 ± 0.03 | 0.16 ± 0.05 | 0.07 ± 0.02 | 0.10 ± 0.03 | 0.05 ± 0.00 |
| Intestine | 0.59 ± 0.14 | 1.05 ± 0.10 | 1.00 ± 0.44 | 0.79 ± 0.21 | 0.41 ± 0.17 | 0.13 ± 0.02 |
| Kidney | 59.2 ± 35.2 | 22.7 ± 9.69 | 13.2 ± 2.41 | 8.58 ± 2.02 | 7.04 ± 3.28 | 3.47 ± 1.24 |
| Liver | 0.91 ± 0.20 | 0.95 ± 0.05 | 1.55 ± 0.32 | 1.28 ± 0.16 | 1.59 ± 0.57 | 1.20 ± 0.09 |
| Heart | 0.87 ± 0.33 | 0.60 ± 0.08 | 0.43 ± 0.07 | 0.32 ± 0.05 | 0.40 ± 0.12 | 0.33 ± 0.01 |
| Lung | 2.24 ± 0.60 | 1.56 ± 0.07 | 0.97 ± 0.15 | 0.66 ± 0.24 | 0.70 ± 0.15 | 0.67 ± 0.05 |
| Brain | 0.06 ± 0.01 | 0.05 ± 0.00 | 0.07 ± 0.02 | 0.03 ± 0.02 | 0.09 ± 0.05 | 0.04 ± 0.00 |
| Muscle | 0.23 ± 0.68 | 0.21 ± 0.04 | 0.21 ± 0.07 | 0.15 ± 0.06 | 0.14 ± 0.08 | 0.14 ± 0.09 |
| LNCaP Tumor | 3.36 ± 0.04 | 2.50 ± 0.50 | 2.09 ± 0.39 | 1.69 ± 0.77 | 1.05 ± 0.03 | 0.83 ± 0.11 |

The results (Mean value ± standard deviation, each n = 3) in PC-3 tumor-transplanted mice and [¹¹¹In]In-PSMA-DA1 are shown in Table 3 below.

The results (Mean value ± standard deviation, each n = 3) in PC-3 tumor-transplanted mice and [¹¹¹In]In-PSMA-DB are shown in Table 4 below.

Both [¹¹¹In]In-PSMA-DA1 and [¹¹¹In]In-PSMA-DB less accumulated at the PC-3 tumor than the result of accumulation at the LNCaP tumor at the same time point, showing that both of the radiolabeled compounds selectively accumulate at the PSMA-positive tumor.

**[Table 3]**

| [¹¹¹In]In-PSMA-DA1 | 24 h |
|---|---|
| Blood | 5.73 ± 0.67 |
| Spleen | 2.52 ± 0.12 |
| Pancreas | 1.37 ± 0.04 |
| Stomach (% ID) | 0.27 ± 0.08 |
| Intestine | 1.37 ± 0.22 |
| Kidney | 9.56 ± 1.55 |
| Liver | 1.60 ± 0.12 |
| Heart | 1.90 ± 0.22 |
| Lung | 4.74 ± 0.67 |
| Brain | 0.17 ± 0.04 |
| Muscle | 0.59 ± 0.02 |
| PC-3 Tumor | 2.69 ± 0.30 |

**[Table 4]**

| [¹¹¹In]In-PSMA-DB | 1 h |
|---|---|
| Blood | 1.92 ± 0.27 |
| Spleen | 2.57 ± 0.39 |
| Pancreas | 0.44 ± 0.06 |
| Stomach (% ID) | 0.12 ± 0.04 |
| Intestine | 0.48 ± 0.04 |
| Kidney | 49.5 ± 12.6 |
| Liver | 0.62 ± 0.03 |
| Heart | 0.49 ± 0.05 |
| Lung | 1.36 ± 0.23 |
| Brain | 0.06 ± 0.00 |
| Muscle | 0.21 ± 0.08 |
| PC-3 Tumor | 0.61 ± 0.06 |

### <SPECT/CT using LNCaP tumor-transplanted mouse>

LNCaP tumor-implanted mice prepared by the method described above were administered saline solutions (1.9 to 3.0 MBq, 150 µL) of [¹¹¹In]In-PSMA-DA1 or [¹¹¹In]In-PSMA-DB from the tail vein. SPECT/CT was performed 24 and 48 hours after administration with FX3300 pre-clinical imaging system, which is manufactured by Gamma Medica-Ideas. Imaging was performed using a pinhole collimator with a diameter of 1.0 mm at a rotation radius of 35 mm, a projection time of 70 seconds, and the number of projections of 32 times under isoflurane anesthesia. After SPECT, CT (Tube voltage: 60 kV, Tube current: 350 µA) was performed. Image reconstruction was performed on the projection data of SPECT through three-dimensional ordered subset expectation maximization method (8 subsets, 5 iterations).

SPECT/CT results are shown in Figure 4. In the figure, the part indicated by the arrow is the location of the tumor, and the part indicated by the circle is the location of the kidney. The higher the SUV, the higher the radioactivity accumulation.

In SPECT/CT imaging using [¹¹¹In]In-PSMA-DA1, significant radioactivity accumulation was observed at the LNCaP tumor (the arrow in the figure) 24 and 48 hours after administration. High radioactivity accumulation was also observed at the kidney (the circle in the figure), but the radioactivity signal was almost the same as at the tumor 48 hours after administration. On the other hand, in SPECT/CT imaging using [¹¹¹In]In-PSMA-DB, radioactivity accumulation was also observed at the LNCaP tumor (the arrow in the figure), but the accumulation was lower than at the kidney (the circle in the figure).

From these results, it has been indicated that [¹¹¹In]In-PSMA-DA1 can clearly draw the highly PSMA-expressing tumor through SPECT and is superior to [¹¹¹In]In-PSMA-DB.

### <Evaluation of suppressing tumor growth by ⁹⁰Y-labeling compound>

LNCaP tumor-implanted mice were administered saline solutions (100 µL) of [⁹⁰Y]Y-PSMA-DA1 (3.7 MBq) or [⁹⁰Y]Y-PSMA-DB (3.7 MBq) obtained by the method described above from the tail vein (n = 7). As the control group, LNCaP tumor-implanted mice were administered 100 µL of saline from the tail vein (n = 7).

After administration of the ⁹⁰Y-labeling compound, the tumor volume and body weight were measured 3 times per week. The tumor volume was calculated based on the calculation formula "(tumor volume) = [(long side) × (short side)²/2]".

The tumor volumes on the day when administration of the ⁹⁰Y-labeling compound was started were 63.1 ± 8.7, 66.1 ± 30.7, and 66.7 ± 25.7 mm³ for the groups administered [⁹⁰Y]Y-PSMA-DA1, [⁹⁰Y]Y-PSMA-DB, and saline, respectively.

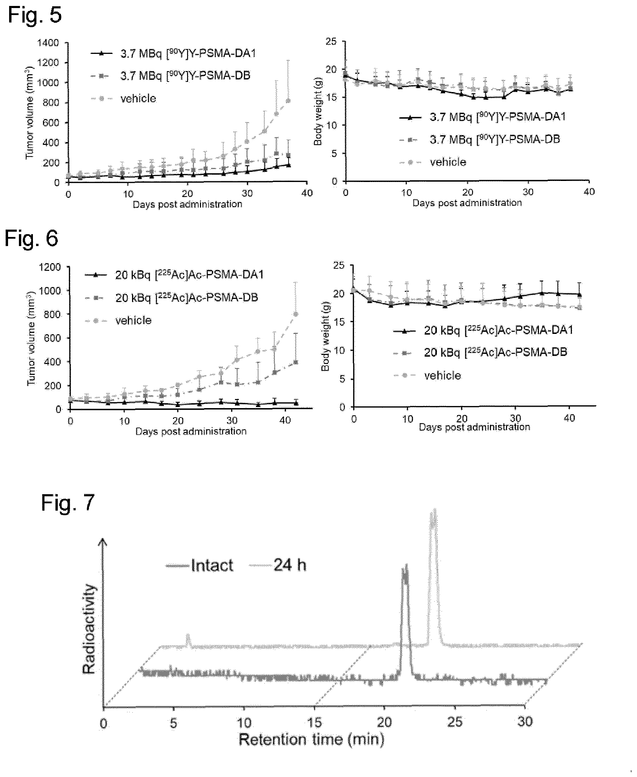

The results of this evaluation are shown in Fig. 5.

When the LNCaP tumor-transplanted mice were administered [⁹⁰Y]Y-PSMA-DA1 and [⁹⁰Y]Y-PSMA-DB, a significant difference in tumor volume was observed 9 days and 33 days after administration, respectively, as compared with the saline-administered group. Compared to [⁹⁰Y]Y-PSMA-DB, the tendency that [⁹⁰Y]Y-PSMA-DA1 suppresses tumor growth was observed. The body weight of the mice was slightly reduced by administration of [⁹⁰Y]Y-PSMA-DA1, but then recovered to a value equivalent to that of the saline-administered group.

### <Evaluation of suppressing tumor growth by ²²⁵Ac-labeling compound>

[²²⁵Ac]Ac-PSMA-DA1 (20 kBq) or ^{[225}Ac]Ac-PSMA-DB (20 kBq) obtained by the above method was dissolved in 5% ethanol-containing acetate buffer (158 mM, pH 6.5, 100 µL), and the solution was administered to LNCaP tumor-transplanted mice from the tail vein (n = 6 or 5). As the control group, 100 µL of 5% ethanol-containing acetate buffer (158 mM, pH 6.5) was administered to LNCaP tumor-transplanted mice from the tail vein (n = 4).

After administration of the ²²⁵Ac-labeling compound, the tumor volume and body weight were measured 2 times per week. The tumor volumes on the day when administration of ²²⁵Ac-labeling compound was started were 75.3 ± 26.0, 80.6 ± 20.8, and 91.1 ± 15.9 mm³ for the groups administered [²²⁵Ac]Ac-PSMA-DA1, [²²⁵Ac]Ac-PSMA-DB, and 5% ethanol-containing acetate buffer, respectively.

The results of this evaluation are shown in Fig. 6.

When the LNCaP tumor-transplanted mice were administered [²²⁵Ac]Ac-PSMA-DA1 and [²²⁵Ac]Ac-PSMA-DB, tumor growth was significantly suppressed as compared with the saline-administered group. In particular, in [²²⁵Ac]Ac-PSMA-DA1-administered group, the tumor hardly grew, and growth suppression was continuously observed until 6 weeks after administration.

The body weight reduction likely due to an influence of [²²⁵Ac]Ac-PSMA-DA1 administration was transient. The body weight reduction likely due to an influence of [²²⁵Ac]Ac-PSMA-DB administration was temporarily accelerated, but there was no sign of recovery thereafter.

### [Reference Example 2, useful to understand the invention]

In this Reference Example, synthesized was a compound (PtDA) that has a structure containing (((S)-5-amino-1-carboxypentyl)carbamoyl)-L-glutamic acid (in the formula (C1), "a" is 2, and "b" is 4.), whose target molecule is PSMA, as the PSMA molecule-binding part, and utilizes a click reaction between an azide group and dibenzocyclooctyne (DBCO) to bond the chelating part and the PSMA molecule-binding part. Next, radiolabeled compounds were obtained respectively, in which these compounds were coordinated with a ¹¹¹In ion as a radioactive metal. The outline of the synthesis route is shown below as synthesis routes (VIII-1) to (VIII-4).

The compound used in Reference Example 2 has a structure containing the chelating part, the PSMA molecule-binding part, and the albumin-binding part. There are two synthesis routes (see synthesis route (VIII-4)) to coordinate with a ¹¹¹In ion, and the resulting radiolabeled compounds are the same in either route.

In PtDA, the chelating part and the PSMA molecule-binding part are indirectly bonded to each other through a chemical structure derived from the click reaction and a polyethylene glycol group, and the chelating part and the atomic group containing the albumin-binding part are indirectly bonded to each other through a lysine-derived linker structure.

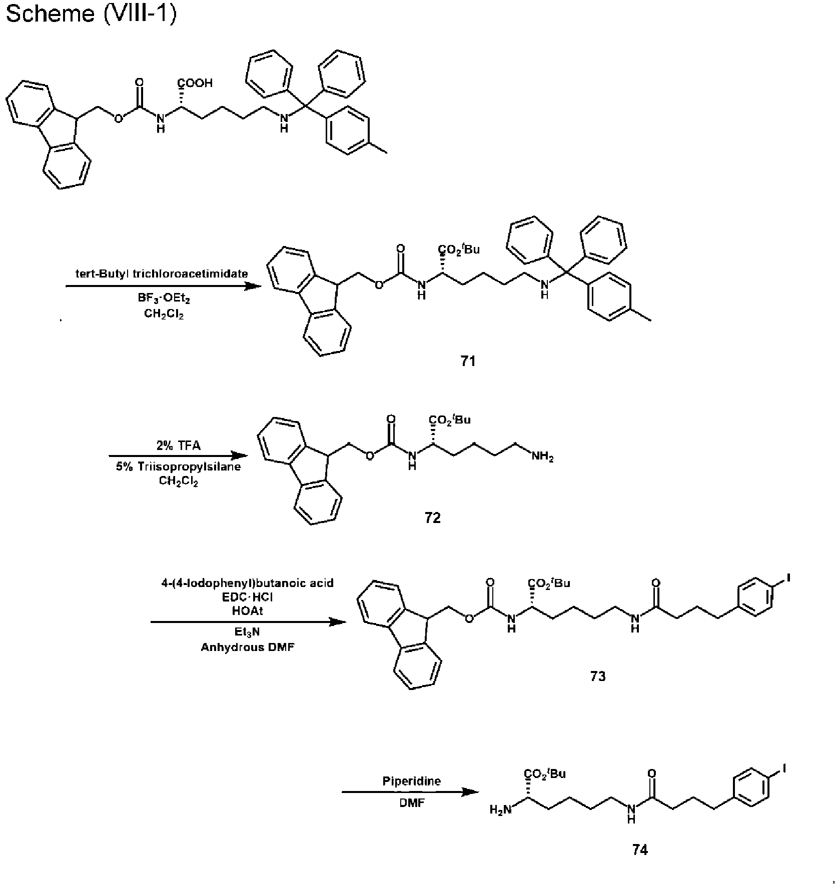

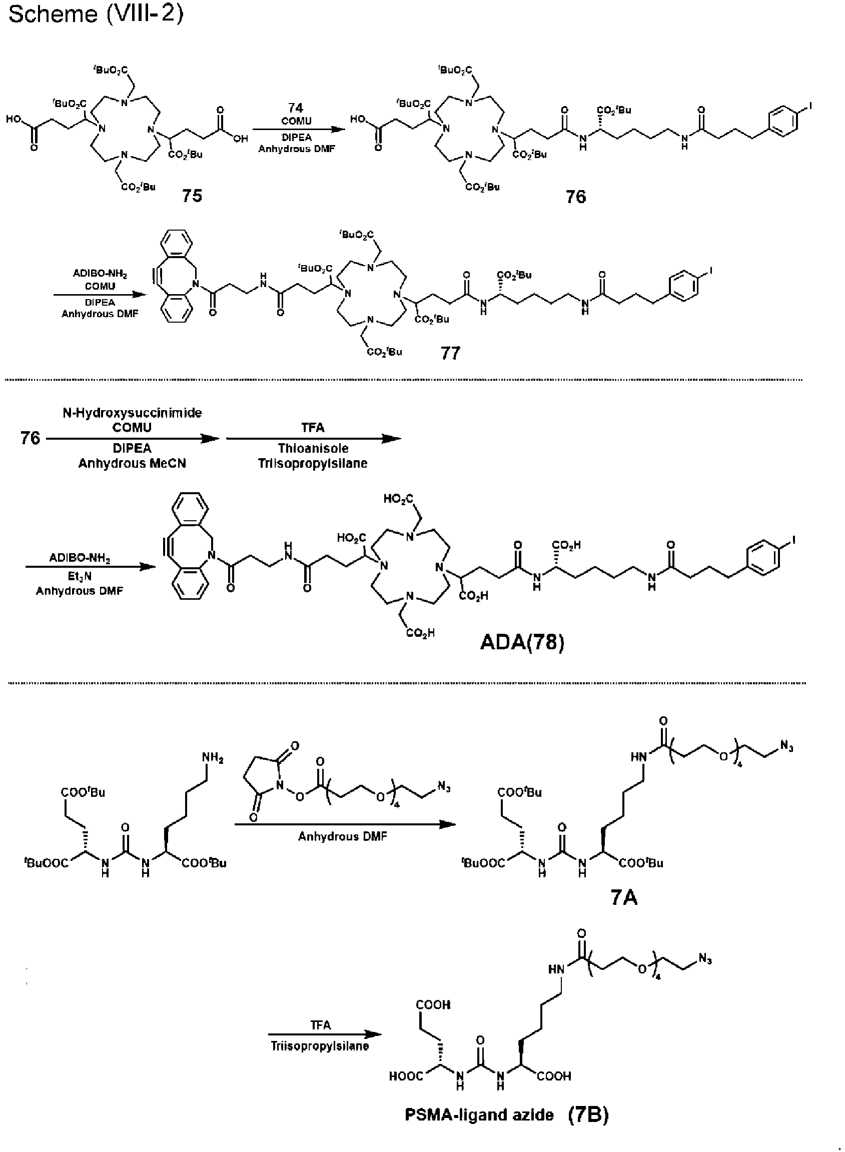

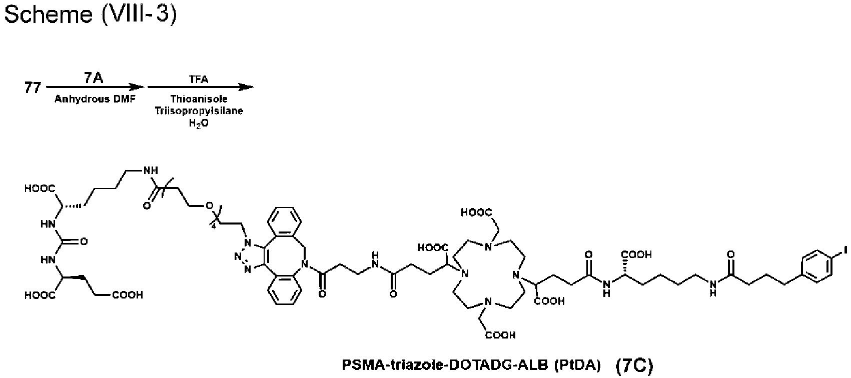

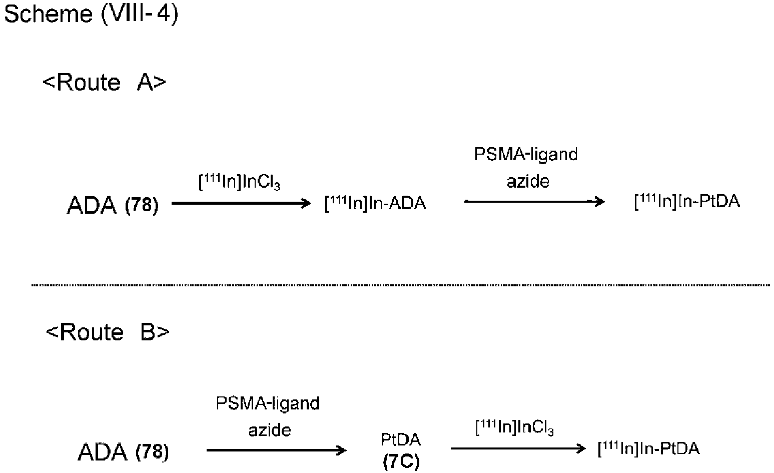

### <Synthesis of compound 71>

N²-[(9H-fluoren-9-ylmethoxy)carbonyl]-N⁶-[(4-methylphenyl)diphenylmethyl]-L-lysine (1000 mg, 1.6 mmol) was dissolved in dichloromethane (10 mL), and then tert-butyl trichloroacetimidate (699.5 mg, 3.2 mmol) and BF₃·OEt₂ (25 µL) were added thereto. The reaction solution was stirred at room temperature for 42 hours. After the solvent was removed, the residue was washed with H₂O (100 mL) and extracted with ethyl acetate/hexane (1/5, 100 mL × 2). The organic layer was dried with sodium sulfide and filtered. The filtrate was distilled off under reduced pressure, and then the residue was purified by medium-pressure column chromatography (ethyl acetate/hexane). The recovery amount, NMR spectrum, and MS were as follows.

Recovery amount: 569 mg (Yield: 52%; calculated from the amount of substance of Compound 71 obtained relative to the amount of substance of N²-[(9H-fluoren-9-ylmethoxy)carbonyl]-N⁶-[(4-methylphenyl)diphenylmethyl]-L-lysine).

¹H-NMR(400MHz,CDCl₃)δ7.69(d,J=7.3Hz,2H),7.56(d,J=7.8Hz,2H),7.45(d,J=7.8Hz,4H),7.3 3(d,J=8.2Hz,4H),7.23(m,6H),7.13(m,2H),7.04(d,J=7.8Hz,2H),5.39(d,J=8.2Hz,1H),4.35(d ,J=6.9Hz,2H),4.25(m,1H),4.18(t,J=6.9Hz,1H),2.26(s,3H),2.12(m,2H),1.77(m,1H),1.59(m, 1H),1.49(m,4H),1.44(s,9H₎. ¹³C-NMR(100MHz,CDCl₃)δ171.6,155.7,146.3(2C),143.7(2C),143.2,141.1(2C),135.4,128.4-128.3(8C),127.6-127.5(6C),126.9(2C),126.0(2C),125.0(2C),119.8(2C),81.7,70.5,66.7,60.2,47.1,43.2,32.7,3 0.4,27.8(3C),22.8,20.7.

HRMS(ESI):m/z681.3677[M+H]⁺.

### <Synthesis of compound 72>

Compound 71 (569 mg, 0.84 mmol) was dissolved in a mixture of trifluoroacetic acid (TFA) (100 µL), triisopropylsilane (250 µL), and dichloromethane (4.65 mL), and the mixture was stirred at room temperature for 6 hours. The solvent was removed, and then the residue was purified by medium-pressure column chromatography (methanol/chloroform). The recovery amount NMR spectrum, and MS were as follows.

Recovery amount: 355 mg (Yield: 100%; calculated from the amount of substance of compound 72 obtained relative to the amount of substance of compound 71).

¹H-NMR(400MHz,CDCl₃)δ7.71(d,J=7.6Hz,2H),7.56(d,J=7.1Hz,2H),7.35(t,J=7.6Hz,2H),7.2 7(t,J=7.6Hz,2H),5.72(d,J=8.0Hz,1H),4.33(d,J=7.1Hz,2H),4.16(t,J=6.6Hz,2H),3.38(s,2H), 1.77-1.56(m,4H),1.43(s,9H),1.27-1.13(m,2H₎. ¹³C-NMR(100MHz,CDCl₃)δ171.5,156.2,143.6(2C),141.1(2C),127.6(2C),127.0(2C),125.0(2C ),119.8(2C),82.3,66.9,54.1,50.0,46.9,39.5,31.1,27.7(3C),26.8,22.1.

HRMS(ESI):m/z425.2437[M+H]⁺.

### <Synthesis of compound 73>

4-(4-iodophenyl) butanoic acid (364 mg, 1.25 mmol) was dissolved in N,N-dimethylformamide (DMF) (3 mL), then 1-ethyl-3-(3-dimethylaminopropyl)-carbodiimide (EDC) hydrochloride (320 mg, 1.7 mmol) and 1-hydroxy-7-azabenzotriazole (HOAt) (228 mg, 1.7 mmol) were added, and the mixture was stirred at 0°C for 15 minutes. Then, compound 72 (355 mg, 0.84 mmol) and triethylamine (169 mg, 1.7 mmol) were added, and the mixture was stirred at 0°C. After 15 hours, the reaction solution was washed with H₂O (100 mL), and extracted with ethyl acetate/hexane (1/5, 100 mL × 2). The organic layer was dried with sodium sulfide, and filtered. The filtrate was distilled off under reduced pressure, and then the residue was purified by medium-pressure column chromatography (ethyl acetate/hexane). The recovery amount, NMR spectrum, and MS were as follows.

Recovery amount: 226 mg (Yield: 39%; calculated from the amount of substance of compound 73 obtained relative to the amount of substance of compound 72).

¹H-NMR(400MHz,CDCl₃)δ7.73(d,J=7.3Hz,2H),7.60-7.50(m,6H),7.37(t,J=7.3Hz,2H),7.28(t,J=7.3Hz,2H),6.83(d,J=8.2Hz,2H),4.38-4.27(m,2H),4.25-4.16(m,2H),3.21-3.14(m,2H),2.48(m,2H),2.08(t,J=7.3Hz,2H),1.91-1.84(m,2H),1.73-1.57(m,2H),1.47-1.29(m, 13H).¹³C-NMR(100MHz,CDCl₃)δ172.8,171.5,156.0,143.5(2C),141.0-140.9(3C),137.1(2C),130.3(2C),127.5(2C),126.9(2C),124.9(2C),119.8(2C),90.8,82.0,66.8 ,53.9,46.9,38.9,35.4,34.4,32.1,28.6,27.9(3C),26.7,22.2.

HRMS(ESI):m/z697.2130[M+H]⁺.

### <Synthesis of compound 74>

Piperidine (1 mL) was added to a DMF (4 mL) solution of compound 73. After stirring at room temperature for 2 hours, the solution was washed with H₂O (100 mL) and extracted with ethyl acetate/hexane (1/5, 100 mL × 2). The organic layer was dried with sodium sulfide and filtered. The filtrate was distilled off under reduced pressure, and then the residue was purified by medium-pressure column chromatography (methanol/chloroform). The recovery amount, NMR spectrum, and MS were as follows.

Recovery amount: 133 mg (Yield: 87%; calculated from the amount of substance of compound 74 obtained relative to the amount of substance of compound 73).

¹H-NMR(400MHz,CDCl₃)δ7.58(d,J=8.2Hz,2H),6.92(d,J=8.2Hz,2H),3.31-3.27(m,1H),3.23(m,2H),2.58(m,2H),2.13(m,2H),1.96-1.88(m,2H),1.74-1.65(m,2H),1.56-1.48(m,2H),1.44(s,9H),1.43-1.40(m,2H₎. ¹³C-NMR(100MHz,CDCl₃)δ175.2,172.3,141.1,137.3(2C),130.5(2C),90.9,80.9,54.7,39.1,35.6, 34.6,34.3,29.2,28.0(3C),26.8,22.9.

HRMS(ESI):m/z475.1453[M+H]⁺.

### <Synthesis of compound 75>

As in the examples described above, compound 75 was synthesized in 3 steps from 1,4,7,10-tetraazacyclododecane (Chem Commun. 2008, 28, 3248-3250).

### <Synthesis of compound 76>

N-[1-(cyano-2-ethoxy-2-oxoethylideneaminooxy)dimethylamino (morpholino)] uronium hexafluorophosphate (COMU) (176 mg, 0.41 mmol) was added to a solution of compound 75 (317 mg, 0.41 mmol) in DMF (2 mL), and the mixture was stirred at 0°C for 15 minutes. N,N-diisopropylethylamine (DIPEA) (53 mg, 0.41 mmol) was added to the reaction solution, the mixture was further stirred at 0°C for 15 minutes, and then compound 74 (163 mg, 0.34 mmol) was added thereto. The mixture was stirred at room temperature for 12 hours, and then the solution was purified by reverse phase HPLC under the following conditions. The recovery amount and MS were as follows.

Purification conditions: Cosmosil 5C₁₈-AR-II column (20 × 250 mm); mobile phase: MeCN/H₂O/TFA [30/70/0.1 (0 min) to 90/10/0.1 (40 min)]; flow rate: 5 mL/min.

Recovery amount: 229 mg (Yield: 55%; calculated from the amount of substance of compound 76 obtained relative to the amount of substance of compound 75).

HRMS(ESI)m/z1229.6182[M+H]⁺.

### <Synthesis of compound 77>

COMU (147 mg, 0.34 mmol) was added to a solution of compound 76 (106 mg, 0.086 mmol) in DMF (0.6 mL), and the mixture was stirred at 0°C for 15 minutes. DIPEA (89 mg, 0.69 mmol) was added to the reaction solution, and the mixture was further stirred at 0°C for 15 minutes, and then dibenzocyclooctyneamine (ADIBO-NH₂) (36 mg, 0.22 mmol) was added thereto. The mixture was stirred at room temperature for 12 hours, and then the solution was purified by reverse phase HPLC under the following conditions. The recovery amount and MS were as follows.

Purification conditions: Cosmosil 5C₁₈-AR-II column (20 × 250 mm); mobile phase: MeCN/H₂O/TFA [20/80/0.1 (0 min) to 90/10/0.1 (35 min)]; flow rate: 5 mL/min.

Recovery amount: 51 mg (Yield: 40%; calculated from the amount of substance of compound 77 obtained relative to the amount of substance of compound 76).

HRMS(ESI):m/z1487.7351[M+H]⁺.

### <Synthesis of compound 78 (ADA)>

COMU (70 mg, 0.16 mmol) was added to a solution of compound 76 (50 mg, 0.041 mmol) in MeCN (0.4 mL), and the mixture was stirred at 0°C for 15 minutes. DIPEA (89 mg, 0.69 mmol) was added to the reaction solution, the mixture was further stirred at 0°C for 15 minutes, and then N-hydroxysuccinimide (19 mg, 0.16 mmol) was added. The reaction solution was stirred at room temperature for 24 hours, and then the solution was washed with H₂O (100 mL) and extracted with ethyl acetate/hexane (1/5, 100 mL × 2). The organic layer was dried with sodium sulfide and filtered. The filtrate was distilled off under reduced pressure, then half of the residue was dissolved in a mixture (95/3/2, 2 mL) of TFA/thioanisole/triisopropylsilane, and the mixture was stirred at room temperature for 11 hours. After the solvent was removed, the residue was dissolved in DMF (0.4 mL) and triethylamine (10 µL), and then ADIBO-NH₂ (6.2 mg, 0.023 mmol) was added. The reaction solution was stirred at room temperature for 24 hours, and then the solution was purified by reverse phase HPLC under the following conditions. The recovery amount and MS were as follows.

Purification conditions: Cosmosil 5C₁₈-AR-II column (4.6 × 150 mm); mobile phase: MeCN/H₂O/TFA [10/90/0.1 (0 min) to 90/10/0.1 (40 min)]; flow rate: 1 mL/min.

Recovery amount: 6.7 mg (Yield: 27%; calculated from the amount of substance of compound 78 obtained relative to the amount of substance of compound 76).

HRMS(ESI):m/z1207.4213[M+H]⁺.

### <Synthesis of compound 7A>

(S)-di-tert-butyl 2-(3-((S)-6-amino-1-tert-butoxy-1-oxohexan-2-yl)ureido) pentanedioate (31 mg, 0.064 mmol) was dissolved in DMF (0.5 mL), and then 2,5-dioxopyrrolidin-1-yl 1-azido-3,6,9,12-tetraoxapentadecan-15-oate 1 (25 mg, 0.064 mmol) was added thereto. After stirring at room temperature for 12 hours, the solution was purified by reverse phase HPLC under the following conditions. The recovery amount , NMR spectrum, and MS were as follows.

Purification conditions: Cosmosil 5C₁₈-AR-II column (10 × 250 mm); mobile phase: MeCN/H₂O/TFA [30/70/0.1 (0 min) to 90/10/0.1 (30 min)]; flow rate: 4 mL/min.

Recovery amount: 35 mg (Yield: 72%; calculated from the amount of substance of compound 7A obtained relative to the amount of substance of (S)-di-tert-butyl 2-(3-((S)-6-amino-1-tert-butoxy-1-oxohexan-2-yl)ureido) pentanedioate).

¹H-NMR(400MHz,CDCl₃)δ4.26-4.23(m,2H),3.75(t,J=5.2Hz,2H),3.69-3.61(m,14H),3.41(t,J=5.2Hz,2H),3.38-3.16(m,2H),2.59(t,J=5.2Hz,2H),2.34(dt,J=2.3,7.5Hz,2H),2.11-1.60(m,4H),1.54(t,J=7.0Hz,2H),1.48-1.40(m,27H),1.39-1.26(m,2H). ¹³C-NMR(100MHz,CDCl₃)δ174.3(2C),172.8(2C),158.4,82.7,82.4,81.2,70.4(2C),70.3,70.1,70. 0(2C),69.9,66.8,53.7, 53.3,50.5,39.2,35.8,31.5,31.3,28.2,27.9(3C),27.8(7C),21.9.

HRMS(ESI):m/z761.4656[M+H]⁺.

### <Synthesis of compound 7B>

Compound 7A (23 mg, 0.030 mmol) was dissolved in TFA (950 µL) and triisopropylsilane (50 µL), and the mixture was stirred at room temperature for 4 hours. After the solvent was removed, the residue was purified by reverse phase HPLC under the following conditions. The recovery amount, NMR spectrum, and MS were as follows.

Purification conditions: Cosmosil 5C₁₈-AR-II column (10 × 250 mm); mobile phase: MeCN/H₂O/TFA [10/90/0.1 (0 min) to 40/60/0.1 (30 min)]; flow rate: 4 mL/min.

Recovery amount: 11 mg (Yield: 63%; calculated from the amount of substance of compound 7B obtained relative to the amount of substance of compound 7A).

¹H-NMR(400MHz,CDCl₃)δ7.52(s,1H),6.37(s,2H),4.39(s,1H),4.31(s,1H),3.66(m,16H),3.40(s, 2H),2.53-1.23(m, 14H).

HRMS(ESI):593.2779[M+H]⁺.

### <Synthesis of compound 7C (PtDA)>

Compound 77 (20 mg, 0.013 mmol) was dissolved in DMF (0.6 mL), and then compound 7A (35 mg, 0.046 mmol) was added thereto. The mixture was stirred at room temperature for 12 hours, and then the solvent was removed. TFA (1.9 mL), thioanisole (60 µL), triisopropylsilane (20 µL), and H₂O (20 µL) were added to the residue, and the mixture was stirred at room temperature for 10 hours. After the solvent was removed, the residue was purified by reverse phase HPLC under the following conditions. The recovery amount and MS were as follows.

Purification conditions: Cosmosil 5C₁₈-AR-II column (4.6 × 150 mm); mobile phase: MeCN/H₂O/TFA [10/90/0.1 (0 min) to 90/10/0.1 (40 min)]; flow rate: 1 mL/min.

Recovery amount: 1.0 mg (Yield: 4.2%; calculated from the amount of substance of compound 7C(PtDA) obtained relative to the amount of substance of compound 77).

HRMS(ESI):m/z900.3488[M+2H]²⁺.

### <Synthesis of [¹¹¹In]In-ADAthrough route A>

A solution (9.2 MBq, 100 µL) of [¹¹¹In]InCl₃ and a solution of compound 78 in dimethyl sulfoxide (DMSO) (0.60 mM, 7 µL) were added to a 2-(N-morpholino)ethanesulfonic acid (MES) buffer (0.1 M, pH 5.7, 100 µL), and the mixture was allowed to stand at 90°C for 5 minutes. Thereafter, the reaction solution was purified by reverse phase HPLC under the following conditions.

Purification conditions: Cosmosil 5C₁₈-AR-II column (4.6 × 150 mm); mobile phase: MeCN/H₂O/TFA [10/90/0.1 (0 min) to 70/30/0.1 (30 min)]; flow rate: 1 mL/min.

The radiochemical yield and radiochemical purity were measured in the same manner as described in Example 1-1. The HPLC conditions used to measure the radiochemical purity was the same as the purification conditions.

The radiochemical conversion rate (the ratio of the radioactivity of [¹¹¹In]In-ADA to the radioactivity of [¹¹¹In]InCl₃) and the radiochemical yield are shown in Table 5 below.

### <Synthesis of [¹¹¹In]In-PtDA through route A>

To a phosphate buffer saline (PBS)/DMSO mixture (9/1, 200 µL) or DMSO (200 µL), [¹¹¹In]In-ADA (0.80 MBq) was added, and compound 7B (0.2 mg) was further added. After standing at 37°C for 10 or 30 minutes, the reaction solution was purified by reverse phase HPLC under the following conditions.

Purification conditions: Cosmosil 5C₁₈-AR-II column (4.6 × 150 mm); mobile phase: MeCN/H₂O/TFA [10/90/0.1 (0 min) to 70/30/0.1 (30 min)]; flow rate: 1 mL/min.

The radiochemical yield and radiochemical purity were measured in the same manner as described in Example 1-1. The HPLC conditions used to measure the radiochemical purity was the same as the purification conditions.

The radiochemical conversion rate (the ratio of the radioactivity of [¹¹¹In]In-PtDA to the radioactivity of [¹¹¹In]In-ADA) and the radiochemical yield are shown in Table 5 below.

### <Synthesis of [¹¹¹In]In-PtDA through route B>

A solution (2.1 MBq, 100 µL) of [¹¹¹In]InCl₃ and a solution of compound 7C in DMSO (0.56 mM, 2 µL) were added to a MES buffer (0.1 M, pH 5.7, 150 µL), and the mixture was allowed to stand at 90°C for 5 minutes. Thereafter, the reaction solution was purified by reverse phase HPLC under the following conditions.

Purification conditions: Cosmosil 5C₁₈-AR-II column (4.6 × 150 mm); mobile phase: MeCN/H₂O/TFA [10/90/0.1 (0 min) to 70/30/0.1 (30 min)]; flow rate: 1 mL/min.

The radiochemical yield and radiochemical conversion rate were measured in the same manner as described in Example 1-1. The HPLC conditions used to measure the radiochemical conversion rate was the same as the purification conditions.

The radiochemical conversion rate (the ratio of the radioactivity of [¹¹¹In]In-PtDA to the radioactivity of [¹¹¹In]InCl₃) and the radiochemical yield are shown in Table 5 below.

**[Table 5]**

| | Compound | Solvent | Reaction conditions | Radiochemical conversion rate (%) | Radiochemical yield (%) |
|---|---|---|---|---|---|
| Route A | [¹¹¹In]In-ADA | MES buffer | 90°C, 5 min | > 95 | 54.9 ± 9.3 |
| | [¹¹¹In]In-PtDA | PBS | 37°C, 10 min | > 95 | 55.3 ± 1.6 |
| | | DMSO | 37°C, 30 min | > 95 | 66.7 ± 7.2 |
| Route B | [¹¹¹In]In-PtDA | MES buffer | 90°C, 5 min | > 95 | 47.4 ± 14.7 |

In addition, In-ADA and In-PtDA in which non-radioactive In is coordinated can be produced by the following method. The obtained In complex was used to identify the HPLC retention time of the radiolabeled compound.

### <Synthesis of non-radioactive In-ADA>

Compound 78 (1 eq) was dissolved in acetate buffer (1.0 M, pH 5.0, 100 µL), and indium (III) chloride anhydrous (10 eq) was added. The reaction solution was stirred at 90°C for 5 minutes, and then the solution was purified by reverse phase HPLC.

Purification conditions: Cosmosil 5C₁₈-AR-II column (4.6 × 150 mm); mobile phase: MeCN/H₂O/TFA [10/90/30.1 (0 min) to 90/10/0.1 (40 min)]; flow rate: 1 mL/min.

MS(ESI):m/z1319.3[M+H]⁺.

### <Synthesis of non-radioactive In-PtDA>

A solution of compound 7C (0.5 mg, 1.25 mmol) in H₂O/MeCN/TFA (49.95/49.95/0.1, 300 µL) was added with indium (III) chloride anhydrous (0.62 mg, 2.8 µmol). After stirring at room temperature for 18 hours, the solution was purified by reverse phase HPLC.

Purification conditions: Cosmosil 5C₁₈-AR-II column (4.6 × 150 mm); mobile phase: MeCN/H₂O/TFA [10/90/0.1 (0 min) to 90/10/0.1 (40 min)]; flow rate: 1 mL/min.

Recovery amount: 0.05 mg (Yield: 9.4%; calculated from the amount of substance of non-radioactive In-PtDA obtained relative to the amount of substance of compound 7C).

HRMS(ESI):m/z956.2893[M+2H]²⁺.

### <Evaluation of distribution coefficient>

[¹¹¹In]In-PtDA (111 kBq) was added to a mixture of PBS (pH 7.4, 3 mL) and 1-octanol (3 mL), and the mixture was dispersed by vortexing for 2 minutes and then centrifuged at 4000 × g for 5 minutes. 1 mL was recovered respectively from the 1-octanol layer and the PBS layer, and the radioactivity of each layer was measured with a gamma counter (n = 3).

As the calculation formula, "(Distribution coefficient) = Log₁₀[(radioactivity of 1-octanol layer [kBq])/(radioactivity of PBS layer [kBq])]" was used.

As a result, LogP of [¹¹¹In]In-PtDA was "-3.08 ± 0.02".

### <Evaluation of stability in plasma>

A saline (20 µL) solution of [¹¹¹In]In-PtDA (259 kBq) was added to mouse plasma (200 µL), and the mixture was allowed to stand at 37°C for 24 hours (n = 3). MeCN (400 µL) was added thereto, and the mixture was centrifuged at 10,000 × g for 5 minutes. The supernatant was filtered, and the filtrate was analyzed by reverse phase HPLC under the following conditions.

Analytical conditions: Cosmosil 5C₁₈-AR-II column (4.6 × 150 mm); mobile phase: MeCN/H₂O/TFA [10/90/0.1 (0 min) to 70/30/0.1 (30 min)]; flow rate: 1 mL/min.

As a result, as shown in Fig. 7, 95% or more of [¹¹¹In]In-PtDA was stably present in mouse plasma even after standing at 37°C for 24 hours.

### <Evaluation of binding using cultured cell line>

In the same manner as in Example 1-1 described above, LNCaP cells and PC-3 cells were cultured and cell-binding assay were performed. Evaluation was carried out by the same experimental procedure and statistical method as in Example 1-1, except that 0.5% FBS-containing RPMI 1640 medium (1 mL) containing [¹¹¹In]In-PtDA (37 kBq) was used.

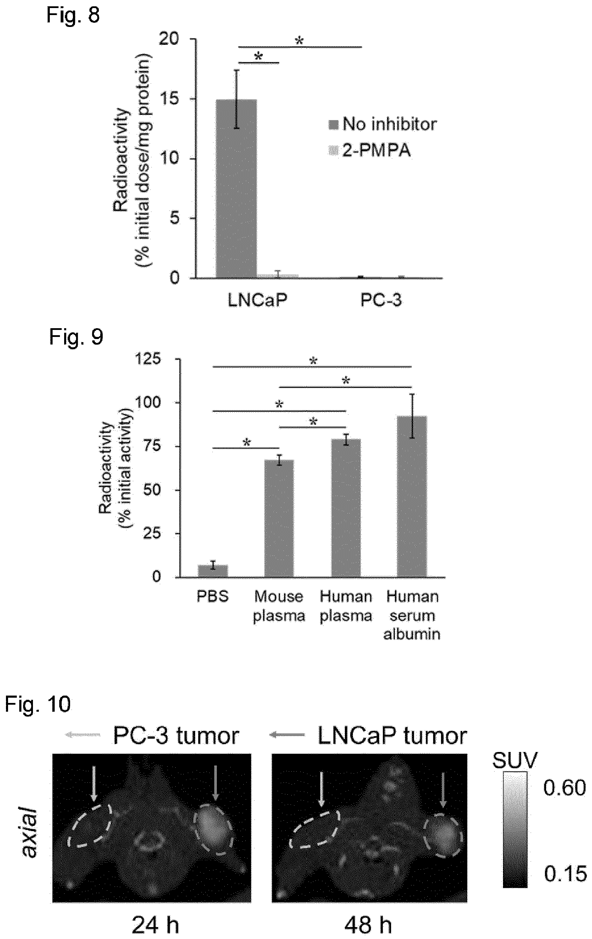

The results are shown in Fig. 8.

[¹¹¹In]In-PtDA showed high binding ability to LNCaP cells (15% ID/mg protein) compared to PC-3 cells (0.15% ID/mg protein) and the bond was significantly reduced (0.36% ID/mg protein) by adding an excess amount of the PSMA inhibitor (2-PMPA). These results showed that [¹¹¹In]In-PtDA specifically binds to PSMA-positive cells.

### <Evaluation of binding to albumin>

As in Example 1-1 described above, PBS, mouse plasma, human plasma, and human albumin were used to evaluate binding to albumin. Evaluation was carried out by the same experimental procedure and statistical method as in Example 1-1, except that a PBS solution (37kBq,50µL) of [¹¹¹In]In-PtDA was used.

The results are shown in Fig. 9.

When [¹¹¹In]In-PtDA was allowed to stand in PBS and then applied to the column, little radioactivity was detected in the eluate (6.9%). On the other hand, when allowed to stand in mouse plasma, human plasma, and a human albumin solution, high radioactivity was observed in the eluate (67.2, 79.0, and 92.4% respectively). Therefore it was shown that [¹¹¹In]In-PtDA binds to plasma albumin.

### <Evaluation of internal radioactivity distribution using LNCaP tumor-transplanted mouse>

LNCaP tumor-implanted mice prepared by the same method as in Example 1-1 were administered a saline solution (241 kBq/100 µL) of [¹¹¹In]In-PtDA from the tail vein (n = 3). Mice were euthanized 1, 24, and 48 hours after administration. Thereafter, blood and each organ were recovered, and the mass and radioactivity of the organs were measured.

The percentage (% ID) of the radioactivity amount to the administered radioactivity amount (injected dose) is shown as the value (% ID/g) divided by the blood mass or the organ mass (g). The higher the value of %ID/g, the higher the abundance of the radiolabeled compound, which indicates that the compound is highly accumulated at the target organ.

The results (Mean value ± standard deviation, each n = 3) in LNCaP tumor-transplanted mice and [¹¹¹In]In-PtDA are shown in Table 6 below.

[¹¹¹In]In-PtDA highly accumulated at the LNCaP tumor (16.0 and 18.7% ID/g; 24 and 48 hours after administration, respectively) and showed retention in blood (6.33 to 19.8% ID/g; 1 to 48 hours after administration). One hour after administration, accumulation at the kidney was 37.2% ID/g, which was significantly lower than [¹¹¹In]In-PSMA-I&T (kidney: 191% ID/g) and [⁶⁸Ga]Ga-PSMA-11 (kidney: 139% ID/g), each of which was a known radiolabeled compound whose target molecule was PSMA (for example, EJNMMI Res, 2012, 2, 23, J Nucl Med, 2017, 58,235-242).

**[Table 6]**

| [¹¹¹In]In-PtDA | 1 h | 24 h | 48 h |
|---|---|---|---|
| Blood | 19.8 ± 2.74 | 12.5 ± 1.47 | 6.33 ± 1.53 |
| Spleen | 15.3 ± 4.89 | 9.69 ± 0.92 | 10.5 ± 5.62 |
| Pancreas | 2.31 ± 0.14 | 2.03 ± 0.48 | 1.12 ± 0.39 |
| Stomach (% ID) | 0.79 ± 0.19 | 0.46 ± 0.23 | 0.29 ± 0.20 |
| Intestine | 2.66 ± 0.28 | 1.52 ± 0.41 | 0.74 ± 0.28 |
| Kidney | 37.2 ± 6.85 | 68.0 ± 2.82 | 55.9 ± 6.39 |
| Liver | 3.51 ± 0.38 | 3.36 ± 0.77 | 2.28 ± 0.75 |
| Heart | 5.49 ± 0.77 | 3.95 ± 0.87 | 1.98 ± 0.41 |
| Lung | 11.9 ± 1.57 | 8.63 ± 1.31 | 6.16 ± 1.56 |
| Brain | 0.36 ± 0.05 | 0.30 ± 0.05 | 0.20 ± 0.07 |
| Muscle | 1.95 ± 0.30 | 1.28 ± 0.34 | 0.81 ± 0.40 |
| LNCaP Tumor | 2.18 ± 0.17 | 16.0 ± 9.49 | 18.7 ± 5.21 |

### <Evaluation of SPECT/CT using tumor-transplanted mouse>

LNCaP cells (1.0 × 10⁷ cells/mouse) were suspended in a mixture of PBS and Matrigel (1 : 1, 150 µL) and subcutaneously transplanted to the right shoulder of a male CB17/IcrJcl-Prkdc^{scid} mouse kept in the same manner as in Example 1-1 under isoflurane anesthesia. In addition, a suspension of PC-3 cells (1.0 × 10⁷ cells/mouse) in a mixture of PBS and Matrigel (1 : 1, 150 µL) was subcutaneously transplanted into the left shoulder of the same mouse under isoflurane anesthesia. Thereafter, the mouse was kept for 40 to 60 days. In this way, a tumor-transplanted mouse in which LNCaP cells and PC-3 cells were simultaneously transplanted into one mouse was obtained.

The tumor-transplanted mouse was then administered saline solution (2.7 MBq, 100 µL) of [¹¹¹In]In-PtDA from the tail vein. SPECT/CT was performed 24 and 48 hours after administration with FX3300 pre-clinical imaging system, which is manufactured by Gamma Medica-Ideas. Imaging was performed using a pinhole collimator with a diameter of 1.0 mm at a rotation radius of 35 mm, a projection time of 70 seconds, and the number of projections of 32 times under isoflurane anesthesia. After SPECT, CT (Tube voltage: 60 kV, Tube current: 350 µA) was performed. Image reconstruction was performed on the projection data of SPECT through three-dimensional ordered subset expectation maximization method (8 subsets, 5 iterations).

SPECT/CT results are shown in Figure 10. The higher the SUV, the higher the radioactivity accumulation.

In SPECT/CT imaging using [¹¹¹In]In-PtDA, high radioactivity accumulation was observed at the LNCaP tumor 24 and 48 hours after administration, but almost no radioactive signal was observed in the PC-3 tumor. This result showed that [¹¹¹In]In-PtDA was able to clearly draw the PSMA-positive tumor.

### [Reference Examples 3-1 to 3-3, useful to understand the invention, and Comparative Examples 2-1 to 2-2]

In this example, compounds whose target molecule was PSMA were synthesized. Next, radiolabeled compounds were obtained in which each of the compounds were coordinated with a ¹¹¹In ion as a radioactive metal. The outline of the synthesis routes is shown below as synthesis routes (X-1) to (X-3).

The compound (Octapa-2, Octapa-3 and Neunpa-2) used in Examples 3-1 to 3-3 have a chemical structure in which the chelating part, the PSMA molecule-binding part, and the albumin-binding part are bonded with each other in a branched manner through the linker structure as shown in the general formula (2).

The compounds (Octapa-1 and Neunpa-1) used in Comparative Examples 2-1 and 2-2 had a structure containing the chelating part and the PSMA molecule-binding part, and not containing the albumin-binding part.

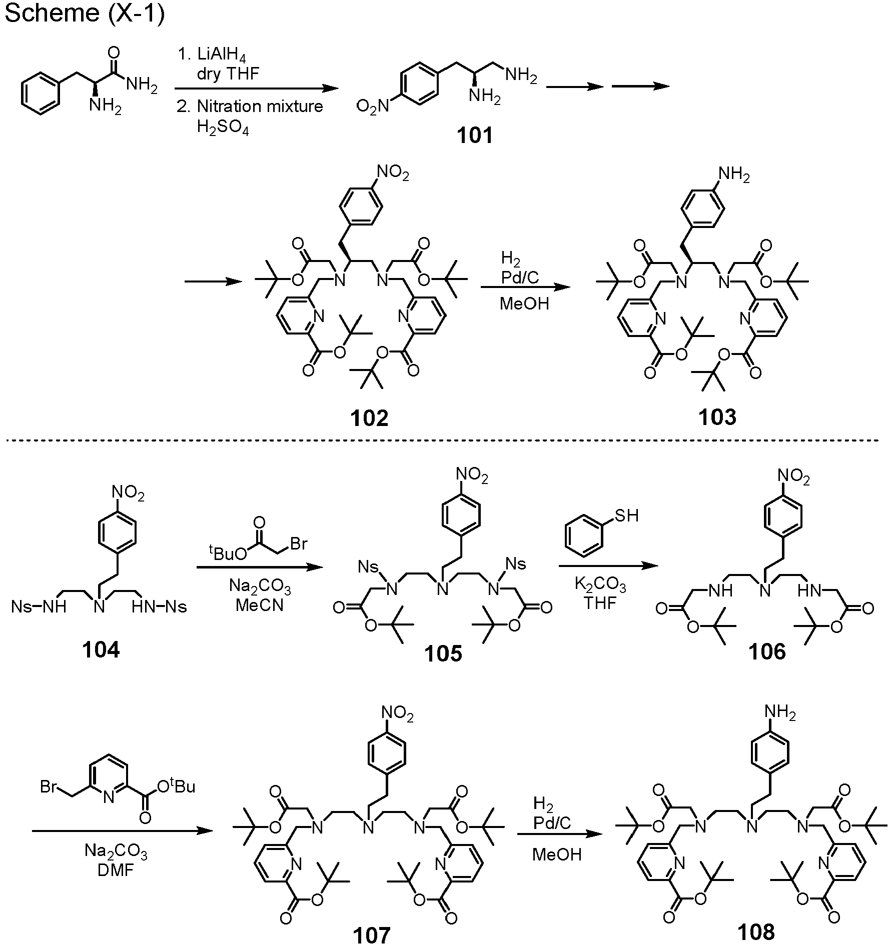

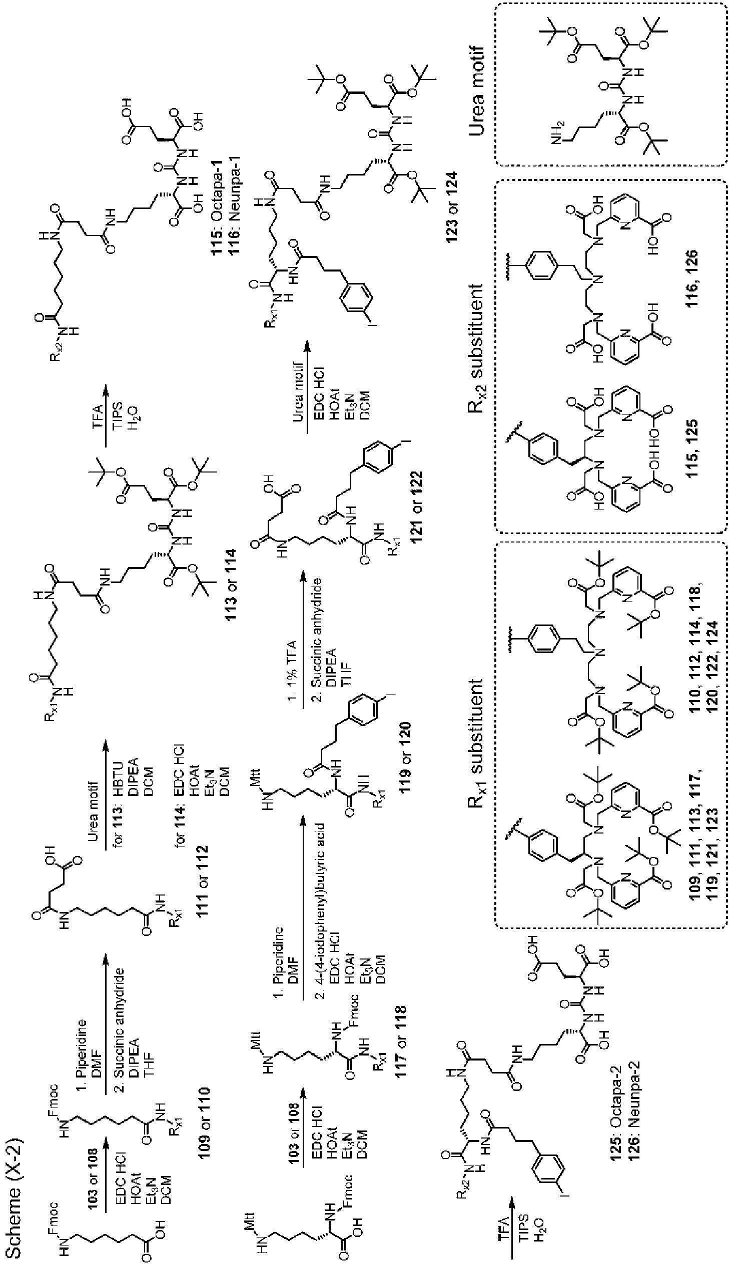

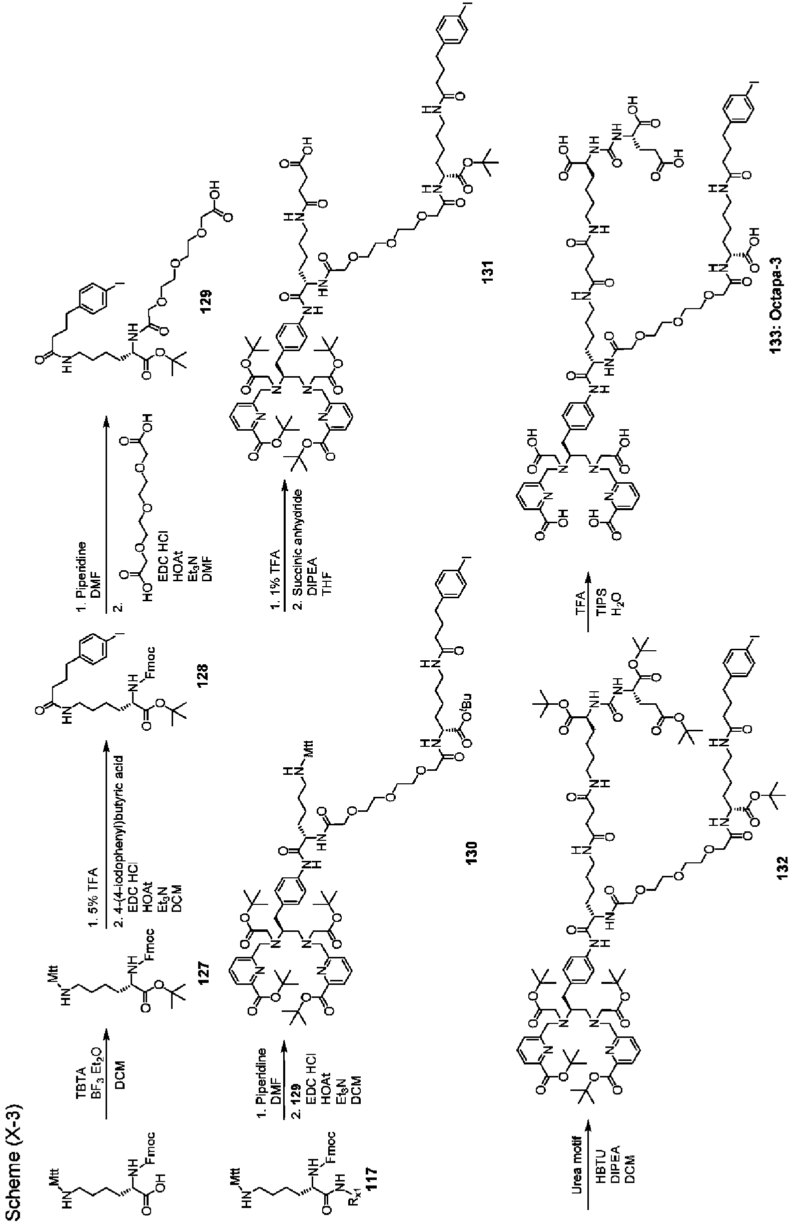

### <Synthesis of compound 101>

L-phenylalanine amide (985 mg, 6.0 mmol) was dissolved in tetrahydrofuran (THF) (20 mL), and a THF (10 mL) solution of LiAlH₄ (1139 mg, 30 mmol) was slowly added at 0°C. The reaction solution was stirred at 60°C for 24 hours, and then H₂O (1.2 mL), a 15% aqueous sodium hydroxide solution (1.2 mL), and H₂O (3.6 mL) were added in this order. The reaction solution was stirred at room temperature for 1 hour, and then filtered through celite, and the filtrate was distilled off under reduced pressure. Concentrated sulfuric acid (5 mL) was added to the residue, and then a mixture of concentrated nitric acid (400 µL) and concentrated sulfuric acid (400 µL) was added. The mixture was neutralized after being stirred at room temperature for 5 hours, and extracted with chloroform (50 mL × 3). The organic layer was dried with sodium sulfide, and filtered. The filtrate was distilled off under reduced pressure and the residue was purified by medium-pressure column chromatography (methanol/chloroform).

Recovery amount: 830 mg (Yield: 71%; calculated from the amount of substance of compound 101 obtained relative to the amount of substance of L-phenylalanine amide).

MS(ESI)m/z: 196.1[M+H]⁺.

### <Synthesis of compound 102>

Compound 102 was synthesized in 3 steps from compound 101 according to a previously reported method (J Am Chem Soc. 2013,135, 12707-12721, J Chem Soc Dalt Trans. 2014, 43, 7176-7190).

### <Synthesis of compound 103>

Compound 102 (288 mg, 0.36 mmol) was dissolved in methanol (5 mL), and palladium carbon (30 mg) was added. The reaction solution was stirred under hydrogen atmosphere for 3 hours, and then filtered through celite, and the filtrate was distilled off under reduced pressure. The residue was purified by medium-pressure column chromatography (methanol/chloroform).

Recovery amount: 150 mg (Yield: 54%; calculated from the amount of substance of compound 103 obtained relative to the amount of substance of compound 102).

¹H-NMR(400MHz,CDCl₃)δ7.82(d,J=6.9Hz,2H),7.70-7.60(m,4H),6.86(d,J=7.6Hz,2H),6.54(d,J=7.6Hz,2H),4.07(m,5H),3.40-3.23(m,5H),2.91-2.89(m,2H),2.73-2.69(m, 1H),2.53-2.50(m,2H), 1.62(s, 18H), 1.40(s, 18H).

MS(ESI)m/z:806.4[M+H]⁺.

### <Synthesis of compound 104>

Compound 104 was synthesized according to a previously reported method (Bioconjug. Chem. 2017, 28, 2145-2159).

### <Synthesis of compound 105>

To a solution of compound 104 (729 mg, 1.17 mmol) in MeCN (40 mL), t-butylbromoacetic acid (396 µL, 2.69 mmol) and sodium carbonate (285 mg, 2.69 mmol) were added, and the mixture was stirred at 60°C for 24 hours. After the temperature was returned to room temperature, the reaction solution was filtered, and the filtrate was distilled off under reduced pressure. The residue was purified by medium-pressure column chromatography (hexane/ethyl acetate).

Recovery amount: 566 mg (Yield: 57%; calculated from the amount of substance of compound 105 obtained relative to the amount of substance of compound 104).

¹H-NMR(400MHz,CDCl₃)δ8.14(d,J=8.7Hz,2H),8.05-8.02(m,2H),7.71-7.67(m,4H),7.62-7.58(m,2H),7.37(d,J=8.7Hz,2H),4.08(s,4H),3.41(t,J=7.0Hz,4H),2.86-2.77(m,8H), 1.34(s, 18H).

MS(ESI)m/z:851.2[M+H]⁺.

### <Synthesis of compound 106>

Thiophenol (253 µL, 1.53 mmol) and potassium carbonate (212 mg, 1.53 mmol) were added to a solution of compound 105 (566 mg, 0.67 mmol) in THF (20 mL), and the mixture was stirred at 50°C for 50 hours. After the temperature was returned to room temperature, the reaction solution was filtered, and the filtrate was distilled off under reduced pressure. The residue was purified by medium-pressure column chromatography (methanol/chloroform).

Recovery amount: 213 mg (Yield: 66%; calculated from the amount of substance of compound 106 obtained relative to the amount of substance of compound 105).

¹H-NMR(400MHz,CDCl₃)δ8.14(d,J=8.2Hz,2H),7.42-7.37(m,2H),3.26(s,4H),2.91-2.65(m,12H),1.47(s,18H).

MS(ESI)m/z:481.3 [M+H]⁺.

### <Synthesis of compound 107>

To a solution of compound 106 (213 mg, 0.44 mmol) in N,N-dimethylformamide (DMF) (15 mL), t-butyl-6-(bromomethyl) picolinate (264 mg, 0.97 mmol) and sodium carbonate (103 mg, 0.97 mmol) were added, and the mixture was stirred at 60°C for 24 hours. After the temperature was returned to room temperature, the reaction solution was filtered, and the filtrate was distilled off under reduced pressure. The residue was purified by medium-pressure column chromatography (methanol/chloroform).

Recovery amount: 224 mg (Yield: 60%; calculated from the amount of substance of compound 107 obtained relative to the amount of substance of compound 106).

¹H-NMR(400MHz,CDCl₃)δ8.06(d,J=8.5Hz,2H),7.88-7.86(m,2H),7.75-7.73(m,4H),7.23(d,J=8.7Hz,2H),3.99(s,4H),3.30(s,4H),2.74-2.63(m, 12H), 1.62(s, 18H),1.45(s,18H).

MS(ESI)m/z:863.5[M+H]⁺.

### <Synthesis of compound 108>

A similar reaction to the synthesis of the compound 103 was performed to obtain 50.7 mg (Yield: 23%; calculated from the amount of substance of compound 108 obtained relative to the amount of substance of compound 107) of compound 108 from compound 107.

¹H-NMR(400MHz,CDCl₃)δ7.86(d,J=7.1Hz,2H),7.78-7.72(m,4H),6.85(d,J=8.2,2H),6.56(d,J=8.2,2H),4.00(s,4H),3.32(s,4H),2.76-2.55(m,12H),1.62(s,18H),1.44(s,18H).MS(ESI)m/z:833.4[M+H]⁺.

### <Synthesis of compound 109>

Compound 103 (56.6 mg, 0.073 mmol), 1-ethyl-3-(3-dimethylaminopropyl)-carbodiimide (EDC) hydrochloride (28.8 mg, 0.15 mmol), 1-hydroxy-7-azabenzotriazole (HOAt) (20.4 mg, 0.15 mmol), and triethylamine (21 µL, 0.15 mmol) were added to a dichloromethane (7 mL) solution of 6-(Fmoc-amino) hexanoic acid (31.1 mg, 0.088 mmol), and the mixture was stirred at room temperature for 3 hours. The solvent was distilled off under reduced pressure, and then the residue was purified by medium-pressure column chromatography (methanol/chloroform).

Recovery amount: 30.2 mg (Yield: 37%; calculated from the amount of substance of compound 109 obtained relative to the amount of substance of compound 103).

HRMS(ESI)m/z:1111.6170[M+H]⁺.

### <Synthesis of compound 110>

A similar reaction to the synthesis of compound 109 was performed to obtain 17.7 mg (Yield: 25%; calculated from the amount of substance of compound 110 obtained relative to the amount of substance of compound 108) of compound 110 from compound 108.

HRMS(ESI)m/z:1168.6372[M+H]⁺.

### <Synthesis of compound 111>

Compound 109 (30.2 mg, 0.027 mmol) was dissolved in a mixture of piperidine (1 mL) and DMF (4 mL), and the mixture was stirred at room temperature for 2.5 hours. Ethyl acetate and H₂O were added to the reaction liquid, and then the organic layer was dried with sodium sulfide and filtered. The filtrate was distilled off under reduced pressure, and then THF (5 mL) was added to the residue. Succinic anhydride (11 mg, 0.11 mmol) and N,N-diisopropylethylamine (DIPEA) (19 µL, 0.11 mmol) were added, and the mixture was stirred at room temperature for 3 hours. The solvent was distilled off under reduced pressure, and then the residue was purified by medium-pressure column chromatography (methanol/chloroform).

Recovery amount: 9.7 mg (Yield: 37%; calculated from the amount of substance of compound 111 obtained relative to the amount of substance of compound 109).

HRMS(ESI)m/z:989.5600[M+H]⁺.

### <Synthesis of compound 112>

A similar reaction to the synthesis of compound 111 was performed to obtain 17.7 mg (yield: 95%) of compound 112 from compound 110.

HRMS(ESI)m/z:1046.6139[M+H]⁺.

### <Synthesis of compound 113>

To a solution of compound 111 (6.2 mg, 6.3 µmol) in dichloromethane (5 mL), (S)-di-tert-butyl 2-(3-((S)-6-amino-1-tert-butoxy-1-oxohexan-2-yl)ureido) pentanedioate (6.2 mg, 13 µmol), 1-[bis(dimethylamino)methylene]-1H-benzotriazolium 3-oxide hexafluorophosphate (HBTU) (5.0 mg, 13 µmol), and DIPEA (10 µL, 0.57 µmol) were added, and the mixture was stirred at room temperature for 26 hours. The solvent was distilled off under reduced pressure, and the residue was purified by medium-pressure column chromatography (methanol/chloroform).

Recovery amount: 4.5 mg (Yield: 49%; calculated from the amount of substance of compound 113 obtained relative to the amount of substance of compound 111).

HRMS(ESI)m/z:729.9404[M+2H]⁺.

### <Synthesis of compound 114>

A solution of compound 112 (19.3 mg, 0.018 mmol) in dichloromethane (5 mL) was added with (S)-di-tert-butyl 2-(3-((S)-6-amino-1-tert-butoxy-1-oxohexan-2-yl)ureido) pentanedioate (17.6 mg, 0.036 mmol), EDC hydrochloride (6.9 mg, 0.036 mmol), HOAt (4.9 mg, 0.036 mmol), and triethylamine (5 µL, 0.036 mmol). The reaction solution was stirred at room temperature for 6 hours, and the solvent was distilled off under reduced pressure. The residue was purified by medium-pressure column chromatography (methanol/chloroform).

Recovery amount: 15.6 mg (Yield: 57%; calculated from the amount of substance of compound 114 obtained relative to the amount of substance of compound 112).

HRMS(ESI)m/z:758.4704[M+2H]⁺.

### <Synthesis of compound 115 (Octapa-1)>

Compound 113 (11.4 mg, 7.8 µmol) was dissolved in a mixture of trifluoroacetic acid (TFA)/H₂O/triisopropylsilane (95/2.5/2.5, 2 mL), and the solution was stirred at room temperature for 3 hours. After the solvent was removed, the residue was purified by reverse phase HPLC under the following conditions.

Purification conditions: Cosmosil 5C₁₈-AR-II column (4.6 × 150 mm); mobile phase: MeCN/H₂O/TFA [10/90/0.1 (0 min) to 30/70/0.1 (30 min)]; flow rate: 1 mL/min.

Recovery amount: 2.2 mg (Yield: 27%; calculated from the amount of substance of compound 115 obtained relative to the amount of substance of compound 113).

HRMS(ESI)m/z:1066.4325[M+H]⁺.

### <Synthesis of compound 116 (Neunpa-1)>

A similar reaction to the synthesis of compound 115 was performed to obtain 1.8 mg (Yield: 43%) of compound 116 from compound 114.

HRMS(ESI)m/z:1123.4924[M+H]⁺.

### <Synthesis of compound 117>

Compound 103 (42.3 mg, 0.055 mmol), EDC hydrochloride (21.1 mg, 0.11 mmol), HOAt (15.0 mg, 0.11 mmol), and triethylamine (15 µL, 0.11 mmol) were added to a dichloromethane (5 mL) solution of N²-[(9H-fluoren-9-ylmethoxy)carbonyl]-N⁶-[(4-methylphenyl)diphenylmethyl]-L-lysine (40.9 mg, 0.065 mmol). The reaction solution was stirred at room temperature for 3 hours, and the solvent was distilled off under reduced pressure. The residue was purified by medium-pressure column chromatography (methanol/chloroform).

Recovery amount: 69.3 mg (Yield: 91%; calculated from the amount of substance of compound 117 obtained relative to the amount of substance of N²-[(9H-fluoren-9-ylmethoxy)carbonyl]-N⁶-[(4-methylphenyl)diphenylmethyl]-L-lysine).

HRMS(ESI)m/z:1382.7418[M+H]⁺.

### <Synthesis of compound 118>

A similar reaction to the synthesis of compound 117 was performed to obtain 123 mg (Yield: 77%; calculated from the amount of substance of compound 118 obtained relative to the amount of substance of compound 108) of compound 118 from compound 108.

HRMS(ESI)m/z:1439.8029[M+H]⁺.

### <Synthesis of compound 119>

Compound 117 (115 mg, 0.083 mmol) was dissolved in a mixture of piperidine (1 mL) and DMF (4 mL), and the mixture was stirred at room temperature for 2.5 hours. Ethyl acetate and H₂O were added to the reaction liquid. Thereafter, the organic layer was dried with sodium sulfide and filtered. The filtrate was distilled off under reduced pressure, and the residue was dissolved in dichloromethane (5 mL), and 4-(4-iodophenyl) butanoic acid (49.3 mg, 0.17 mmol), EDC hydrochloride (32.6 mg, 0.17 mmol), HOAt (23.1 mg, 0.17 mmol), and triethylamine (20 µL, 0.17 mmol) were added. The reaction liquid was stirred at room temperature for 7 hours, and then the solvent was distilled off under reduced pressure. The residue was purified by medium-pressure column chromatography (methanol/chloroform).

Recovery amount: 62.4 mg (Yield: 52%; calculated from the amount of substance of compound 119 obtained relative to the amount of substance of compound 117).

HRMS(ESI)m/z:1432.6529[M+H]⁺.

### <Synthesis of compound 120>

A similar reaction to the synthesis of compound 119 was performed to obtain 84.2 mg (Yield: 66%; calculated from the amount of substance of compound 120 obtained relative to the amount of substance of compound 118) of compound 120 from compound 118.

HRMS(ESI)m/z:1489.7260[M+H]⁺.

### <Synthesis of compound 121>

Compound 119 (62.4 mg, 0.044 mmol) was dissolved in 1% TFA-containing dichloromethane (5 mL) and stirred at room temperature for 2 hours. After the solvent was removed, the residue was dissolved in THF (5 mL), and then succinic anhydride (8.1 mg, 0.088 mmol) and DIPEA (15 µL, 0.088 mmol) were added. The solution was stirred at room temperature for 4 hours, and then the solvent was removed. The residue was purified by medium pressure column chromatography (methanol/chloroform).

Recovery amount: 65 mg (Yield: 100%; calculated from the amount of substance of compound 121 obtained relative to the amount of substance of compound 119).

HRMS(ESI)m/z:1276.5478[M+H]⁺.

### <Synthesis of compound 122>

A similar reaction to the synthesis of compound 121 was performed to obtain 39.1 mg (Yield: 35%; calculated from the amount of substance of compound 122 obtained relative to the amount of substance of compound 120) of compound 122 from compound 120.

FIRMS(ESI)m/z:1333.5464[M+H]⁺.

### <Synthesis of compound 123>

A solution of compound 121 (65 mg, 0.051 mmol) in dichloromethane (5 mL) was added with (S)-di-tert-butyl 2-(3-((S)-6-amino-1-tert-butoxy-1-oxohexan-2-yl)ureido) pentanedioate (37.3 mg, 0.077 mmol), EDC hydrochloride (19.2 mg, 0.10 mmol), HOAt (13.6 mg, 0.10 mmol), and triethylamine (15 µL, 0.10 mmol). The reaction solution was stirred at room temperature for 5 hours, and the solvent was distilled off under reduced pressure. The residue was purified by medium-pressure column chromatography (methanol/chloroform).

Recovery amount: 54.3 mg (Yield: 61%; calculated from the amount of substance of compound 123 obtained relative to the amount of substance of compound 121).

HRMS(ESI)m/z: 873.4248[M+2H]⁺.

### <Synthesis of compound 124>

A similar reaction to the synthesis of compound 123 was performed to obtain 22.3 mg (Yield: 25%; calculated from the amount of substance of compound 124 obtained relative to the amount of substance of compound 122) of compound 124 from compound 122.

HRMS(ESI)m/z:902.4312[M+2H]⁺.

### <Synthesis of compound 125 (Octapa-2)>

Compound 123 (9.6 mg, 6.6 µmol) was dissolved in a mixture of TFA/H₂O/triisopropylsilane (95/2.5/2.5, 1 mL), and the mixture was stirred at room temperature for 2 hours. After removing the solvent, the residue was purified by reverse phase HPLC under the following conditions.

Purification conditions: Cosmosil 5C₁₈-AR-II column (4.6 × 150 mm); mobile phase: MeCN/H₂O/TFA [20/80/0.1 (0 min) to 50/50/0.1 (30 min)]; flow rate: 1 mL/min.

Recovery amount: 1.3 mg (Yield: 17%; calculated from the amount of substance of compound 125 obtained relative to the amount of substance of compound 123).

HRMS(ESI)m/z:677.2144[M+2H]⁺.

### <Synthesis of compound 126 (Neunpa-2)>

A similar reaction to the synthesis of compound 125 was performed to obtain 2.0 mg (Yield: 47%; calculated from the amount of substance of compound 126 obtained relative to the amount of substance of compound 124) of compound 126 from compound 124.

HRMS(ESI)m/z:705.74021[M+2H]⁺.

### <Synthesis of compound 127>

To a solution of N²-[(9H-fluoren-9-ylmethoxy)carbonyl]-N⁶-[(4-methylphenyl)diphenylmethyl]-L-lysine (625 mg, 1.0 mmol) in dichloromethane (20 mL), tert-butyl trichloroacetimidate (437 mg, 2.0 mmol) and BF₃·OEt₂ (20 µL, 0.16 mmol) were added. The reaction solution was stirred overnight at room temperature, and then the solvent was distilled off under reduced pressure. The residue was purified by medium-pressure column chromatography (hexane/ethyl acetate).

Recovery amount: 322 mg (Yield: 47%; calculated from the amount of substance of compound 127 obtained relative to the amount of substance of N²-[(9H-fluoren-9-ylmethoxy)carbonyl]-N⁶-[(4-methylphenyl)diphenylmethyl]-L-lysine).

¹H-NMR(400MHz,CDCl₃)δ7.71-7.69(m,2H),7.56(d,J=7.2Hz,2H),7.46(d,J=8.1Hz,4H),7.35-7.30(m,4H),7.26-7.21(m,6H),7.15-7.11(m,2H),7.04(d,J=8.1Hz,2H),5.38(d,J=8.7Hz,1H),4.35(d,J=6.4Hz,2H),4.28-4.23(m,1H),4.18(t,J=7.5Hz,1H),2.26(s,3H),2.12-2.11(m,2H),1.81-1.75(m,1H),1.59-1.44(m,14H).

MS(ESI)m/z:681.4[M+H]⁺.

### <Synthesis of compound 128>

Compound 127 (200 mg, 0.47 mmol) was dissolved in 5% TFA-containing dichloromethane (10 mL), and the mixture was stirred at room temperature for 3 hours. The solvent was removed, and then the residue was purified by medium-pressure column chromatography (methanol/chloroform). The obtained amine was dissolved in DMF (10 mL), and 4-(4-iodophenyl) butanoic acid (273 mg, 0.94 mmol), EDC hydrochloride (180 mg, 0.94 mmol), HOAt (123 mg, 0.94 mmol), and triethylamine (130 µL, 0.94 mmol) were added thereto. The reaction liquid was stirred overnight at room temperature, and then ethyl acetate and H₂O were added thereto. The organic layer was dried with sodium sulfide, and filtered. The filtrate was distilled off under reduced pressure, and then the residue was purified by medium-pressure column chromatography (methanol/chloroform).

Recovery amount: 161 mg (Yield: 50%; calculated from the amount of substance of compound 128 obtained relative to the amount of substance of compound 127).

¹H-NMR(400MHz,CDCl₃)δ7.76(d,J=7.5Hz,2H),7.60-7.53(m,4H),7.39(t,J=7.5Hz,2H),7.30(t,J=7.5Hz,2H),6.92-6.86(m,2H),4.40-4.09(m,4H),3.27-3.19(m,2H),2.52(t,J=7.8Hz,2H),1.93-1.80(m,3H),1.71-1.62(m,1H),1.52-1.36(m,13H).

MS(ESI)m/z:697.2[M+H]⁺.

### <Synthesis of compound 129>

Compound 128 (473 mg, 0.68 mmol) was dissolved in a mixture of piperidine (2 mL) and DMF (8 mL), and the mixture was stirred at room temperature for 1 hour. Ethyl acetate and H₂O were added to the reaction liquid. Thereafter, the organic layer was dried with sodium sulfide and filtered. The filtrate was distilled off under reduced pressure, and the residue was purified by medium-pressure column chromatography (methanol/chloroform). The obtained amine was dissolved in DMF (10 mL), and 3,6,9-trioxaundecanedioic acid (91.1 mg, 0.41 mmol), EDC hydrochloride (78.6 mg, 0.41 mmol), HOAt (55.8 mg, 0.41 mmol), and triethylamine (57 µL, 0.41 mmol) were added. The reaction liquid was stirred at room temperature for 5 hours, and then ethyl acetate and H₂O were added. The organic layer was dried with sodium sulfide, and filtered. The filtrate was distilled off under reduced pressure, and the residue was then purified by medium-pressure column chromatography (methanol/chloroform).

Recovery amount: 87.9 mg (Yield: 37%; calculated from the amount of substance of compound 129 obtained relative to the amount of substance of compound 128).

¹H-NMR(400MHz,CD₃OD)δ7.58(d,J=8.7Hz,2H),6.96(d,J=7.5,2H)4.41-4.29(m,2H),4.09-4.02(m,2H),3.85-3.73(m,8H),3.60(q,J=7.2Hz,2H),3.35(s,2H),3.10(t,J=4.0Hz,2H),2.55(t,J=7.5Hz,2H),2.16-2.14(m,2H),1.89-1.81(m,2H),1.41(s,9H),1.32-1.27(m,3H),1.18(t,J=7.0Hz,2H).

MS(ESI)m/z:701.2[M+Na]⁺.

### <Synthesis of compound 130>

Compound 117 (400 mg, 0.29 mmol) was dissolved in a mixture of piperidine (1 mL) and DMF (4 mL), and the mixture was stirred at room temperature for 2.5 hours. Ethyl acetate and H₂O were added to the reaction liquid. Thereafter, the organic layer was dried with sodium sulfide and filtered. The filtrate was distilled off under reduced pressure, and the residue was purified by medium-pressure column chromatography (methanol/chloroform). The obtained amine was dissolved in DMF (10 mL), and compound 129 (170 mg, 0.25 mmol), EDC hydrochloride (95.9 mg, 0.50 mmol), HOAt (68.1 mg, 0.50 mmol), and triethylamine (69 µL, 0.50 mmol) were added. The reaction liquid was stirred at room temperature for 2 hours, and then ethyl acetate and H₂O were added. The organic layer was dried with sodium sulfide, and filtered. The filtrate was distilled off under reduced pressure, and then the residue was purified by medium-pressure column chromatography (methanol/chloroform).

Recovery amount: 272 mg (Yield: 60%; calculated from the amount of substance of compound 130 obtained relative to the amount of substance of compound 117).

HRMS(ESI)m/z:911.4302[M+2H]⁺.

### <Synthesis of compound 131>

Compound 130 (272 mg, 0.15 mmol) was dissolved in 1% TFA-containing dichloromethane (3 mL), and the mixture was stirred at room temperature for 1.5 hours. After the solvent was removed, the residue was dissolved in THF (5 mL), and succinic anhydride (30 mg, 0.30 mmol) and DIPEA (52 µL, 0.30 mmol) were added. The reaction solution was stirred at room temperature for 2 hours, and the solvent was distilled off under reduced pressure. The residue was purified by medium-pressure column chromatography (methanol/chloroform).

Recovery amount: 230 mg (Yield: 92%; calculated from the amount of substance of compound 131 obtained relative to the amount of substance of compound 130).

HRMS(ESI)m/z:832.8701[M+2H]⁺.

### <Synthesis of compound 132>

(S)-di-tert-butyl 2-(3-((S)-6-amino-1-tert-butoxy-1-oxohexan-2-yl)ureido) pentanedioate (63.4 mg, 0.13 mmol), HBTU (83.4 mg, 0.22 mmol) and DIPEA (38 µL, 0.22 mmol) were added to a solution of compound 131 (186 mg, 0.11 mmol) in dichloromethane (5 mL), and the mixture was stirred at room temperature for 1.5 hours. The solvent was distilled off under reduced pressure, and the residue was purified by medium-pressure column chromatography (methanol/chloroform).

Recovery amount: 80.4 mg (Yield: 34%; calculated from the amount of substance of compound 132 obtained relative to the amount of substance of compound 131).

HRMS(ESI)m/z:1068.0419[M+2H]⁺.

### <Synthesis of compound 133 (Octapa-3)>

Compound 132 (10 mg, 4.7 µmol) was dissolved in a mixture of TFA/H₂O/triisopropylsilane (95/2.5/2.5, 2 mL), and the solution was stirred overnight at room temperature. After the solvent was removed, the residue was purified by reverse phase HPLC under the following conditions.

Purification conditions: Cosmosil 5C₁₈-AR-II column (4.6 × 150 mm); mobile phase: MeCN/H₂O/TFA [20/80/0.1 (0 min) to 50/50/0.1 (30 min)]; flow rate: 1 mL/min.

Recovery amount: 2.3 mg (Yield: 29%; calculated from the amount of substance of compound 133 obtained relative to the amount of substance of compound 132).

HRMS(ESI)m/z:843.2900[M+2H]⁺.

### <Production of radiolabeled compound with ¹¹¹In-labeling (Examples 3-1 to 3-2, and Comparative Example 2-1)>

For each of the three Octapa derivatives (Octapa-1 to -3), a solution (100 µL) of [¹¹¹In]InCl₃ was mixed with an acetate buffer (pH 5.5, 10 mM, 350 µL), and an aqueous solution (< 10% DMSO, 10 µM, 50 µL) of the labeling precursor (Compound 115, 125, or 133) was added. After left to stand at room temperature for 15 minutes, the reaction solution was purified by reverse phase HPLC under the following conditions.

Purification conditions: Cosmosil 5C₁₈-MS-II column (4.6 × 150 mm); mobile phase: MeCN/acetate buffer (pH 4.5, 10 mM) [10/90 (0 min) to 30/70 (30 min) or 20/80 (0 min) to 50/50 (30 min)]; flow rate: 1 mL/min.

The radiochemical yield and radiochemical purity were measured in the same manner as described in Example 1-1. The HPLC conditions used to measure the radiochemical purity was the same as the purification conditions.

As a result, the following three kinds of radiolabeled compound were obtained with a radiochemical yield of 55 to 93% and a radiochemical purity of 99% or more.
- Comparative Example 2-1: [¹¹¹In]In-Octapa-1
- Reference Example 3-1: [¹¹¹In]In-Octapa-2
- Reference Example 3-2: [¹¹¹In]In-Octapa-3

### <Production of radiolabeled compound with ¹¹¹In-labeling (Reference Example 3-3 and Comparative Example 2-2>

For the two Neunpa derivatives (Neunpa-1 and -2), a solution (100 µL) of [¹¹¹In]InCl₃ was mixed with an acetate buffer (pH 4.0, 10 mM, 350 µL), and an aqueous solution (< 10% DMSO, 10 µM, 50 µL) of the labeling precursor (compound 116 or 126) was added. The mixture was allowed to stand at room temperature for 15 minutes, and then the reaction solution was purified by reverse phase HPLC under the following conditions.

Purification conditions: Cosmosil 5C₁₈-AR-II column (4.6 × 150 mm); mobile phase: MeCN/H₂O/TFA [10/90/0.1 (0 min) to 30/70/0.1 (30 min) or 20/80/0.1 (0 min) to 50/50/0.1 (30 min)]; flow rate: 1 mL/min.

The radiochemical yield and radiochemical purity were measured in the same manner as described in Example 1-1. The HPLC conditions used to measure the radiochemical purity was the same as the purification conditions.

As a result, the following two kinds of radiolabeled compound were obtained with a radiochemical yield of 55 to 93% and a radiochemical purity of 99% or more.
- Comparative Example 2-2: [¹¹¹In]In-Neunpa-1
- Reference Example 3-3: [¹¹¹In]In-Neunpa-2

### <Binding assay using cultured cells>

LNCaP cells and PC-3 cells cultured in the same method as in Example 1-1 were each seeded on a 12 well plate at 4.0 × 10⁵ cells/well, and left standing at 37°C under 5% CO₂ for 48 hours.

The culture medium was removed, and an assay medium (0.5% FBS-containing RPMI 1640 medium) solution (1 mL) containing the radiolabeled compound (37 kBq each) in Examples 3-1 to 3-3 or Comparative Examples 2-1 to 2-2 was added. Thereafter, the plate was allowed to stand at 37°C under 5% CO₂ for 1 hour.

In the inhibition experiments, after removing the culture medium, an assay medium (1 mL) containing: the radiolabeled compound described above; and 2-(phosphonomethyl) pentanedioic acid (2-PMPA) (PSMAinhibitor; final concentration: 100 µM) was added. Thereafter, the plate was allowed to stand at 37°C under 5% CO₂ for 1 hour.

After removing the assay medium, each well was washed with assay medium (1 mL), and the cells were lysed with 1 N aqueous sodium hydroxide solution (200 µL × 2).

Radioactivity of each of the cell lysates was measured with a gamma counter. Separately, the total protein concentration in the cell lysate was calculated using BCA Protein Assay Kit, which is manufactured by Thermo Fisher Scientific K.K. The value (% ID/mg protein) obtained by dividing the percentage (% ID) of the sample's radioactivity amount to the added radioactivity amount by the total protein amount was calculated for each sample.

The data were expressed as mean value ± standard deviation. The significant difference test was performed using Student's t-test and one-way analysis of variance (ANOVA) test with Dunnet's post-hoc test, and p < 0.05 was defined as having a significant difference.

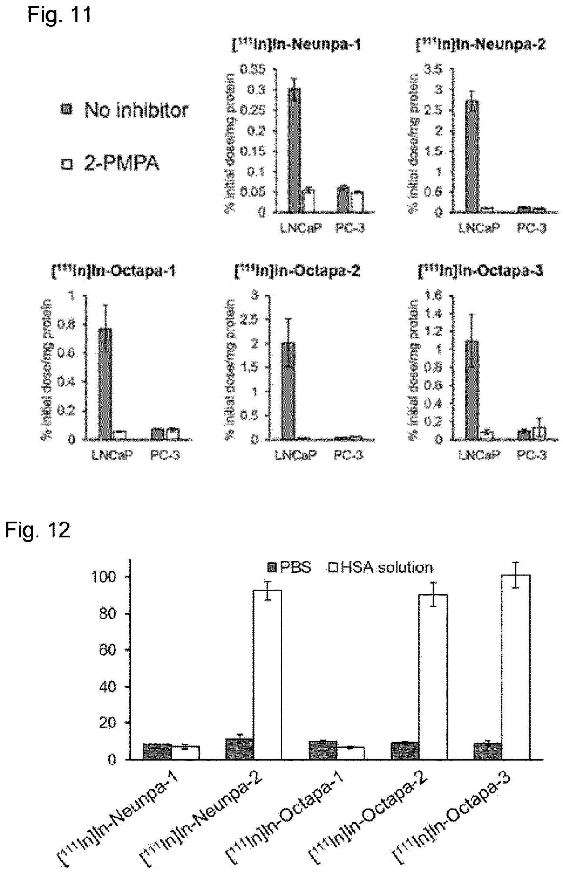

The results of the evaluation of binding to cultured cells are shown in Fig. 11. The higher the value, the higher the abundance of the radiolabeled compound, which indicates that the compound is highly accumulated.

The radiolabeled compounds in Examples 3-1 to 3-3 and Comparative Examples 2-1 to 2-2 showed high binding ability to PSMA-positive LNCaP cells compared to PSMA-negative PC-3 cells, and the bonds were significantly reduced by adding an excess amount of the PSMA inhibitor (2-PMPA). Therefore, it was shown that the radiolabeled compounds of Examples and Comparative Examples specifically bind to the PSMA positive-cells, respectively.

### <Binding assay to albumin>

As in Examples described above, PBS and human albumin were used to evaluate binding to albumin. Evaluation was performed by the same experimental procedure and statistical method as in Example 1-1 except that PBS solutions (37 kBq, 50 µL each) of the radiolabeled compounds in Examples 3-1 to 3-3 and Comparative Examples 2-1 to 2-2 were used, respectively.

The results are shown in Fig. 12.

The radioactivity ratio of the eluates of the HSA solutions using Examples 3-1 to 3-3 ([¹¹¹In]In-Octapa-2, [¹¹¹In]In-Octapa-3, and [¹¹¹In]In-Neunpa-2) were significantly higher than the radioactivity ratio of the PBS eluates.

On the other hand, the radioactivity ratio of the eluates of the HSA solutions of Comparative Examples 2-1 to 2-2 ([¹¹¹In]In-Octapa-1 and [¹¹¹In]In-Neunpa-1) was low as in the case of PBS. These results showed that each of the compounds and radiolabeled compounds used in Examples 3-1 to 3-3 had a binding ability to albumin.

### <Evaluation of internal radioactivity distribution using tumor-transplanted mouse>

LNCaP tumor-implanted mice prepared by the same method as in Example 1-1 were administered a saline solution (259 kBq/100µL each) of each of the radiolabeled compounds in Examples 3-1 to 3-3 or Comparative Examples 2-1 to 2-2 from the tail vein (n = 2 to 3). Mice were euthanized 1, 4, 24, 48, 96, and 192 hours after administration. Thereafter, blood and each organ were recovered, and the mass and radioactivity of the organs were measured.

The percentage (% ID) of the radioactivity amount to the administered radioactivity amount (injected dose) is shown as the value (% ID/g) divided by the blood mass or the organ mass (g). The higher the value of %ID/g, the higher the abundance of the radiolabeled compound, which indicates that the compound is highly accumulated at the target organ.

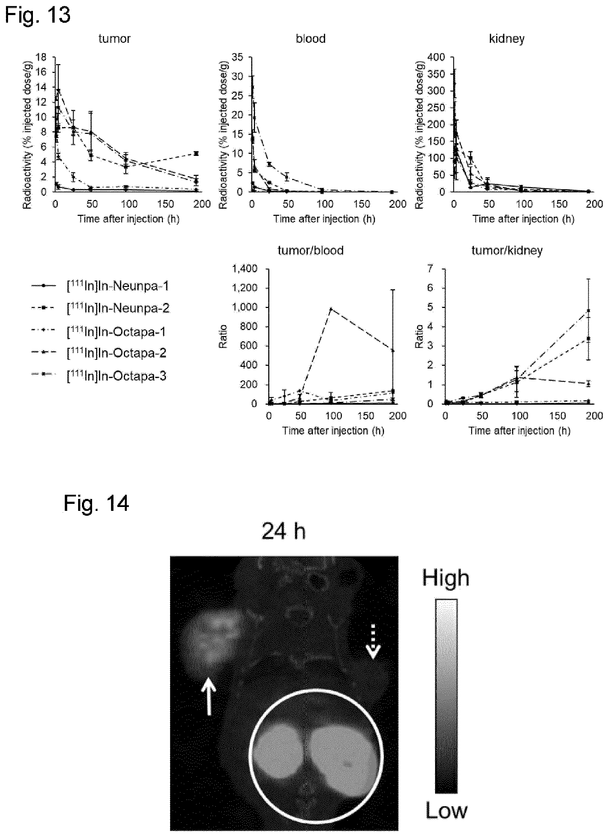

The results (Mean value ± standard deviation, each n = 2 to 3) in LNCaP tumor-transplanted mouse and each of the radiolabeled compounds are shown in Tables 7 to 11 and Figure 13 below.

It was confirmed that, compared to Comparative Examples 2-1 to 2-2, each of the radiolabeled compounds of Examples 3-1 to 3-3 had high retention in blood and tumor accumulation while accumulation at the kidney was reduced to the same extent. This was particularly remarkable in Reference Example 3-1 ([¹¹¹In]In-Octapa-2), Reference Example 3-2 ([¹¹¹In]In-Octapa-3), and Reference Example 3-3 ([¹¹¹In]In-Neunpa-2).

### • Comparative Example 2-1 ([¹¹¹In]In-Octapa-1; Mean value ± standard deviation, n = 3 for each, and n = 2 only for the point of 192 hours).

**[Table 7]**

| [¹¹¹In]In-Octapa-1 | 1 h | 4 h | 24 h | 48 h | 96 h | 192 h |
|---|---|---|---|---|---|---|
| Blood | 0.48 ± 0.09 | 0.21 ± 0.18 | 0.02 | 0.00 ± 0.00 | 0.02 ± 0.01 | 0.00 |
| Spleen | 10.9 ± 5.59 | 3.55 ± 1.75 | 2.48 ± 1.44 | 1.64 ± 0.53 | 2.06 ± 0.88 | 0.85 |
| Pancreas | 0.62 ± 0.19 | 0.24 ± 0.07 | 0.12 ± 0.03 | 0.05 ± 0.01 | 0.04 ± 0.00 | 0.05 |
| Stomach (% ID) | 0.08 ± 0.01 | 0.37 ± 0.22 | 0.03 ± 0.01 | 0.07 ± 0.04 | 0.03 ± 0.01 | 0.01 |
| Intestine | 0.40 ± 0.13 | 3.50 ± 1.21 | 0.27 ± 0.08 | 0.52 ± 0.33 | 0.13 ± 0.05 | 0.02 |
| Kidney | 322 ± 42.8 | 112 ± 54.6 | 24.9 ± 10.9 | 7.48 ± 1.23 | 7.24 ± 3.00 | 2.16 |
| Liver | 0.36 ± 0.13 | 0.13 ± 0.02 | 0.06 ± 0.01 | 0.04 ± 0.01 | 0.08 ± 0.02 | 0.05 |
| Heart | 0.32 ± 0.08 | 0.18 ± 0.03 | 0.04 ± 0.01 | 0.02 ± 0.03 | 0.03 ± 0.00 | 0.01 |
| Lung | 0.99 ± 0.22 | 0.23 ± 0.10 | 0.13 ± 0.06 | 0.06 ± 0.02 | 0.09 ± 0.03 | 0.04 |
| Brain | 0.02 ± 0.00 | 0.02 ± 0.01 | 0.04 ± 0.04 | 0.01 ± 0.00 | 0.01 ± 0.00 | 0.01 |
| Muscle | 0.20 ± 0.01 | 0.10 ± 0.02 | 0.01 ± 0.01 | 0.01 ± 0.02 | 0.03 ± 0.04 | 0.02 |
| LNCaP Tumor | 9.72 ± 1.65 | 4.73 ± 0.40 | 2.00 ± 0.61 | 0.58 ± 0.11 | 0.72 ± 0.11 | 0.36 |

### • Reference Example 3-1 ([¹¹¹In]In-Octapa-2; Mean value ± standard deviation, and each n = 3).

**[Table 8]**

| [¹¹¹In]In-Octapa-2 | 1 h | 4 h | 24 h | 48 h | 96 h | 192 h |
|---|---|---|---|---|---|---|
| Blood | 14.4 ± 1.81 | 6.73 ± 1.63 | 0.84 ± 0.27 | 0.26 ± 0.23 | 0.01 ± 0.01 | 0.01 ± 0.01 |
| Spleen | 18.5 ± 4.39 | 13.2 ± 2.66 | 1.97 ± 0.90 | 1.13 ± 0.70 | 0.61 ± 0.04 | 0.44 ± 0.12 |
| Pancreas | 1.73 ± 0.18 | 1.59 ± 0.94 | 0.19 ± 0.05 | 0.13 ± 0.06 | 0.06 ± 0.02 | 0.08 ± 0.02 |
| Stomach (% ID) | 0.51 ± 0.22 | 0.23 ± 0.02 | 0.21 ± 0.15 | 0.16 ± 0.14 | 0.04 ± 0.02 | 0.01 ± 0.01 |
| Intestine | 1.55 ± 0.21 | 1.65 ± 0.37 | 0.82 ± 0.68 | 0.68 ± 0.32 | 0.45 ± 0.29 | 0.27 ± 0.18 |
| Kidney | 121 ± 14.9 | 177 ± 35.7 | 55.3 ± 19.3 | 22.4 ± 14.5 | 3.27 ± 0.26 | 1.76 ± 0.70 |
| Liver | 2.75 ± 0.54 | 1.67 ± 0.47 | 0.33 ± 0.02 | 0.32 ± 0.10 | 0.22 ± 0.01 | 0.20 ± 0.05 |
| Heart | 3.98 ± 0.51 | 1.89 ± 0.49 | 0.33 ± 0.05 | 0.18 ± 0.12 | 0.07 ± 0.02 | 0.06 ± 0.02 |
| Lung | 10.1 ± 1.34 | 5.02 ± 0.89 | 0.74 ± 0.12 | 0.45 ± 0.18 | 0.14 ± 0.08 | 0.07 ± 0.02 |
| Brain | 0.20 ± 0.03 | 0.14 ± 0.02 | 0.03 ± 0.01 | 0.02 ± 0.01 | 0.01 ± 0.00 | 0.01 ± 0.01 |
| Muscle | 1.44 ± 0.24 | 0.63 ± 0.19 | 0.07 ± 0.02 | 0.04 ± 0.05 | 0.05 ± 0.02 | 0.01 ± 0.01 |
| LNCaP Tumor | 12.5 ± 0.26 | 13.7 ± 3.24 | 8.64 ± 2.27 | 8.10 ± 2.67 | 4.48 ± 0.78 | 1.76 ± 0.45 |

### • Reference Example 3-2 ([¹¹¹In]In-Octapa-3; Mean value ± standard deviation, and each n = 3).

**[Table 9]**

| [¹¹¹In]In-Octapa-3 | 1 h | 4 h | 24 h | 48 h | 96 h | 192 h |
|---|---|---|---|---|---|---|
| Blood | 27.2 ± 2.92 | 19.3 ± 3.84 | 7.20 ± 0.50 | 3.88 ± 1.13 | 0.63 ± 0.32 | 0.03 ± 0.00 |
| Spleen | 8.56 ± 0.95 | 7.42 ± 0.88 | 2.87 ± 0.40 | 1.76 ± 0.39 | 0.88 ± 0.32 | 0.31 ± 0.11 |
| Pancreas | 2.59 ± 0.26 | 2.17 ± 0.15 | 1.24 ± 0.16 | 0.60 ± 0.13 | 0.22 ± 0.06 | 0.07 ± 0.01 |
| Stomach (% ID) | 0.49 ± 0.04 | 0.78 ± 0.28 | 0.25 ± 0.08 | 0.13 ± 0.04 | 0.06 ± 0.03 | 0.01 ± 0.00 |
| Intestine | 2.60 ± 0.48 | 3.23 ± 0.18 | 1.23 ± 0.28 | 0.62 ± 0.14 | 0.29 ± 0.09 | 0.05 ± 0.01 |
| Kidney | 57.1 ± 17.4 | 100 ± 6.33 | 26.4 ± 2.50 | 17.3 ± 5.57 | 4.33 ± 2.06 | 0.27 ± 0.02 |
| Liver | 4.32 ± 0.40 | 3.36 ± 0.39 | 1.73 ± 0.09 | 1.42 ± 0.08 | 0.70 ± 0.25 | 0.35 ± 0.04 |
| Heart | 6.53 ± 0.66 | 5.98 ± 0.93 | 2.50 ± 0.17 | 1.37 ± 0.18 | 0.43 ± 0.17 | 0.12 ± 0.02 |
| Lung | 16.7 ± 1.41 | 14.1 ± 2.06 | 5.85 ± 0.39 | 3.18 ± 0.68 | 0.78 ± 0.29 | 0.12 ± 0.04 |
| Brain | 0.40 ± 0.05 | 0.31 ± 0.07 | 0.16 ± 0.01 | 0.12 ± 0.02 | 0.07 ± 0.02 | 0.02 ± 0.01 |
| Muscle | 1.51 ± 0.23 | 1.26 ± 0.18 | 0.61 ± 0.04 | 0.26 ± 0.05 | 0.08 ± 0.03 | 0.01 ± 0.02 |
| LNCaP Tumor | 7.79 ± 1.11 | 11.3 ± 2.39 | 8.04 ± 0.38 | 7.79 ± 2.72 | 4.17 ± 0.80 | 1.31 ± 0.49 |

### • Comparative Example 2-2 ([¹¹¹In]In-Neunpa-1; Mean value ± standard deviation, n = 3 for each, and n = 2 only for the point of 192 hours).

**[Table 10]**

| [¹¹¹In] In-Neunpa-1 | 1 h | 4 h | 24 h | 48 h | 96 h | 192 h |
|---|---|---|---|---|---|---|
| Blood | 2.30 ± 0.28 | 1.24 ± 0.15 | 0.20 ± 0.02 | 0.09 ± 0.03 | 0.06 ± 0.02 | 0.01 |
| Spleen | 6.83 ± 1.70 | 4.76 ± 1.17 | 2.74 ± 0.07 | 2.62 ± 0.79 | 2.47 ± 0.60 | 1.05 |
| Pancreas | 0.57 ± 0.14 | 0.50 ± 0.13 | 0.28 ± 0.03 | 0.38 ± 0.06 | 0.32 ± 0.02 | 0.37 |
| Stomach (% ID) | 0.17 ± 0.02 | 0.11 ± 0.03 | 0.05 ± 0.00 | 0.09 ± 0.06 | 0.07 ± 0.03 | 0.02 |
| Intestine | 0.47 ± 0.03 | 0.66 ± 0.08 | 0.32 ± 0.02 | 0.32 ± 0.06 | 0.16 ± 0.07 | 0.06 |
| Kidney | 214 ± 53.0 | 125 ± 89.2 | 13.5 ± 0.43 | 25.2 ± 17.1 | 15.1 ± 4.76 | 2.79 |
| Liver | 0.60 ± 0.07 | 0.69 ± 0.05 | 0.69 ± 0.05 | 0.80 ± 0.13 | 0.77 ± 0.07 | 0.63 |
| Heart | 0.70 ± 0.03 | 0.47 ± 0.08 | 0.21 ± 0.01 | 0.18 ± 0.05 | 0.28 ± 0.06 | 0.22 |
| Lung | 1.75 ± 0.18 | 1.03 ± 0.13 | 0.34 ± 0.05 | 0.32 ± 0.04 | 0.39 ± 0.06 | 0.20 |
| Brain | 0.05 ± 0.00 | 0.04 ± 0.00 | 0.02 ± 0.01 | 0.03 ± 0.01 | 0.03 ± 0.00 | 0.03 |
| Muscle | 0.28 ± 0.06 | 0.11 ± 0.04 | 0.10 ± 0.00 | 0.10 ± 0.03 | 0.09 ± 0.03 | 0.07 |
| LNCaP Tumor | 1.06 ± 0.21 | 0.69 ± 0.30 | 0.28 ± 0.02 | 0.30 ± 0.11 | 0.32 ± 0.09 | 0.15 |

### • Reference Example 3-3 ([¹¹¹In]In-Neunpa-2; Mean value ± standard deviation, and each n = 3).

**[Table 11]**

| [¹¹¹In]In-Neunpa-2 | 1 h | 4 h | 24 h | 48 h | 96 h | 192 h |
|---|---|---|---|---|---|---|
| Blood | 13.6 ± 0.68 | 5.73 ± 0.26 | 2.48 ± 0.07 | 0.20 ± 0.02 | 0.13 ± 0.12 | 0.02 ± 0.02 |
| Spleen | 16.9 ± 2.52 | 8.16 ± 3.89 | 2.67 ± 0.59 | 0.83 ± 0.20 | 0.47 ± 0.23 | 0.88 ± 0.34 |
| Pancreas | 1.61 ± 0.42 | 0.82 ± 0.05 | 0.65 ± 0.05 | 0.25 ± 0.09 | 0.18 ± 0.12 | 0.29 ± 0.11 |
| Stomach (% ID) | 0.23 ± 0.06 | 0.29 ± 0.18 | 0.13 ± 0.06 | 0.06 ± 0.03 | 0.03 ± 0.01 | 0.02 ± 0.01 |
| Intestine | 1.22 ± 0.30 | 1.05 ± 0.29 | 0.68 ± 0.11 | 0.23 ± 0.05 | 0.17 ± 0.10 | 0.10 ± 0.05 |
| Kidney | 90.1 ± 5.81 | 126 ± 17.6 | 101 ± 18.7 | 11.7 ± 3.83 | 9.71 ± 10.8 | 1.83 ± 0.67 |
| Liver | 2.23 ± 0.34 | 1.29 ± 0.02 | 1.00 ± 0.15 | 0.58 ± 0.23 | 1.30 ± 1.30 | 0.64 ± 0.24 |
| Heart | 3.74 ± 0.04 | 1.78 ± 0.15 | 0.79 ± 0.08 | 0.14 ± 0.03 | 0.14 ± 0.11 | 0.22 ± 0.10 |
| Lung | 8.99 ± 1.68 | 4.14 ± 0.61 | 2.26 ± 0.29 | 0.40 ± 0.10 | 0.36 ± 0.24 | 0.29 ± 0.07 |
| Brain | 0.18 ± 0.00 | 0.12 ± 0.01 | 0.08 ± 0.01 | 0.03 ± 0.00 | 0.03 ± 0.02 | 0.02 ± 0.00 |
| Muscle | 1.09 ± 0.25 | 0.61 ± 0.11 | 0.27 ± 0.11 | 0.05 ± 0.04 | 0.03 ± 0.02 | 0.08 ± 0.00 |
| LNCaP Tumor | 7.44 ± 0.18 | 8.57 ± 0.61 | 8.61 ± 1.06 | 4.92 ± 0.70 | 3.35 ± 0.90 | 5.16 ± 0.23 |

### <Evaluation of SPECT/CT using tumor-transplanted mouse>

A mouse transplanted simultaneously with tumors of LNCaP cells and PC-3 cells was obtained in the same manner as in Example 2, and then a saline solution (7.3 MBq, 150 µL) of Reference Example 3-1 ([¹¹¹In]In-Octapa-2) was administered from the tail vein. SPECT/CT was performed 24 hours after administration in the same manner as in Example 2 and the obtained image was reconstructed.

SPECT/CT results are shown in Figure 14.

In SPECT/CT imaging using [¹¹¹In]In-Octapa-2, 24 hours after administration, high radioactivity accumulation was observed in the LNCaP tumor (the solid arrow in the figure), but radioactivity signal was hardly observed in the PC-3 tumor (the dashed arrow in the figure). Radioactivity accumulation at the kidney (the circle in the figure) was also observed. This result showed that [¹¹¹In]In-Octapa-2 was capable of clearly drawing the highly PSMA-expressing tumor.

### <Examples 4-1 to 4-2>

In Examples 4-1 to 4-2, synthesized was a compound (PSMA-NAT-DA1) that has a structure containing (((S)-5-amino-1-carboxypentyl)carbamoyl)-L-glutamic acid (In the formula (C1), "a" is 2, and "b" is 4.), whose target molecule is PSMA, as the PSMA molecule-binding part, and has a chemical structure linearly containing the PSMA molecule-binding part and the albumin-binding part through the chelating part as shown in the general formula (1). Next, radiolabeled compounds were obtained, in which PSMA-NAT-DA1 was coordinated with a ¹¹¹In ion or a ²²⁵Ac ion each as a radioactive metal. Details are described below.

PSMA-NAT-DA1 has a structure containing the chelating part, the PSMA molecule-binding part, and the albumin-binding part.

In PSMA-NAT-DA1, the chelating part and the PSMA molecule-binding part are indirectly bonded to each other through a chemical structure derived from trans-4-aminocyclohexane-1-carboxylic acid and naphthylglycine, and the chelating part and the atomic group containing the albumin-binding part are indirectly bonded to each other through a lysine-derived linker structure.

The outline of the synthesis route in Examples 4-1 to 4-2 is shown below.

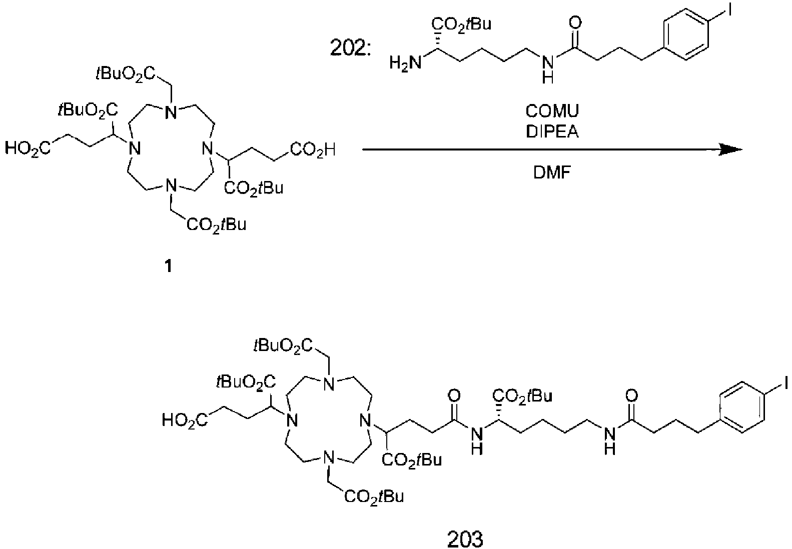

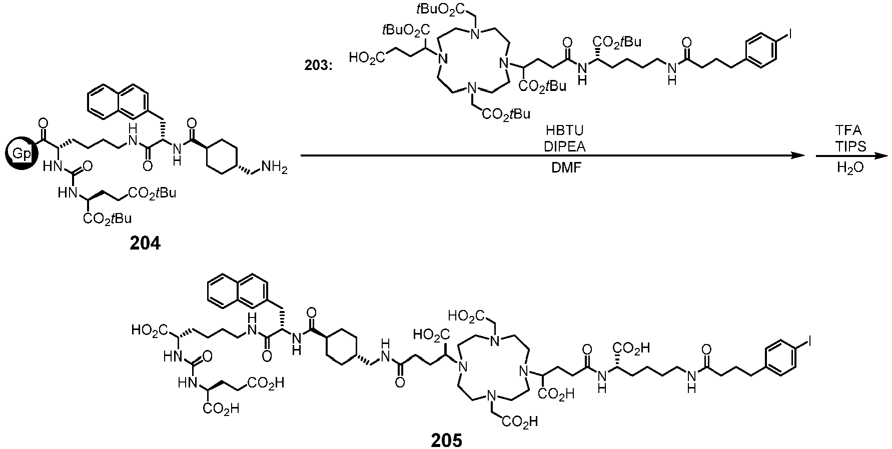

### (1) Synthesis of PSMA-NAT-DA1

PSMA-NAT-DA1 was synthesized according to the following procedures.

### <Synthesis of compounds 202 and 203>

Compound 1 was synthesized in the same manner as described in Example 1-1.

Compound 202 was synthesized in 4 steps from N2-[(9H-fluoren-9-ylmethoxy)carbonyl]-N6-[(4-methylphenyl)diphenylmethyl]-L-lysine according to a previously reported method (Chem Commun. 2021, 57, 6432-6435).

N-[1-(cyano-2-ethoxy-2-oxoethylideneaminooxy)dimethylamino(morpholino)] uronium hexafluorophosphate (COMU) (72.4 mg, 0.17 mmol) and N,N-diisopropylethylamine (DIPEA) (95 µL, 0.17 mmol) were added under ice cooling to a solution of compound 1 (131 mg, 0.17 mmol) in N,N-dimethylformamide (DMF) (0.4 mL), and the mixture was stirred for 15 minutes. Compound 202 (66.8 mg, 0.14 mmol) was added, and the mixture was stirred at room temperature for 5 days. Then, the solution was purified by reverse phase HPLC to obtain compound 203.

Purification conditions: Cosmosil 5C₁₈-AR-II column (10 × 250 mm); mobile phase: MeCN/H₂O [3/7 (0 min) to 9/1 (60 min)]; flow rate: 5 mL/min.

MS(ESI):m/z1230[M+H]⁺.

### <Synthesis of compound 204>

Compound 204 was obtained in 9 steps from Fmoc-Lys (ivDde)-OH with reference to the method previously reported (J. Med. Chem. 2016, 59, 1761-1775, Mol. Pharm. 2018, 15, 3502-3511).

In the chemical formula of compound 204, the chemical structure represented by symbol Gp represents a resin.

### <Synthesis of compound 205>

Compound 203 (49.6 mg, 0.044 mmol), 1-[bis(dimethylamino)methylene]-1H-benzotriazolium 3-oxid hexafluorophosphate (HBTU) (16.7 mg, 0.44 mmol), and DIPEA (188 µL, 0.44 mmol) were added to compound 204, to which a resin was bonded, and the mixture was shaken in DMF overnight. After washing with DMF, trifluoroacetic acid (TFA)/triisopropylsilane/H₂O (95 : 2.5 : 2.5, 2 mL) was added. After shaking for 3 hours, the solution was purified by reverse phase HPLC under the following conditions to obtain the desired compound 205 (PSMA-NAT-DA1).

Purification conditions: Cosmosil 5C₁₈-AR-II column (4.6 × 250 mm); mobile phase: MeCN/H₂O/TFA [10/90/0.1 (0 min) to 90/10/0.1 (40 min)]; flow rate: 5 mL/min.

Recovery amount: 11 mg (16%; calculated from the amount of substance of compound 205 obtained relative to the amount of substance of compound 203).

MS(ESI)m/z:MS(ESI)m/z793 [M+2H]²⁺.

### (2) ¹¹¹In labeling (Example 4-1)

The desired radiolabeled compound ([¹¹¹In]In-PSMA-NAT-DA1) was obtained according to the following procedures.

A solution (3.7 MBq, 100 µL) of [¹¹¹In]InCl₃ and a dimethyl sulfoxide solution (1 µg/µL, 5 µL) of compound 205 were added to a 2-morpholinoethanesulfonic acid buffer (0.1 M, pH 5.7, 150 µL), and the mixture was allowed to stand at 90 °C for 30 minutes. Thereafter, the reaction solution was purified by reverse phase HPLC to obtain a radiolabeled compound ([¹¹¹In]In-PSMA-NAT-DA1).

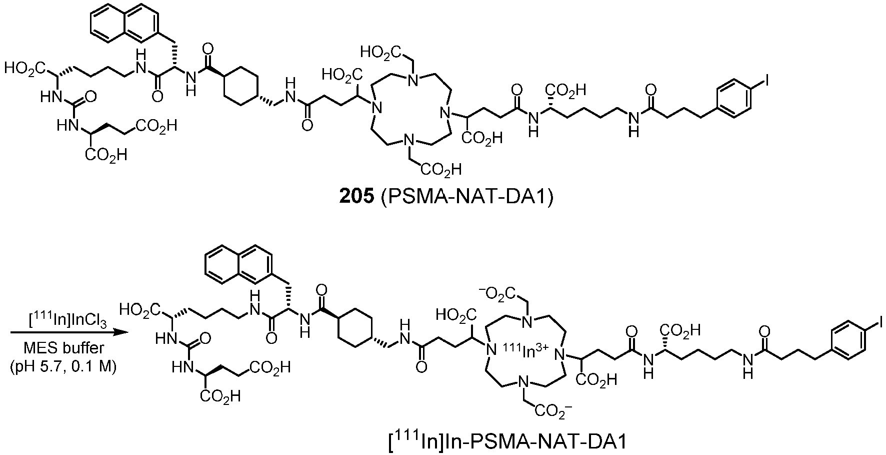

The radiochemical purity of the obtained [¹¹¹In]In-PSMA-NAT-DA1 was measured by the following method. That is, a part of the HPLC preparative liquid of [¹¹¹In]In-PSMA-NAT-DA1 was analyzed again under the following HPLC conditions, and the percentage of the area value of [¹¹¹In]In-PSMA-NAT-DA1 to the area value of all detected peaks was defined as radiochemical purity (%).

The radiochemical yield was measured in the same manner as described in Example 1-1. HPLC conditions used for measuring the radiochemical purity are shown below.

As a result, the radiochemical yield was 9%, and the radiochemical purity was 95% or more. When the same synthesis was performed again, the radiochemical yield was improved to 60%.

Purification conditions: Cosmosil 5C₁₈-AR-II column (4.6 × 150 mm); mobile phase: MeCN/H₂O/TFA [10/90/0.1 (0 min) to 90/10/0.1 (40 min)]; flow rate: 1 mL/min.

### (3) ²²⁵Ac labeling (Example 4-2)

The desired radiolabeled compound ([²²⁵Ac]Ac-PSMA-NAT-DA1) was obtained according to the following procedures.

To a 0.2 mol/L hydrochloric acid solution (1.0 MBq, 20 µL) of [²²⁵Ac]AcCl₃ dispensed in a Protein LoBind Tube 1.5mL (manufactured by EPPENDORF), 0.1 M acetic acid-ammonium acetate buffer (pH 5.5, 170 µL) and a DMSO solution (2.0 mM/L, 10 µL) of PSMA-NAT-DA1 were added, and the mixture was allowed to stand at 70°C for 1 hour. H₂O (0.8 mL) was added to the reaction solution, and the mixture was passed through an Oasis HLB Light column. After H₂O (10 mL) was passed through the column, 70% EtOH (0.5 mL) was passed through the column to obtain a 70% ethanol solution of the desired radiolabeled compound ([²²⁵Ac]Ac-PSMA-NAT-DA1).

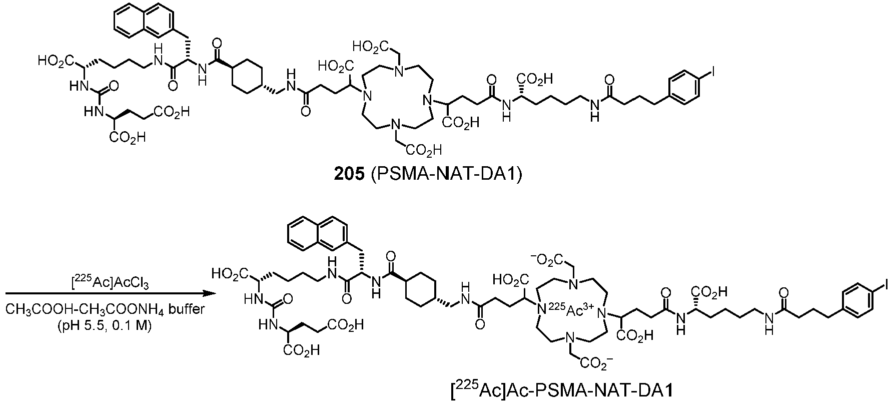

The radiochemical yield was measured by the following method. The radioactivity of the obtained radiolabeled compound was measured with a gamma ray spectrometer, and the percentage to the radioactivity of the solution of [²²⁵Ac]AcCl₃ used in the reaction was defined as radiochemical yield (%).

The radiochemical purity of the obtained radiolabeled compound was measured by the following method. That is, a part of the solution of the radiolabeled compound was analyzed by TLC (iTLC-SG; mobile phase: mixed solution of H₂O/MeCN = 1 : 1), and the percentage of the area value of the radiolabeled compound to the area value of all detected peaks was defined as radiochemical purity (%). As a result, the radiochemical yield was 35%, and the radiochemical purity was 86%.

### <Binding assay using cultured cells>

LNCaP cells and PC-3 cells cultured in the same method as in Example 1-1 were each seeded on a 12 well plate at 4.0 × 10⁵ cells/well, and left standing at 37°C under 5% CO₂ for 48 hours.

The culture medium was removed, and an assay medium (0.5% FBS-containing RPMI 1640 medium) solution (1 mL) containing [¹¹¹In]In-PSMA-NAT-DA1(8.5 kBq) was added. Thereafter, the plate was allowed to stand at 37°C under 5% CO₂ for 1 hour.

In the inhibition experiments, after removing the culture medium, an assay medium (1 mL) solution containing: [¹¹¹In]In-PSMA-NAT-DA1 (37 kBq); and 2-(phosphonomethyl) pentanedioic acid (2-PMPA) (PSMAinhibitor; final concentration: 100 µM) was added. Thereafter, the plate was allowed to stand at 37°C under 5% CO₂ for 1 hour.

After removing the assay medium, each well was washed with assay medium (1 mL) containing neither the radiolabeled compound nor 2-PMPA, and the cells were lysed with 1 N aqueous sodium hydroxide solution (200 µL × 2).

The radioactivity for each of the assay medium and the cell lysate was measured with a gamma counter. Separately, the total protein concentration in the cell lysate was calculated using BCA Protein Assay Kit, which is manufactured by Thermo Fisher Scientific K.K. The value (% ID/mg protein) obtained by dividing the percentage (% ID) of the sample's radioactivity amount to the added radioactivity amount by the total protein amount was calculated for each sample.

The data were expressed as mean value ± standard deviation. The significant difference test was performed using Student's t-test and one-way analysis of variance (ANOVA) test with Dunnet's post-hoc test, and p < 0.05 was defined as having a significant difference.

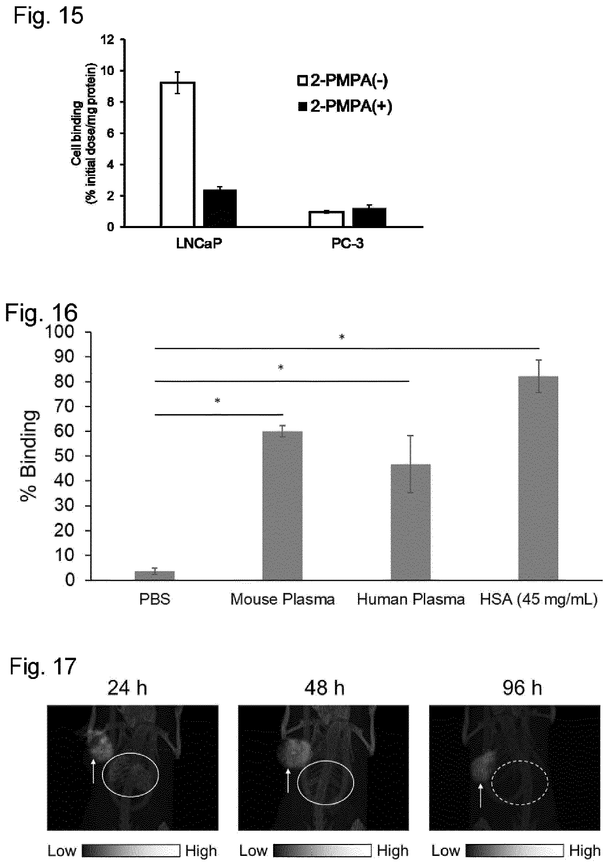

The results of the evaluation of binding to cultured cells are shown in Fig. 15. The higher the value, the higher the abundance of the radiolabeled compound, which indicates that the compound is highly accumulated.

[¹¹¹In]In-PSMA-NAT-DA1 showed high binding ability to LNCaP cells compared to PC-3 cells, and the bond was significantly reduced by adding an excess amount of the PSMA inhibitor (2-PMPA). These results showed that [¹¹¹In]In-PSMA-NAT-DA1 specifically binds to highly PSMA-expressing cells.

### <Binding assay to albumin>

A phosphate buffered saline (PBS) solution (37 kBq, 50 µL) of [¹¹¹In]In-PSMA-NAT-DA1 was added to 200 µL of a PBS solution (45 mg/mL) of human serum albumin (HSA), mouse plasma, human plasma, or PBS, and the mixture was allowed to stand at 37°C for 10 minutes. Thereafter, the reaction liquid was added to a spin column (Sephadex G-50; manufactured by Cytiva), and centrifuged at 1500 × g at 4°C for 2 minutes. After separation, the radioactivity of the column and the eluate was measured with a gamma counter.

The data were expressed as mean value ± standard deviation. The significant difference test was performed using Student's t-test and one-way analysis of variance (ANOVA) test with Dunnet's post-hoc test, and p < 0.05 was defined as having a significant difference.

The results of the evaluation of binding to albumin are shown in Fig. 16. The higher the value, the higher the binding ability to albumin.

It is considered that: when a compound to be evaluated binds to albumin to form a composite, the compound passes through the column because of the increased molecular size; and when it does not bind to albumin, it is retained on the gel in the column.

When [¹¹¹In]In-PSMA-NAT-DA1 was allowed to stand in PBS and then applied to the column, no significant radioactivity was observed in the eluate. On the other hand, when left standing in mouse plasma, human plasma, and the HSA solution, the radioactivity in the eluate of [¹¹¹In]In-PSMA-NAT-DA1 was significantly high, suggesting that [¹¹¹In]In-PSMA-NAT-DA1 binds to plasma albumin.

### <Evaluation of internal radioactivity distribution using LNCaP or PC-3 tumor-transplanted mouse>

The animal test was performed in compliance with the guidelines of Kyoto University Animal Experiment Committee. Male CBI7/IcrJcl-Prkdc^{scid} mice were purchased from CLEAJapan, Inc. The animals were kept under 12 h/12 h, day/night cycle conditions and fed ad libitum with food and water. LNCaP cells (4.3 × 10⁶ cells/mouse) or PC-3 cells (3.3 × 10⁶ cells/mouse) were suspended in a mixture of PBS and Matrigel manufactured by Corning Life Sciences (1 : 1, 150 µL) and the suspension was subcutaneously transplanted to the right shoulder of CBI7/IcrJcl-Prkdc^{scid} mice under isoflurane anesthesia. Thereafter, the mice were kept for 40 to 60 days.

LNCaP or PC-3 tumor-transplanted mice were administered a saline solution (259 kBq, 100 µL) of [¹¹¹In]In-PSMA-NAT-DA1 from the tail vein (n = 3 each). Mice were euthanized 1, 4, 24, 48, 96, and 192 hours after administration. Thereafter, blood and each organ were recovered, and the mass and radioactivity of the organs were measured.

LNCaP tumor-transplanted mice were administered [¹¹¹In]In-PSMA-NAT-DA1, and the pharmacokinetics (% ID/g) was evaluated. The results are shown in Table 12 (Mean value ± standard deviation, n = 3).

**[Table 12]**

| [¹¹¹In]In-PSMA-NAT-DA1 | 1 h | 4 h | 24 h | 48 h | 96 h | 192 h |
|---|---|---|---|---|---|---|
| Blood | 39.8 ± 2.91 | 38.8 ± 8.25 | 10.9 ± 5.55 | 12.3 ± 1.56 | 4.07 ± 1.37 | 2.47 ± 0.52 |
| Spleen | 31.2 ± 2.84 | 23.0 ± 3.16 | 13.5 ± 8.18 | 17.8 ± 1.05 | 6.33 ± 5.25 | 3.77 ± 0.54 |
| Pancreas | 7.32 ± 1.27 | 5.85 ± 1.34 | 2,15 ± 1.39 | 2.26 ± 0.15 | 2.60 ± 3.18 | 0.94 ± 0.32 |
| Stomach (% ID) | 0.94 ± 0.35 | 1.32 ± 0.19 | 0.28 ± 0.16 | 0.44 ± 0.09 | 0.19 ± 0.10 | 0.12 ± 0.01 |
| Intestine | 3.40 ± 0.24 | 3.47 ± 0.42 | 1.26 ± 0.70 | 1.39 ± 0.06 | 0.86 ± 0.54 | 0.55 ± 0.15 |
| Kidney | 41.5 ± 4.27 | 66.2 ± 13.0 | 37.1 ± 19.3 | 79.0 ± 6.78 | 22.0 ± 14.9 | 11.9 ± 2.08 |
| Liver | 8.24 ± 2.38 | 8.38 ± 2.27 | 2.73 ± 1.08 | 4.49 ± 0.71 | 2.31 ± 0.89 | 2.25 ± 0.45 |
| Heart | 10.8 ± 1.31 | 12.2 ± 2.44 | 3.14 ± 1.52 | 4.68 ± 0.92 | 2.10 ± 0.83 | 1.19 ± 0.24 |
| Lung | 28.1 ± 8.65 | 26.5 ± 5.70 | 8.76 ± 4.02 | 11.1 ± 2.24 | 4.55 ± 2.33 | 2.81 ± 0.36 |
| Brain | 0.63 ± 0.03 | 0.83 ± 0.20 | 0.27 ± 0.13 | 0.45 ± 0.03 | 0.18 ± 0.04 | 0,26 ± 0.02 |
| Muscle | 2.82 ± 0.55 | 3.69 ± 0.83 | 1.23 ± 0.36 | 1.48 ± 0.31 | 0.59 ± 0.09 | 0.33 ± 0.05 |
| LNCaP Tumor | 23.0 ± 2.20 | 43.6 ± 8.49 | 51.9 ± 39.3 | 131.6 ± 22.0 | 56.2 ± 35.6 | 35.0 ± 27.3 |

[¹¹¹In]In-PSMA-NAT-DA1 showed high accumulation at the LNCaP tumor (23.0 to 131.6% ID/g; 1 to 48 hours after administration). In addition, retention in blood was exhibited (12.3% ID/g; 48 hours after administration), and 24 hours after administration, the tumor/kidney ratio exceeded 1. These results revealed that [¹¹¹In]In-PSMA-NAT-DA1 has high accumulation at the highly PSMA-expressing tumor.

A PC-3 tumor-transplanted mouse was administered [¹¹¹In]In-PSMA-NAT-DA1, and the pharmacokinetics (% ID/g) was evaluated. The results are shown in Table 13 (Mean value ± standard deviation, n = 3).

**[Table 13]**

| | 24 h |
|---|---|
| Blood | 5.73 ± 1.10 |
| Spleen | 6.94 ± 4.55 |
| Pancreas | 1.25 ± 0.04 |
| Stomach (% ID) | 0.15 ± 0.03 |
| Intestine | 0.68 ± 0.07 |
| Kidney | 27.9 ± 605 |
| Liver | 1.74 ± 0.37 |
| Heart | 2.67 ± 0.58 |
| Lung | 7.41 ± 1.13 |
| Brain | 0.22 ± 0.03 |
| Muscle | 0.83 ± 0.26 |
| PC-3 Tumor | 3.21 ± 1.00 |

[¹¹¹In]In-PSMA-NAT-DA1 accumulation at the PC-3 tumor showed a significantly lower value than that of accumulation at the LNCaP tumor at the same time point, indicating that the radiolabeled compounds selectively accumulate at the PSMA-positive tumor.

### <SPECT/CT using LNCaP tumor-transplanted mouse>

A LNCaP tumor-implanted mouse prepared by the method described above was administered a saline solution (1.57 to 2.59 MBq, 150 µL) of [¹¹¹In]In-PSMA-NAT-DA1 from the tail vein. SPECT/CT was performed 24, 48, and 96 hours after administration with FX3300 pre-clinical imaging system, which is manufactured by Gamma Medica-Ideas. Imaging was performed using a pinhole collimator with a diameter of 1.0 mm at a rotation radius of 35 mm, a projection time of 70 seconds, and the number of projections of 32 times under isoflurane anesthesia. After SPECT, CT (Tube voltage: 60 kV, Tube current: 350 µA) was performed. Image reconstruction was performed on the projection data of SPECT through three-dimensional ordered subset expectation maximization method (8 subsets, 5 iterations).

SPECT/CT results are shown in Figure 17. In the figure, the part indicated by the arrow is the location of the tumor, and the part indicated by the circle is the location of the kidney. The higher the SUV, the higher the radioactivity accumulation.

In the SPECT/CT imaging, significant radioactivity accumulation was observed at the LNCaP tumor (the arrow in the figure) 24 and 48 hours after administration. Radioactivity accumulation was also observed at the kidney (the solid circles in the figure) 24 and 48 hours after administration, but was hardly observed 96 hours after administration (the broken line circles in the figure). From these results, it has been indicated that [¹¹¹In]In-PSMA-NAT-DA1 can clearly draw the highly PSMA-expressing tumor through SPECT and is superior to [¹¹¹In]In-PSMA-DB.

## Claims

1. A compound represented by the following general formula (1): 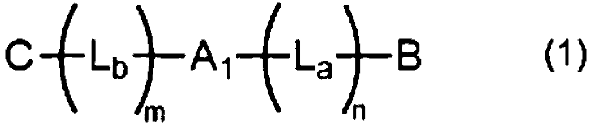
wherein, in the general formula (1), A₁ represents a chelating part capable of coordinating with a radioactive metal ion;
B represents an atomic group containing an albumin-binding part;
C represents an atomic group containing a PSMA molecule-binding part;
Lₐ represents a linker structure comprising a structure derived from a compound capable of forming an amide bond;
L_{b} represents a linker structure that is identical to or different from Lₐ, comprising a structure derived from an amino acid having a structure containing an alicyclic of 5 to 10 carbon atoms;
"m" and "n" each independently represent 0 or 1;
B or Lₐ is bonded to an arbitrary position of A₁; and
C or L_{b} is bonded to A₁ at a position different from the position at which B or Lₐ is bonded to A₁,
wherein, in the formula (1), the chelating part represented by A₁ has a structure derived from a compound represented by any one of the following formulas (A1) to (A9): 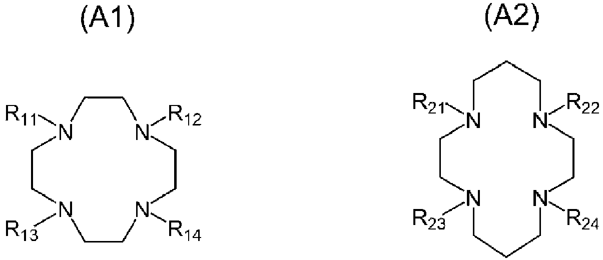 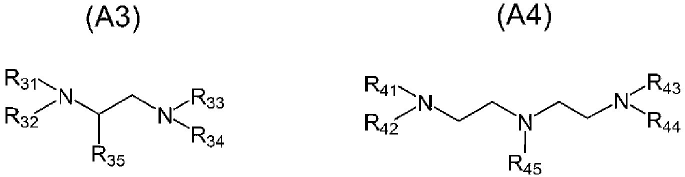 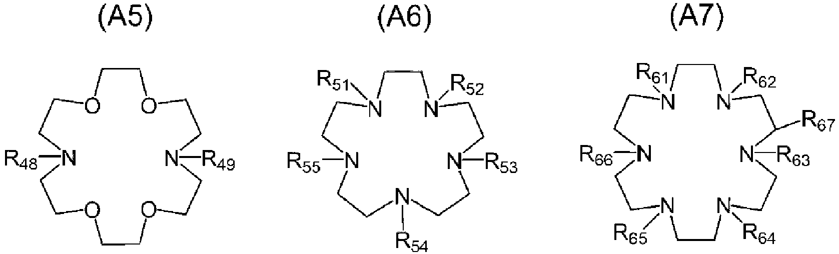 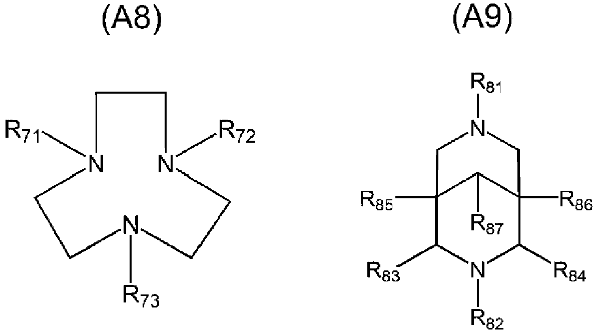
wherein in the formula (A1), R₁₁, R₁₂, R₁₃, and R₁₄ are each independently any of groups consisting of -(CH₂)ₚCOOH, -(CH₂)ₚC₅H₅N, -(CH₂)ₚPO₃H₂, - (CH₂)ₚCONH₂, and -(CHCOOH)(CH₂)ₚCOOH; and "p" is an integer of 0 or more and 3 or less;
wherein in the formula (A2), R₂₁, R₂₂, R₂₃, and R₂₄ each independently represent a carboxy group or a carboxyalkyl group having 2 or 3 carbon atoms;
wherein in the formula (A3), R₃₁, R₃₂, R₃₃, and R₃₄ each independently represent an atomic group having a hydrogen atom and 2 or more and 10 or less carbon atoms and optionally containing a nitrogen atom or an oxygen atom; and R₃₅ represents a hydrogen atom, a carboxy group, or a carboxyalkyl group having 2 or 3 carbon atoms;
wherein in the formula (A4), R₄₁, R₄₂, R₄₃, and R₄₄ each independently represent an atomic group having a hydrogen atom and 2 or more and 10 or less carbon atoms and optionally containing a nitrogen atom or an oxygen atom; and R_{4S} represents a hydrogen atom, a carboxy group, or a carboxyalkyl group having 2 or 3 carbon atoms;
wherein in the formula (A5), R₄₈ and R₄₉ each independently represent an atomic group having a hydrogen atom and 2 or more and 10 or less carbon atoms and optionally containing a nitrogen atom or an oxygen atom;
wherein in the formula (A6), R₅₁, R₅₂, R₅₃, R₅₄, and R₅₅ each independently represent an atomic group having a hydrogen atom and 2 or more and 10 or less carbon atoms and optionally containing a nitrogen atom or an oxygen atom;
wherein in the formula (A7), R₆₁, R₆₂, R₆₃, R₆₄, R₆₅, and R₆₆ each independently represent an atomic group having a hydrogen atom and 2 or more and 10 or less carbon atoms and optionally containing a nitrogen atom or an oxygen atom; and R₆₇ represents a hydrogen atom, a carboxy group, or a carboxyalkyl group having 2 or 3 carbon atoms;
wherein in the formula (A8), R₇₁, R₇₂ and R₇₃ each independently represent an atomic group having a hydrogen atom and 2 or more and 10 or less carbon atoms and optionally containing a nitrogen atom or an oxygen atom; and
wherein in the formula (A9), R₈₁ and R₈₂ are each independently an alkyl group having 1 or more and 5 or less carbon atoms, and the terminal of the alkyl group may be substituted with a pyridyl group substituted with 1 or more carboxy groups; R₈₇ is a hydroxyl group or the oxygen atom (=O) of a carbonyl group; R₈₃ and R₈₄ are a substituted or unsubstituted pyridinyl group; R₈₅ and R₈₆ are each independently -COO-Rₐ; and Rₐ is an alkyl group having 1 or more and 5 or less carbon atoms;
wherein, in the formula (1), the albumin-binding part has a structure represented by the following formula (B1) or (B2): 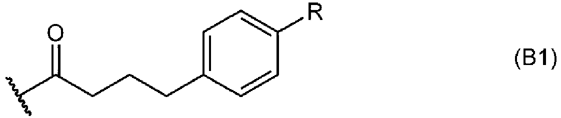 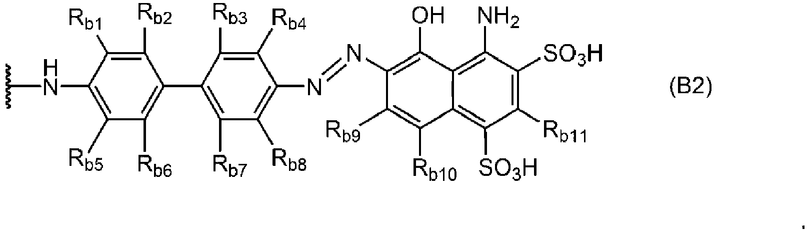
wherein, in the formula (B1), R represents a hydrogen atom, a halogen atom, or an alkyl group having 1 or more and 5 or less carbon atoms; and the wavy line part represents a part bonding to A₁ or Lₐ in the formula (1), and
in the formula (B2), R_{b1} to R_{b11} each independently represent a hydrogen atom, a halogen atom, a hydroxyl group, a cyano group, a substituted or unsubstituted alkyl group having 1 or more and 6 or less carbon atoms, or a substituted or unsubstituted alkoxy group having 1 or more and 6 or less carbon atoms; and the wavy line part represents a part bonding to A₁ or Lₐ in the formula (1); and
wherein, in the formula (1), C represents the following formula (C1): 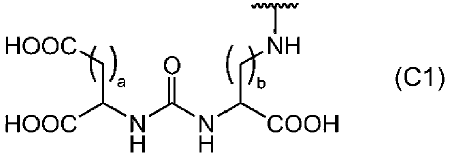
wherein, in the formula (C1), the wavy line part represents a part bonding to A₁ or L_{b} in the general formula (1); and
"a" and "b" each independently represent an integer of 1 or more and 7 or less.

2. The compound according to claim 1, wherein, in the formula (1), A₁ is DOTA, HOPO, Octapa, Macropa, or a derivative thereof.

3. The compound according to claim 1 or 2, wherein, in the formula (1), the albumin-binding part has a structure represented by the formula (B1).

4. The compound according to claim 1, wherein the compound represented by the general formula (1) is a compound represented by the following formula 205:

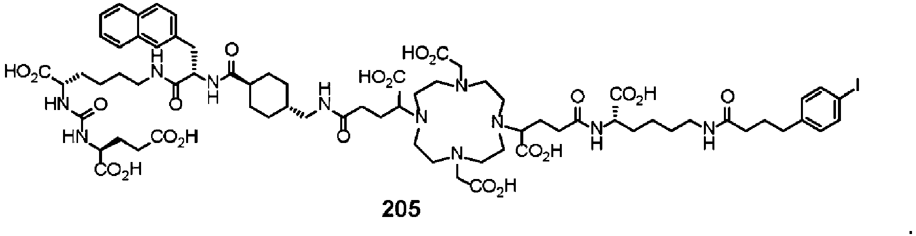

5. A radiolabeled compound comprising: a radioactive metal ion and the compound according to any one of claims 1 to 4 which is coordinated with the radioactive metal ion.

6. The radiolabeled compound according to claim 5, wherein the radioactive metal is ⁶⁸Ga, ⁶⁴Cu, ⁶⁷Cu, ⁸⁹Zr, ⁹⁰Y, ^{99m}Tc, ¹¹¹In, ¹⁷⁷Lu, ¹⁸⁶Re, ¹⁸⁸Re, or ²²⁵Ac.

7. A radioactive pharmaceutical composition comprising the radiolabeled compound according to claim 5 or 6, as an active ingredient.

8. A method for manufacturing a radiolabeled compound, the method comprising: coordinating the compound according to any one of claims 1 to 4 to a radioactive metal ion to obtain a radiolabeled compound.

9. The method for manufacturing a radiolabeled compound according to claim 8, wherein the compound used is represented by the following general formula (1S): 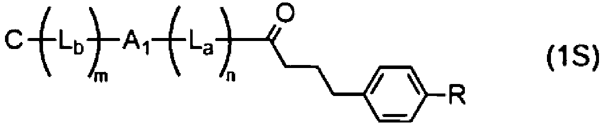
wherein, in the formula (1S), A₁ represents a chelating part capable of coordinating with a radioactive metal ion;
C represents an atomic group containing a PSMA molecule-binding part;
Lₐ represents a linker structure comprising a structure derived from a compound capable of forming an amide bond;
L_{b} represents a linker structure that is identical to or different from Lₐ, comprising a structure derived from an amino acid having a structure containing an alicyclic of 5 to 10 carbon atoms;
"m" and "n" each independently represent 0 or 1; and
R represents a hydrogen atom, a halogen atom, or an alkyl group having 1 or more and 5 or less carbon atoms; and
wherein, in the formula (1S), the chelating part represented by A₁ has a structure derived from a compound represented by any one of the following formulas (A1) to (A9): 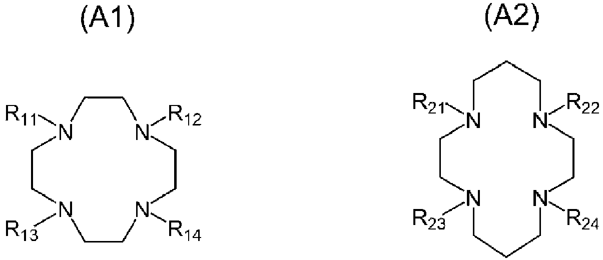 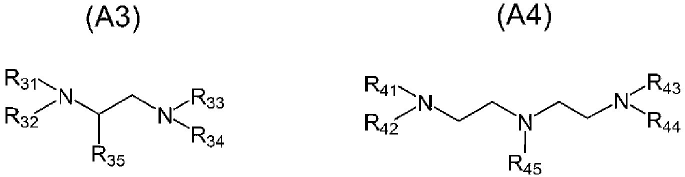 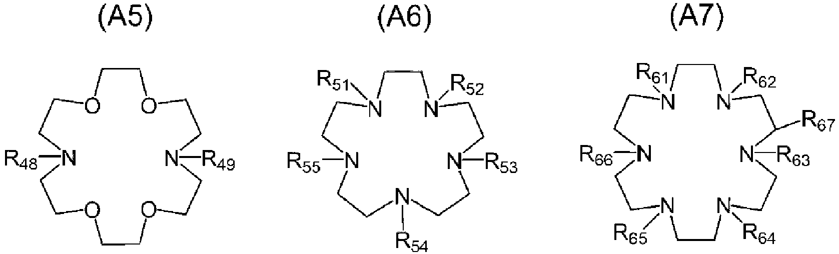 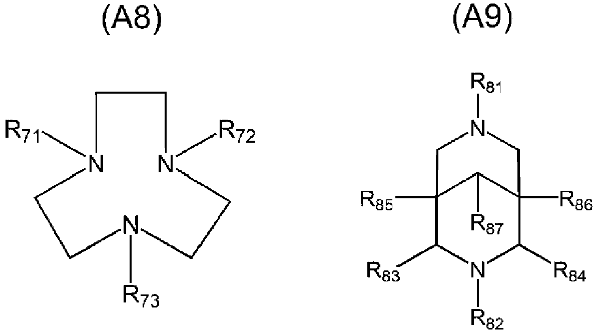
wherein in the formula (A1), R₁₁, R₁₂, R₁₃, and R₁₄ are each independently any of groups consisting of -(CH₂)ₚCOOH, -(CH₂)ₚC₅H₅N, -(CH₂)ₚPO₃H₂, - (CH₂)ₚCONH₂, and -(CHCOOH)(CH₂)ₚCOOH; and "p" is an integer of 0 or more and 3 or less;
wherein in the formula (A2), R₂₁, R₂₂, R₂₃, and R₂₄ each independently represent a carboxy group or a carboxyalkyl group having 2 or 3 carbon atoms;
wherein in the formula (A3), R₃₁, R₃₂, R₃₃, and R₃₄ each independently represent an atomic group having a hydrogen atom and 2 or more and 10 or less carbon atoms and optionally containing a nitrogen atom or an oxygen atom; and R₃₅ represents a hydrogen atom, a carboxy group, or a carboxyalkyl group having 2 or 3 carbon atoms;
wherein in the formula (A4), R₄₁, R₄₂, R₄₃, and R₄₄ each independently represent an atomic group having a hydrogen atom and 2 or more and 10 or less carbon atoms and optionally containing a nitrogen atom or an oxygen atom; and R₄₅ represents a hydrogen atom, a carboxy group, or a carboxyalkyl group having 2 or 3 carbon atoms;
wherein in the formula (A5), R₄₈ and R₄₉ each independently represent an atomic group having a hydrogen atom and 2 or more and 10 or less carbon atoms and optionally containing a nitrogen atom or an oxygen atom;
wherein in the formula (A6), R₅₁, R₅₂, R₅₃, R₅₄, and R₅₅ each independently represent an atomic group having a hydrogen atom and 2 or more and 10 or less carbon atoms and optionally containing a nitrogen atom or an oxygen atom;
wherein in the formula (A7), R₆₁, R₆₂, R₆₃, R₆₄, R₆₅, and R₆₆ each independently represent an atomic group having a hydrogen atom and 2 or more and 10 or less carbon atoms and optionally containing a nitrogen atom or an oxygen atom; and R₆₇ represents a hydrogen atom, a carboxy group, or a carboxyalkyl group having 2 or 3 carbon atoms;
wherein in the formula (A8), R₇₁, R₇₂ and R₇₃ each independently represent an atomic group having a hydrogen atom and 2 or more and 10 or less carbon atoms and optionally containing a nitrogen atom or an oxygen atom; and
wherein in the formula (A9), R₈₁ and R₈₂ are each independently an alkyl group having 1 or more and 5 or less carbon atoms, and the terminal of the alkyl group may be substituted with a pyridyl group substituted with 1 or more carboxy groups; R₈₇ is a hydroxyl group or the oxygen atom (=O) of a carbonyl group; R₈₃ and R₈₄ are a substituted or unsubstituted pyridinyl group; R₈₅ and R₈₆ are each independently -COO-Rₐ; and Rₐ is an alkyl group having 1 or more and 5 or less carbon atoms; and
wherein, in the formula (1S), C represents the following formula (C1): 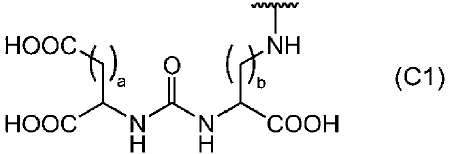
wherein, in the formula (C1), the wavy line part represents a part bonding to A₁ or L_{b} in the general formula (1); and
"a" and "b" each independently represent an integer of 1 or more and 7 or less.

## Patentansprüche

1. Verbindung, die dargestellt wird durch die folgende allgemeine Formel (1): 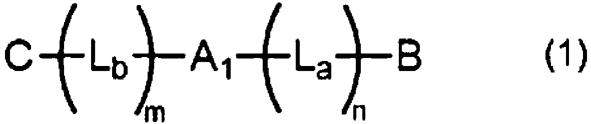
wobei in der allgemeinen Formel (1) A₁ einen Chelatbildungsteil darstellt, der fähig ist um mit einem radioaktiven Metallion zu koordinieren;
B eine Atomgruppe darstellt, die einen Albumin-Bindungsteil enthält;
C eine Atomgruppe darstellt, die einen PSMA-Molekül-Bindungsteil enthält;
Lₐ eine Linkerstruktur darstellt, die eine Struktur umfasst, die von einer Verbindung stammt, die fähig ist zur Bildung einer Amidbindung;
L_{b} eine Linkerstruktur darstellt, die mit Lₐ identisch oder von dieser verschieden ist, umfassend eine Struktur, die von einer Aminosäure stammt, die einen alicyclischen Rest mit 5 bis 10 Kohlenstoffatomen enthält;
"m" und "n" jeweils unabhängig voneinander 0 oder 1 darstellen;
B oder Lₐ an eine beliebige Position von A₁ gebunden ist; und
C oder L_{b} an A₁ an einer Position gebunden ist, die sich von der Position unterscheidet, an der B oder Lₐ an A₁ gebunden ist,
wobei in der Formel (1) der durch A₁ dargestellte Chelatbildungsteil eine Struktur aufweist, die von einer Verbindung stammt, die dargestellt wird durch eine der folgenden Formeln (A1) bis (A9): 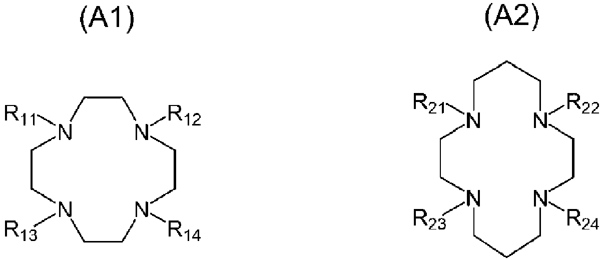 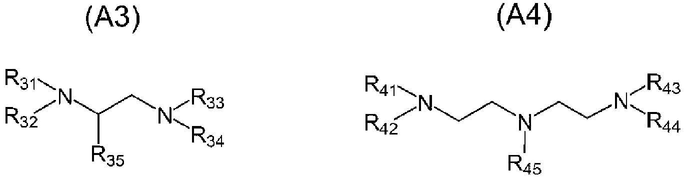 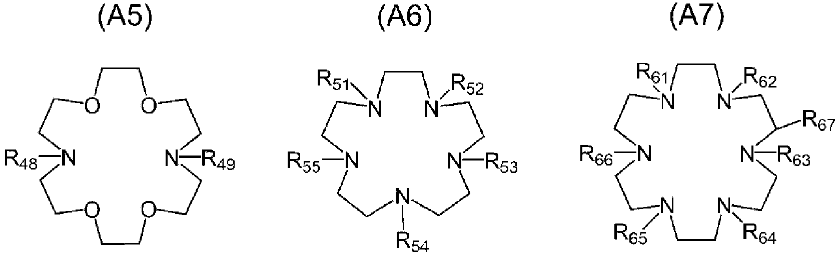 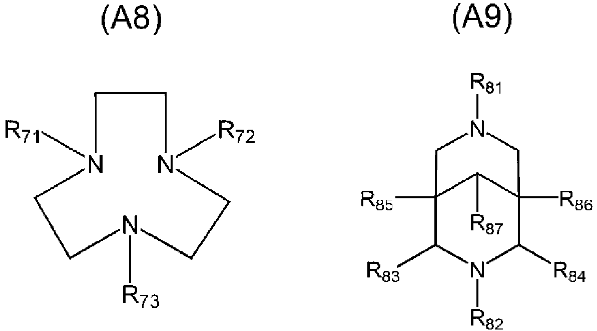
wobei in der Formel (A1) R₁₁, R₁₂, R₁₃ und R₁₄ jeweils unabhängig voneinander eine der Gruppen bestehend aus -(CH₂)ₚCOOH, -(CH₂)ₚC₅H₅N, -(CH₂)ₚPO₃H₂, -(CH₂)ₚCONH₂ und -(CHCOOH)(CH₂)ₚCOOH ist; und "p" eine ganze Zahl von 0 oder mehr und 3 oder weniger ist;
wobei in der Formel (A2) R₂₁, R₂₂, R₂₃ und R₂₄ jeweils unabhängig voneinander eine Carboxygruppe oder eine Carboxyalkylgruppe mit 2 oder 3 Kohlenstoffatomen darstellen;
wobei in der Formel (A3) R₃₁, R₃₂, R₃₃ und R₃₄ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten; und R₃₅ ein Wasserstoffatom, eine Carboxygruppe oder eine Carboxyalkylgruppe mit 2 oder 3 Kohlenstoffatomen darstellt;
wobei in der Formel (A4) R₄₁, R₄₂, R₄₃ und R₄₄ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten; und R₄₅ ein Wasserstoffatom, eine Carboxygruppe oder eine Carboxyalkylgruppe mit 2 oder 3 Kohlenstoffatomen darstellt;
wobei in der Formel (A5) R₄₈ und R₄₉ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten;
wobei in der Formel (A6) R₅₁, R₅₂, R₅₃, R₅₄ und R₅₅ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten;
wobei in der Formel (A7) R₆₁, R₆₂, R₆₃, R₆₄, R₆₅ und R₆₆ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten; und R₆₇ ein Wasserstoffatom, eine Carboxygruppe oder eine Carboxyalkylgruppe mit 2 oder 3 Kohlenstoffatomen darstellt;
wobei in der Formel (A8) R₇₁, R₇₂ und R₇₃ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthält; und
wobei in der Formel (A9) R₈₁ und R₈₂ jeweils unabhängig voneinander eine Alkylgruppe mit 1 oder mehr und 5 oder weniger Kohlenstoffatomen darstellen, und das Ende der Alkylgruppe mit einer Pyridylgruppe substituiert sein kann, die mit 1 oder mehr Carboxygruppen substituiert ist; R₈₇ eine Hydroxylgruppe oder das Sauerstoffatom (=O) einer Carbonylgruppe darstellt; R₈₃ und R₈₄ eine substituierte oder unsubstituierte Pyridinylgruppe darstellen; R₈₅ und R₈₆ jeweils unabhängig voneinander -COO-Rₐ darstellen; und Rₐ eine Alkylgruppe mit 1 oder mehr und 5 oder weniger Kohlenstoffatomen darstellt;
wobei in der Formel (1) der Albumin-Bindungsteil eine Struktur aufweist, die dargestellt wird durch die folgende Formel (B1) oder (B2): 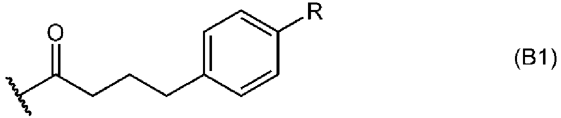 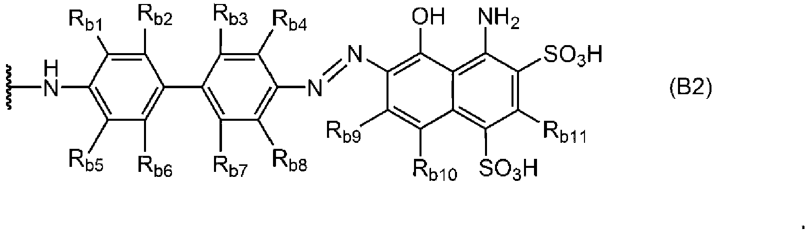
wobei in der Formel (B1) R ein Wasserstoffatom, ein Halogenatom oder eine Alkylgruppe mit 1 oder mehr und 5 oder weniger Kohlenstoffatomen darstellt; und der Wellenlinienabschnitt einen Abschnitt darstellt, der an A₁ oder Lₐ in der Formel (1) gebunden ist, und
wobei in der Formel (B2) R_{b1} bis R_{b11} jeweils unabhängig voneinander ein Wasserstoffatom, ein Halogenatom, eine Hydroxylgruppe, eine Cyanogruppe, eine substituierte oder unsubstituierte Alkylgruppe mit 1 oder mehr und 6 oder weniger Kohlenstoffatomen oder eine substituierte oder unsubstituierte Alkoxygruppe mit 1 oder mehr und 6 oder weniger Kohlenstoffatomen darstellen; und der Wellenlinienabschnitt einen Abschnitt darstellt, der an A₁ oder Lₐ in der Formel (1) gebunden ist; und
wobei in der Formel (1) C die folgende Formel (C1) darstellt: 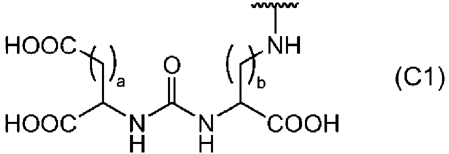
wobei in der Formel (C1) der Wellenlinienabschnitt einen Abschnitt darstellt, der an A₁ oder L_{b} in der allgemeinen Formel (1) gebunden ist; und
"a" und "b" jeweils unabhängig voneinander eine ganze Zahl von 1 oder mehr und 7 oder weniger darstellen.

2. Verbindung nach Anspruch 1, wobei in der Formel (1) A₁ DOTA, HOPO, Octapa, Macropa oder ein Derivat davon ist.

3. Verbindung nach Anspruch 1 oder 2, wobei in der Formel (1) der Albumin-Bindungsteil eine durch die Formel (B1) dargestellte Struktur aufweist.

4. Verbindung nach Anspruch 1, wobei die durch die allgemeine Formel (1) dargestellte Verbindung eine Verbindung ist, die dargestellt wird durch die folgende Formel 205:

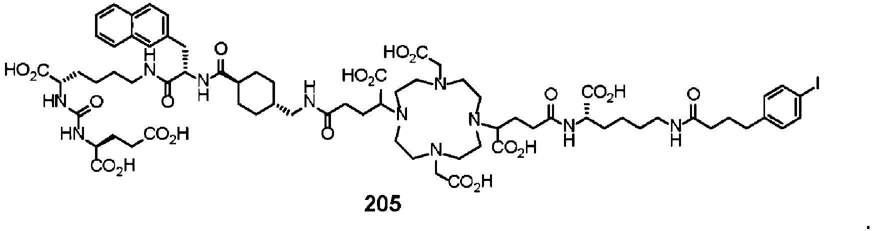

5. Radioaktiv markierte Verbindung, die umfasst: ein radioaktives Metallion und die Verbindung nach einem der Ansprüche 1 bis 4, die an das radioaktive Metallion koordiniert ist.

6. Radioaktiv markierte Verbindung nach Anspruch 5, wobei das radioaktive Metall ⁶⁸Ga, ⁶⁴Cu, ⁶⁷Cu, ⁸⁹Zr, ⁹⁰Y, ^{99m}Tc, ¹¹¹In, ¹⁷⁷Lu, ¹⁸⁶Re, ¹⁸⁸Re oder ²²⁵Ac ist.

7. Radioaktive pharmazeutische Zusammensetzung, die die radioaktiv markierte Verbindung nach Anspruch 5 oder 6 als Wirkstoff umfasst.

8. Verfahren zur Herstellung einer radioaktiv markierten Verbindung, wobei das Verfahren umfasst: Koordinieren der Verbindung nach einem der Ansprüche 1 bis 4 an ein radioaktives Metallion, um eine radioaktiv markierte Verbindung zu erhalten.

9. Verfahren zur Herstellung einer radioaktiv markierten Verbindung nach Anspruch 8, wobei die verwendete Verbindung dargestellt wird durch die folgende allgemeine Formel (1S): 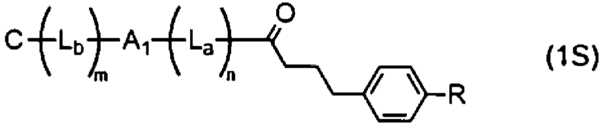
wobei in der Formel (1S) A₁ einen Chelatbildungsteil darstellt, der fähig ist um mit einem radioaktiven Metallion zu koordinieren;
C eine Atomgruppe darstellt, die einen PSMA-Molekül-Bindungsteil enthält;
Lₐ eine Linkerstruktur darstellt, die eine Struktur umfasst, die von einer Verbindung stammt, die fähig ist zur Bildung einer Amidbindung;
L_{b} eine Linkerstruktur darstellt, die mit Lₐ identisch oder von dieser verschieden ist, umfassend eine Struktur, die von einer Aminosäure stammt, die einen alicyclischen Rest mit 5 bis 10 Kohlenstoffatomen enthält;
"m" und "n" jeweils unabhängig voneinander 0 oder 1 darstellen; und R ein Wasserstoffatom, ein Halogenatom oder eine Alkylgruppe mit 1 oder mehr und höchstens 5 Kohlenstoffatomen darstellt; und
wobei in der Formel (1S) der durch A₁ dargestellte Chelatbildungsteil eine Struktur aufweist, die von einer Verbindung stammt, die dargestellt wird durch eine der folgenden Formeln (A1) bis (A9): 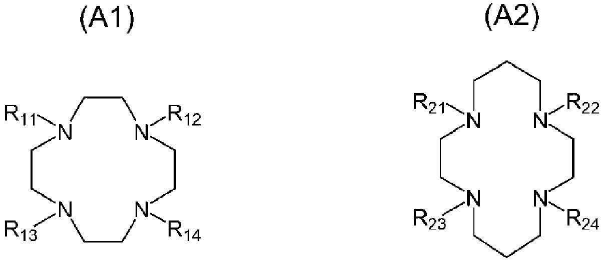 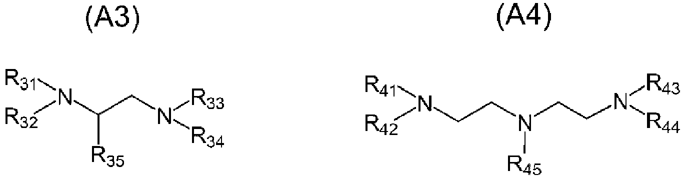 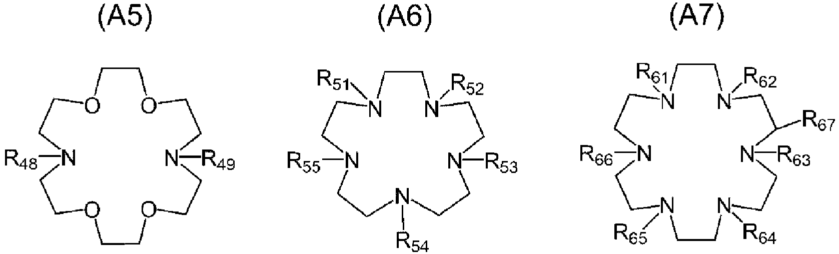 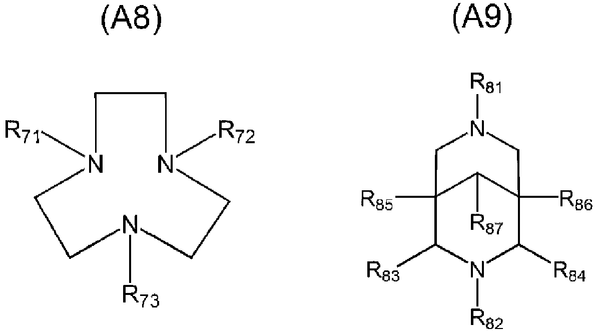
wobei in der Formel (A1) R₁₁, R₁₂, R₁₃ und R₁₄ jeweils unabhängig voneinander eine der Gruppen bestehend aus -(CH₂)ₚCOOH, -(CH₂)ₚC₅H₅N, -(CH₂)ₚPO₃H₂, -(CH₂)ₚCONH₂ und -(CHCOOH)(CH₂)ₚCOOH ist; und "p" eine ganze Zahl von 0 oder mehr und 3 oder weniger ist;
wobei in der Formel (A2) R₂₁, R₂₂, R₂₃ und R₂₄ jeweils unabhängig voneinander eine Carboxygruppe oder eine Carboxyalkylgruppe mit 2 oder 3 Kohlenstoffatomen darstellen;
wobei in der Formel (A3) R₃₁, R₃₂, R₃₃ und R₃₄ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten; und R₃₅ ein Wasserstoffatom, eine Carboxygruppe oder eine Carboxyalkylgruppe mit 2 oder 3 Kohlenstoffatomen darstellt;
wobei in der Formel (A4) R₄₁, R₄₂, R₄₃ und R₄₄ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten; und R₄₅ ein Wasserstoffatom, eine Carboxygruppe oder eine Carboxyalkylgruppe mit 2 oder 3 Kohlenstoffatomen darstellt;
wobei in der Formel (A5) R₄₈ und R₄₉ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten;
wobei in der Formel (A6) R₅₁, R₅₂, R₅₃, R₅₄ und R₅₅ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten;
wobei in der Formel (A7) R₆₁, R₆₂, R₆₃, R₆₄, R₆₅ und R₆₆ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthalten; und R₆₇ ein Wasserstoffatom, eine Carboxygruppe oder eine Carboxyalkylgruppe mit 2 oder 3 Kohlenstoffatomen darstellt;
wobei in der Formel (A8) R₇₁, R₇₂ und R₇₃ jeweils unabhängig voneinander eine Atomgruppe mit einem Wasserstoffatom und 2 oder mehr und 10 oder weniger Kohlenstoffatomen darstellen und optional ein Stickstoffatom oder ein Sauerstoffatom enthält; und
wobei in der Formel (A9) R₈₁ und R₈₂ jeweils unabhängig voneinander eine Alkylgruppe mit 1 oder mehr und 5 oder weniger Kohlenstoffatomen darstellen, und das Ende der Alkylgruppe mit einer Pyridylgruppe substituiert sein kann, die mit 1 oder mehr Carboxygruppen substituiert ist; R₈₇ eine Hydroxylgruppe oder das Sauerstoffatom (=O) einer Carbonylgruppe darstellt; R₈₃ und R₈₄ eine substituierte oder unsubstituierte Pyridinylgruppe darstellen; R₈₅ und R₈₆ jeweils unabhängig voneinander -COO-Rₐ darstellen; und Rₐ eine Alkylgruppe mit 1 oder mehr und 5 oder weniger Kohlenstoffatomen darstellt; und
wobei in der Formel (1S) C die folgende Formel (C1) darstellt: 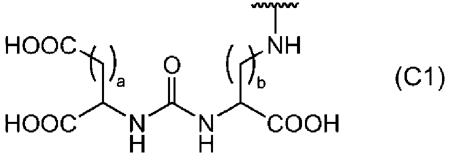
wobei in der Formel (C1) der Wellenlinienabschnitt einen Abschnitt darstellt, der an A₁ oder L_{b} in der allgemeinen Formel (1) gebunden ist; und
"a" und "b" jeweils unabhängig voneinander eine ganze Zahl von 1 oder mehr und 7 oder weniger darstellen.

## Revendications

1. Composé représenté par la formule générale (1) suivante : 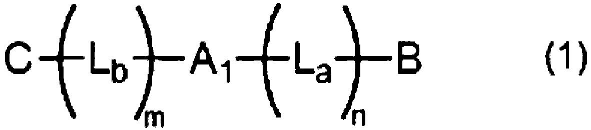
dans lequel, dans la formule générale (1), A₁ représente une partie chélatante capable de coordiner avec un ion métallique radioactif ;
B représente un groupe atomique contenant une partie de liaison à l'albumine ;
C représente un groupe atomique contenant une partie de liaison à la molécule de PSMA ;
Lₐ représente une structure de lieur comprenant une structure dérivée d'un composé capable de former une liaison amide ;
L_{b} représente une structure de lieur identique ou différente de Lₐ, comprenant une structure dérivée d'un acide aminé ayant une structure contenant un alicyclique de 5 à 10 atomes de carbone ;
« m » et « n » représentent chacun indépendamment 0 ou 1 ;
B ou Lₐ est lié à une position arbitraire de A₁ ; et
C ou L_{b} est lié à A₁ à une position différente de la position à laquelle B ou Lₐ est lié à A₁,
dans lequel, dans la formule (1), la partie chélatante représentée par A₁ possède une structure dérivée d'un composé représenté par l'une quelconque des formules (A1) à (A9) suivantes : 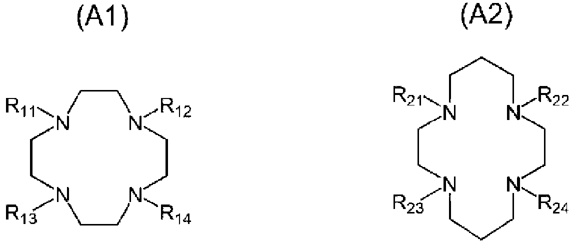 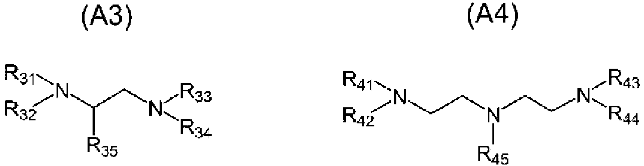 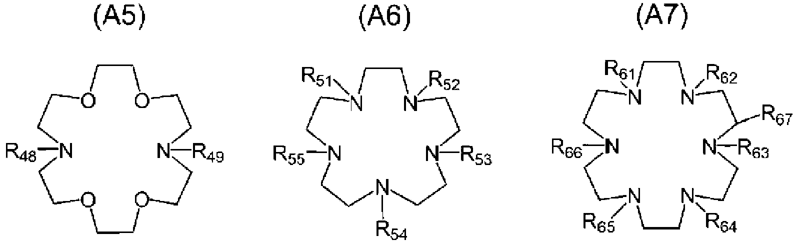 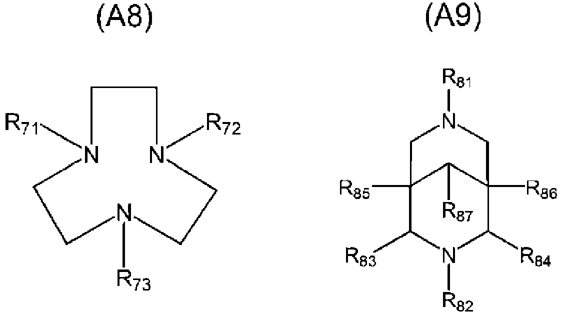
dans lequel, dans la formule (A1), R₁₁, R₁₂, R₁₃ et R₁₄ sont chacun indépendamment l'un quelconque des groupes constitués par -(CH₂)ₚCOOH, -(CH₂)ₚC₅H₅N, -(CH₂)ₚPO₃H₂, -(CH₂)ₚCONH₂, et -(CHCOOH)(CH₂)ₚCOOH ; et « p » est un entier de 0 ou plus et de 3 ou moins ;
dans lequel, dans la formule (A2), R₂₁, R₂₂, R₂₃ et R₂₄ représentent chacun indépendamment un groupe carboxy ou un groupe carboxyalkyle ayant 2 ou 3 atomes de carbone ;
dans lequel, dans la formule (A3), R₃₁, R₃₂, R₃₃ et R₃₄ représentent chacun indépendamment un groupe atomique possédant un atome d'hydrogène et 2 ou plus et 10 ou moins atomes de carbone et contenant éventuellement un atome d'azote ou un atome d'oxygène ; et R₃₅ représente un atome d'hydrogène, un groupe carboxy ou un groupe carboxyalkyle ayant 2 ou 3 atomes de carbone ;
dans lequel, dans la formule (A4), R₄₁, R₄₂, R₄₃ et R₄₄ représentent chacun indépendamment un groupe atomique possédant un atome d'hydrogène et 2 ou plus et 10 ou moins atomes de carbone et contenant éventuellement un atome d'azote ou un atome d'oxygène ; et R₄₅ représente un atome d'hydrogène, un groupe carboxy ou un groupe carboxyalkyle possédant 2 ou 3 atomes de carbone ;
dans lequel, dans la formule (A5), R₄₈ et R₄₉ représentent chacun indépendamment un groupe atomique possédant un atome d'hydrogène et 2 ou plus et 10 ou moins atomes de carbone et contenant éventuellement un atome d'azote ou un atome d'oxygène ;
dans lequel dans la formule (A6), R₅₁, R₅₂, R₅₃, R₅₄, et R₅₅ représentent chacun indépendamment un groupe atomique présentant un atome d'hydrogène et 2 ou plus et 10 ou moins atomes de carbone et contenant éventuellement un atome d'azote ou un atome d'oxygène ;
dans lequel, dans la formule (A7), R₆₁, R₆₂, R₆₃, R₆₄, R₆₅, et R₆₆ représentent chacun indépendamment un groupe atomique possédant un atome d'hydrogène et 2 ou plus et 10 ou moins atomes de carbone et contenant éventuellement un atome d'azote ou un atome d'oxygène ; et R₆₇ représente un atome d'hydrogène, un groupe carboxy ou un groupe carboxyalkyle possédant 2 ou 3 atomes de carbone ;
dans lequel, dans la formule (A8), R₇₁, R₇₂ et R₇₃ représentent chacun indépendamment un groupe atomique possédant un atome d'hydrogène et 2 ou plus et 10 ou moins atomes de carbone et contenant éventuellement un atome d'azote ou un atome d'oxygène ; et
dans lequel, dans la formule (A9), R₈₁ et R₈₂ représentent chacun indépendamment un groupe alkyle ayant 1 ou plus et 5 ou moins atomes de carbone, et la terminaison du groupe alkyle peut être substituée par un groupe pyridinyle substitué par 1 ou plusieurs groupes carboxy ; R₈₇ représente un groupe hydroxyle ou l'atome d'oxygène (=O) d'un groupe carbonyle ; R₈₃ et R₈₄ représentent un groupe pyridinyle substitué ou non substitué ; R₈₅ et R₈₆ représentent chacun indépendamment -COO-Rₐ ; et Rₐ est un groupe alkyle possédant 1 ou plus et 5 ou moins atomes de carbone ;
dans lequel dans la formule (1), la partie de liaison à l'albumine possède une structure représentée par la formule (B1) ou (B2) suivante : 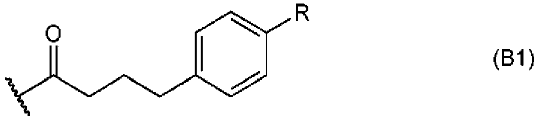 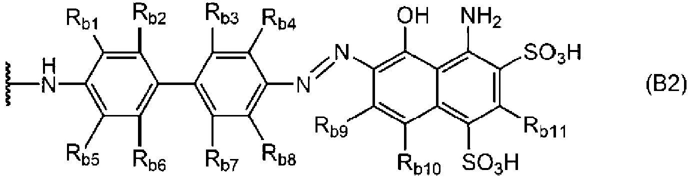
dans lequel, dans la formule (B1), R représente un atome d'hydrogène, un atome d'halogène ou un groupe alkyle ayant 1 ou plus et 5 ou moins atomes de carbone ; et la partie à ligne ondulée représente une partie de liaison à A₁ ou Lₐ dans la formule (1), et
dans la formule (B2), R_{b1} à R_{b11} représentent chacun indépendamment un atome d'hydrogène, un atome d'halogène, un groupe hydroxyle, un groupe cyano, un groupe alkyle substitué ou non substitué ayant 1 ou plus et 6 ou moins atomes de carbone ou un groupe alcoxy substitué ou non substitué ayant 1 ou plus et 6 ou moins atomes de carbone ; et la partie à ligne ondulée représente une partie de liaison à A₁ ou Lₐ dans la formule (1) ; et
dans lequel, dans la formule (1), C représente la formule (C1) suivante : 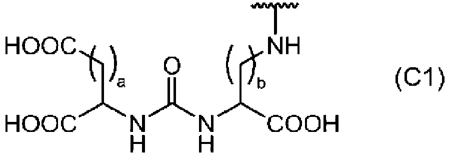
dans lequel, dans la formule (C1), la partie à ligne ondulée représente une partie de liaison à A₁ ou L_{b} dans la formule générale (1) ; et
« a » et « b » représentent chacun indépendamment un entier de 1 ou plus et de 7 ou moins.

2. Composé selon la revendication 1, dans lequel, dans la formule (1), A₁ est DOTA, HOPO, Octapa, Macropa, ou un dérivé correspondant.

3. Composé selon la revendication 1 ou 2, dans lequel, dans la formule (1), la partie de liaison à l'albumine a une structure représentée par la formule (B1).

4. Composé selon la revendication 1, dans lequel le composé représenté par la formule générale (1) est un composé représenté par la formule 205 suivante :

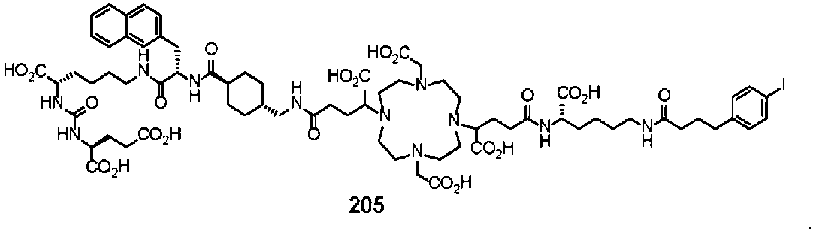

5. Composé radiomarqué comprenant : un ion métallique radioactif et le composé selon l'une quelconque des revendications 1 à 4 qui est coordiné avec l'ion métallique radioactif.

6. Composé radiomarqué selon la revendication 5, dans lequel le métal radioactif est ⁶⁸Ga, ⁶⁴Cu, ⁶⁷Cu, ⁸⁹Zr, ⁹⁰Y, ^{99m}Tc, ¹¹¹In, ¹⁷⁷Lu, ¹⁸⁶Re, ¹⁸⁸Re, ou ²²⁵Ac.

7. Composition pharmaceutique radioactive comprenant le composé radiomarqué selon la revendication 5 ou 6, en tant qu'ingrédient actif.

8. Procédé de fabrication d'un composé radiomarqué, le procédé comprenant : la coordination du composé selon l'une quelconque des revendications 1 à 4 à un ion métallique radioactif pour obtenir un composé radiomarqué.

9. Procédé de fabrication d'un composé radiomarqué selon la revendication 8, dans lequel le composé utilisé est représenté par la formule générale (1S) suivante : 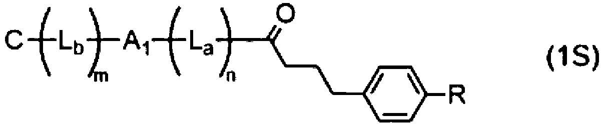
dans lequel, dans la formule (1S), A₁ représente une partie chélatante capable de coordiner avec un ion métallique radioactif ;
C représente un groupe atomique contenant une partie de liaison à la molécule de PSMA ;
Lₐ représente une structure de lieur comprenant une structure dérivée d'un composé capable de former une liaison amide ;
L_{b} représente une structure de lieur identique ou différente de Lₐ, comprenant une structure dérivée d'un acide aminé ayant une structure contenant un alicyclique de 5 à 10 atomes de carbone ;
« m » et « n » représentent chacun indépendamment 0 ou 1 ; et
R représente un atome d'hydrogène, un atome d'halogène, ou un groupe alkyle comportant 1 ou plus et 5 ou moins atomes de carbone ; et
dans lequel, dans la formule (1S), la partie chélatante représentée par A₁ possède une structure dérivée d'un composé représenté par l'une quelconque des formules (A1) à (A9) suivantes : 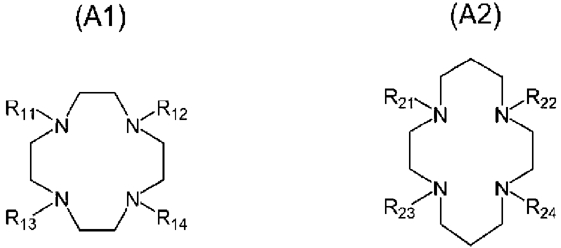 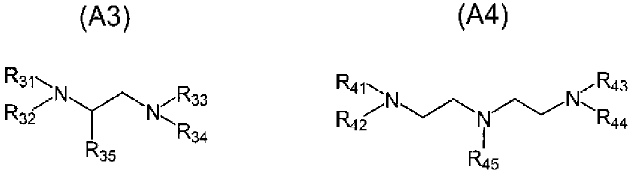 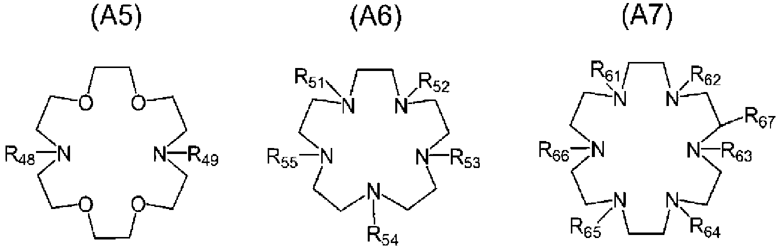 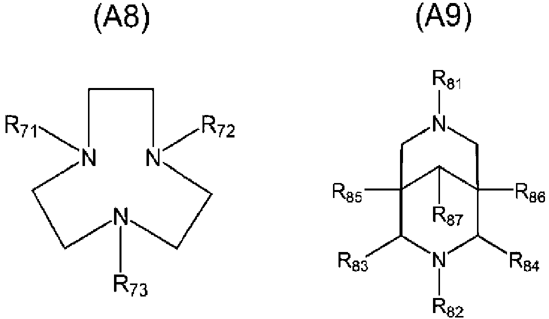
dans lequel, dans la formule (A1), R₁₁, R₁₂, R₁₃ et R₁₄ sont chacun indépendamment l'un quelconque des groupes constitués par -(CH₂)ₚCOOH, -(CH₂)ₚC₅H₅N, -(CH₂)ₚPO₃H₂, -(CH₂)ₚCONH₂, et -(CHCOOH)(CH₂)ₚCOOH ; et « p » est un entier de 0 ou plus et de 3 ou moins ;
dans lequel, dans la formule (A2), R₂₁, R₂₂, R₂₃ et R₂₄ représentent chacun indépendamment un groupe carboxy ou un groupe carboxyalkyle ayant 2 ou 3 atomes de carbone ;
dans lequel, dans la formule (A3), R₃₁, R₃₂, R₃₃ et R₃₄ représentent chacun indépendamment un groupe atomique possédant un atome d'hydrogène et 2 ou plus et 10 ou moins atomes de carbone et contenant éventuellement un atome d'azote ou un atome d'oxygène ; et R₃₅ représente un atome d'hydrogène, un groupe carboxy ou un groupe carboxyalkyle ayant 2 ou 3 atomes de carbone ;
dans lequel, dans la formule (A4), R₄₁, R₄₂, R₄₃ et R₄₄ représentent chacun indépendamment un groupe atomique possédant un atome d'hydrogène et 2 ou plus et 10 ou moins atomes de carbone et contenant éventuellement un atome d'azote ou un atome d'oxygène ; et R₄₅ représente un atome d'hydrogène, un groupe carboxy ou un groupe carboxyalkyle possédant 2 ou 3 atomes de carbone ;
dans lequel, dans la formule (A5), R₄₈ et R₄₉ représentent chacun indépendamment un groupe atomique possédant un atome d'hydrogène et 2 ou plus et 10 ou moins atomes de carbone et contenant éventuellement un atome d'azote ou un atome d'oxygène ;
dans lequel dans la formule (A6), R₅₁, R₅₂, R₅₃, R₅₄, et R₅₅ représentent chacun indépendamment un groupe atomique présentant un atome d'hydrogène et 2 ou plus et 10 ou moins atomes de carbone et contenant éventuellement un atome d'azote ou un atome d'oxygène ;
dans lequel, dans la formule (A7), R₆₁, R₆₂, R₆₃, R₆₄, R₆₅, et R₆₆ représentent chacun indépendamment un groupe atomique possédant un atome d'hydrogène et 2 ou plus et 10 ou moins atomes de carbone et contenant éventuellement un atome d'azote ou un atome d'oxygène ; et R₆₇ représente un atome d'hydrogène, un groupe carboxy ou un groupe carboxyalkyle possédant 2 ou 3 atomes de carbone ;
dans lequel, dans la formule (A8), R₇₁, R₇₂ et R₇₃ représentent chacun indépendamment un groupe atomique possédant un atome d'hydrogène et 2 ou plus et 10 ou moins atomes de carbone et contenant éventuellement un atome d'azote ou un atome d'oxygène ; et
dans lequel, dans la formule (A9), R₈₁ et R₈₂ représentent chacun indépendamment un groupe alkyle ayant 1 ou plus et 5 ou moins atomes de carbone, et la terminaison du groupe alkyle peut être substituée par un groupe pyridinyle substitué par 1 ou plusieurs groupes carboxy ; R₈₇ représente un groupe hydroxyle ou l'atome d'oxygène (=O) d'un groupe carbonyle ; R₈₃ et R₈₄ représentent un groupe pyridinyle substitué ou non substitué ; R₈₅ et R₈₆ représentent chacun indépendamment -COO-Rₐ ; et Rₐ est un groupe alkyle possédant 1 ou plus et 5 ou moins atomes de carbone ; et
dans lequel, dans la formule (1S), C représente la formule (C1) suivante : 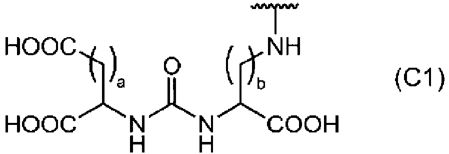
dans lequel, dans la formule (C1), la partie à ligne ondulée représente une partie de liaison à A₁ ou L_{b} dans la formule générale (1) ; et
« a » et « b » représentent chacun indépendamment un entier de 1 ou plus et de 7 ou moins.
